# EUROPEAN PATENT APPLICATION

(11) **EP 1 649 852 A1**
(43) Date of publication of application: **26.04.2006**
(21) Application number: 04747921.7
(22) Date of filing: 16.07.2004
(51) Int. Cl.: A61K 31/167, A61P 17/00, A61P 35/00

(54) **CHROMATOSIS REMEDIES**

(30) Priority: 16.07.2003 JP 2003197807
(71) Applicant: Institute of Medicinal Molecular Design, Inc., Tokyo 113-0033 (JP)
(72) Inventor: ITAI, A. INST. OF MEDICINAL MOLECULAR DESIGN INC., Bunkyo-ku, Tokyo 1130033 (JP); MUTO, S. INST. OF MEDICINAL MOLECULAR DESIGN INC., Bunkyo-ku, Tokyo 1130033 (JP)
(74) Representative: Sternagel, Fleischer, Godemeyer & Partner
(86) International application number: PCT/JP2004/010558
(87) International publication number: WO 2005/007151

(57) **Abstract**

A medicament for preventive and/or therapeutic treatment of dermal pigmentation and/or development of skin cancer, which comprises as an active ingredient a substance selected from the group consisting of a compound represented by the following general formula (I) and a pharmacologically acceptable salt thereof, and a hydrate thereof and a solvate thereof: 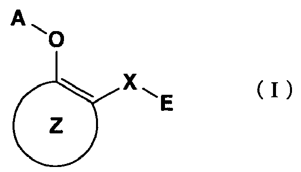 wherein X represents a connecting group whose number of atoms in a main chain is 2 to 5 (said connecting group may be substituted), A represents hydrogen atom or acetyl group, E represents an aryl group which may be substituted or a heteroaryl group which may be substituted, ring Z represents an arene which may have one or more substituents in addition to the group represented by formula -O-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula -X-E wherein each of X and E has the same meaning as that defined above.

## Description

### Field of Invention

The present invention relates to medicaments effective for preventive and/or therapeutic treatment of pigmentation and development of skin cancer caused by ultraviolet irradiation to skin.

### Background Art

Ultraviolet irradiation to skin causes burn-like damage to skin, and also induces hypodermic pigmentation to darken the skin. This phenomenon is well known as sunburn; however, a mechanism of the hypodermic pigmentation has not been clearly revealed until a recent date. When the skin is exposed to ultraviolet light, cytokines such as TNF(tumor necrosis factor), IL-1(interleukin-1), and bFGF (basic fibroblast growth factor) are secreted upon the stimulation, and then transformation and proliferation of melanocytes occur due to the stimulation, which are melanin production cells, to produce a large amount of melanin pigment, and successively the pigment moves to epidermal keratinocytes and deposits to darken the skin (American Journal of Pathology, Vol. 158, No.3, p.943-953, 2001).

Therefore, possible means to prevent the dermal pigmentation caused by the ultraviolet irradiation include prevention of exposure of skin to ultraviolet light, inhibition of proliferation of melanocytes, or inhibition of synthesis of melanin pigments. As for agents for prevention of ultraviolet exposure, liniments for skin have been practically used. However, the agents have only a short period of duration and insufficient preventive effects against ultraviolet light. Also as for the inhibitors of the synthesis of melanin pigments, skin liniments have been practically used. However, the liniments fail to have satisfactory effects, and moreover, kojic acid that is known as a typical active ingredient has revealed to be carcinogenic. So far, no effective means are available for prevention of the dermal pigmentation caused by ultraviolet irradiation.

Furthermore, it is a well known fact that ultraviolet irradiation to skin is a cause to develop a skin cancer, and as for a mechanism of the above cancer development, transformation and proliferation of cells caused by ultraviolet irradiation have been focused (Cancer Research, Vol.62, No.22, p.6724-6730, 2002). Ultraviolet light may possibly cause damage to cellular DNAs in tissues as well as stimulate cell proliferation, some cells with abnormal proliferation appear among cells under proliferation, which leads to the onset of a skin cancer such as melanoma.

Accordingly, an inhibitor with safety against cellular transformation and/or proliferation of melanocytes upon ultraviolet irradiation is expected to have inhibitory action against pigmentation as well as preventive effect on skin cancer development. However, no drug having the above action has been reported so far.

N-Phenylsalicylamide derivatives are disclosed as a plant growth inhibitor in the specification of U.S. Patent No.4,358,443. As medicaments, said derivatives are described as anti-inflammatory agents in the specification of European Patent No.0,221,211, Japanese Patent Unexamined Publication (KOKAI) No.(Sho)62-99329, and the specification of U.S. Patent No.6,117,859. Furthermore, they are disclosed as NF- *κ* B inhibitors in the pamphlets of International Publication WO99/65499, International Publication WO02/49632, and International Publication WO02/076918, and as inhibitors against the production of cytokines in the pamphlet of International Publication WO02/051397.

Furthermore, N-arylsalicylamide derivatives and N-heteroarylsalicylamide derivatives are disclosed as:
(1) inhibitors against activation of NF- κ B (the pamphlet of International Patent Publication WO03/103654);
(2) medicaments for treatment of cancer (the pamphlet of International Patent Publication WO03/103655);
(3) medicaments for treatment of neurodegenerative diseases (the pamphlet of International Patent Publication WO03/103657);
(4) medicaments for treatment of diabetes (the pamphlet of International Patent Publication WO03/103648);
(5) antiallergic agents (the pamphlet of International Patent Publication WO03/103665);
(6) inhibitors against activation of AP-1 and NFAT (the pamphlet of International Patent Publication WO03/103647);
(7) immunity-related protein kinase inhibitors (the pamphlet of International Patent Publication WO03/103658).

However, the aforementioned specifications of U.S. Patent No.4,358,443 and European Patent No.0,221,211, Japanese Patent Unexamined Publication (KOKAI) No.(Sho)62-99329, the specification of U.S. Patent No.6,117,859, the pamphlets of International Publication WO99/65499, International Publication WO02/49632, International Publication WO02/076918, International Publication WO02/051397, International Patent Publication WO03/103654, International Patent Publication WO03/103655, International Patent Publication WO03/103657, International Patent Publication WO03/103648, International Patent Publication WO03/103665, International Patent Publication WO03/103647, and International Patent Publication WO03/103658 do not teach or suggest that the compounds disclosed therein are useful for preventive and/or therapeutic treatment of dermal pigmentation and/or development of skin cancer, and that those compounds have inhibitory activity against transformation and/or proliferation of melanocytes caused by ultraviolet irradiation.

### Disclosure of the Invention

An object of the present invention is to provide medicaments which inhibit dermal pigmentation and also development of skin cancer. In order to solve the aforementioned object, the inventors of the present invention conducted various studies on inhibitory activity of salicylamide derivatives against proliferation of melanocytes under ultraviolet stimulation, which derivatives are generally considered to have low toxicity and are known to have inhibitory activity against release of cytokines. As a result, they found that N-substituted salicylamide·derivatives, particularly, N-arylsalicylamide derivatives, more specifically, N-phenylsalicylamide derivatives whose aniline moiety is substituted in the 2- and 5-position or in the 3- and 5-position, and N-(thiazol-2-yl)salicylamide derivatives whose thiazole ring is substituted in the 4- and 5-position had extremely superior inhibitory activity against transformation and proliferation of melanocytes under ultraviolet stimulation, and thereby preventive and/or therapeutic treatment of dermal pigmentation and/or development of skin cancer were achievable. The inventors also conducted studies on analogous hydroxyaryl derivatives. The present invention was achieved on the basis of these findings.

The present invention thus provides:
(1) a medicament for preventive and/or therapeutic treatment of dermal pigmentation and/or development of skin cancer, which comprises as an active ingredient a substance selected from the group consisting of a compound represented by the following general formula (I) and a pharmacologically acceptable salt thereof, and a hydrate thereof and a solvate thereof: 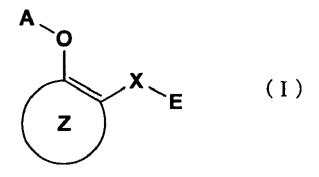 wherein X represents a connecting group whose number of atoms in a main chain is 2 to 5 (said connecting group may be substituted),
   A represents hydrogen atom or acetyl group,
   E represents an aryl group which may be substituted or a heteroaryl group which may be substituted,
   ring Z represents an arene which may have one or more substituents in addition to the group represented by formula -O-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula ― X―E wherein each of X and E has the same meaning as that defined above.

Examples of preferred medicaments provided by the present invention include:
(2) the aforementioned medicament which comprises as an active ingredient a substance selected from the group consisting of the compound and a pharmacologically acceptable salt thereof, and a hydrate thereof and a solvate thereof, wherein X is a group selected from the following connecting group α (said group may be substituted),
   A is hydrogen atom or acetyl group,
   E is a C₆ to C₁₀ aryl group which may be substituted or a 5- to 13-membered heteroaryl group which may be substituted,
   ring Z is a C₆ to C₁₀ arene which may have one or more substituents in addition to the group represented by formula ―O―A wherein A has the same meaning as that defined above and the group represented by formula ―X―E wherein each of X and E has the same meaning as that defined above, or a 5- to 13-membered heteroarene which may have one or more substituents in addition to the group represented by formula ―0―A wherein A has the same meaning as that defined above and the group represented by formula -X-E wherein each ofX and E has the same meaning as that defined above;
   [Connecting Group α] The following formulas: 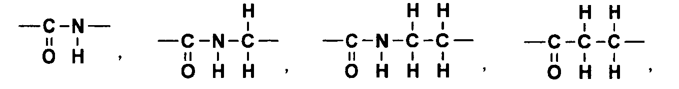 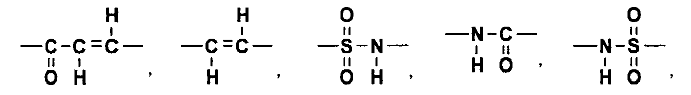 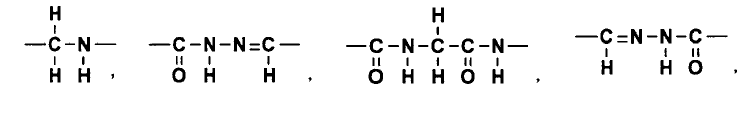 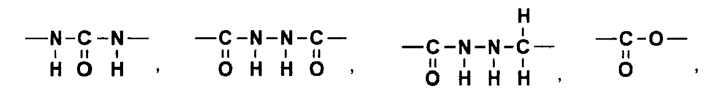 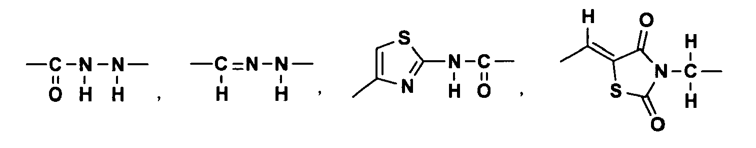 wherein a bond at the left end binds to ring Z and a bond at the right end binds to E.
(3) the aforementioned medicament which comprises as an active ingredient a substance selected from the group consisting of the compound and a pharmacologically acceptable salt thereof, and a hydrate thereof and a solvate thereof, wherein X is a group represented by the following formula (said group may be substituted): 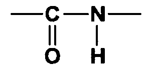 wherein a bond at the left end binds to ring Z and a bond at the right end binds to E, A is hydrogen atom or acetyl group,
   E is a C₆ to C₁₀ aryl group which may be substituted or a 5- to 13-membered heteroaryl group which may be substituted,
   ring Z is a C₆ to C₁₀ arene which may have one or more substituents in addition to the group represented by formula - O-A wherein A has the same meaning as that defined above and the group represented by formula -X-E wherein each of X and E has the same meaning as that defined above, or a 5- to 13-membered heteroarene which may have one or more substituents in addition to the group represented by formula -O-A wherein A has the same meaning as that defined above and the group represented by formula -X-E wherein each of X and E has the same meaning as that defined above;
(4) the aforementioned medicament which comprises as an active ingredient a substance selected from the group consisting of the compound and a pharmacologically acceptable salt thereof, and a hydrate thereof and a solvate thereof, wherein A is hydrogen atom or acetyl group,
   E is a phenyl group which may be substituted or a thiazol-2-yl group which may be substituted,
   ring Z is a benzene ring which may have one or more substituents in addition to the group represented by formula -O-A wherein A has the same meaning as that defined above and the group represented by formula -X-E wherein each of X and E has the same meaning as that defined above, or a naphthalene ring which may have one or more substituents in addition to the group represented by formula -O-A wherein A has the same meaning as that defined above and the group represented by formula ―X―E wherein each of X and E has the same meaning as that defined above;
(5) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a 2,5-di-substituted phenyl group, a 3,5-di-substituted phenyl group, or a 4,5-di-substituted thiazol-2-yl group,
   ring Z is a benzene ring which has one to three substituents in addition to the group represented by formula ― O―A wherein A has the same meaning as that defined above and the group represented by formula -X-E wherein each of X and E has the same meaning as that defined above;
(6) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a 2,5-di-substituted phenyl group wherein at least one of said substituents is trifluoromethyl group, a 3,5-di-substituted phenyl group wherein at least one of said substituents is trifluoromethyl group, or a 4,5-di-substituted thiazol-2-yl group,
   ring Z is a benzene ring which has one to three groups selected from the following substituent group γ -1z, in addition to the group represented by formula ―O―A wherein A has the same meaning as that defined above and the group represented by formula -X-E wherein each of X and E has the same meaning as that defined above; [Substituent Group γ -1z] a halogen atom, nitro group, cyano group, hydroxy group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group [thiophen-2-yl group], 3-thienyl group [thiophen-3-yl group], 1-pyrrolyl group [pyrrol-1-yl group], 2-methylthiazol-4-yl group, imidazo[1,2-a]pyridin-2-yl group, 2-pyridyl group [pyridin-2-yl group], acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group
(7) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a group selected from the group consisting of the following substituent group δ -3e, substituent group δ -5e, and substituent group δ -8e,
   the following partial formula (Iz-1) in the general formula (I) containing ring Z 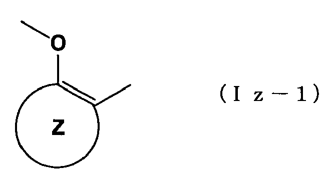 is the following formula (Iz-2): 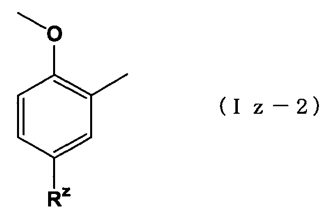 wherein R^{z} represents a group selected from the following substituent group γ -2z; [Substituent Group δ -3e] 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 2-(4-cyanophenoxy)-5-(trifluoromethyl)phenyl group, 2-(4-methoxyphenoxy)-5-(trifluoromethyl)phenyl group [Substituent Group δ -5e] 3,5-bis(trifluoromethyl)phenyl group, 3-fluoro-5-(trifluoromethyl)phenyl group, 3-bromo-5-(trifluoromethyl)phenyl group, 3-methoxy-5-(trifluoromethyl)phenyl group, 3-methoxycarbonyl-5-(trifluoromethyl)phenyl group, 3-carboxy-5-(trifluoromethyl)phenyl group [Substituent Group δ -8e] 5-bromo-4-[(1,1-dimethyl)ethyl]thiazol-2-yl group, 5-bromo-4-(trifluoromethyl)thiazol-2-yl group, 5-cyano-4-[(1,1-dimethyl)ethyl]thiazol-2-yl group, 5-methylthiazol-2-yl group, 4,5-dimethylthiazol-2-yl group, 5-methyl-4-phenylthiazol-2-yl group, 5-(4-fluorophenyl)-4-methylthiazol-2-yl group, 4-methyl-5-[3-(trifluoromethyl)phenyl]thiazol-2-yl group, 4-[(1,1-dimethyl)ethyl]-5-ethylthiazol-2-yl group, 4-ethyl-5-phenylthiazol-2-yl group, 4-isopropyl-5-phenylthiazol-2-yl group, 4-butyl-5-phenylthiazol-2-yl group, 4-[(1,1-dimethyl)ethyl]-5-[(2,2-dimethyl)propionyl]thiazol-2-yl group, 4-[(1,1-dimethyl)ethyl]-5-(ethoxycarbonyl)thiazol-2-yl group, 4-[(1,1-dimethyl)ethyl]-5-piperidinothiazol-2-yl group, 4-[(1,1-dimethyl)ethyl]-5-morpholinothiazol-2-yl group, 4-[(1,1-dimethyl)ethyl]-5-(4-methylpiperazin-1-yl)thiazol-2-yl group, 4-[(1,1-dimethyl)ethyl]-5-(4-phenylpiperazin-1-yl)thiazol-2-yl group, 5-carboxymethyl-4-phenylthiazol-2-yl group, 4,5-diphenylthiazol-2-yl group, 4-benzyl-5-phenylthiazol-2-yl group, 5-phenyl-4-(trifluoromethyl)thiazol-2-yl group, 5-acetyl-4-phenylthiazol-2-yl group, 5-benzoyl-4-phenylthiazol-2-yl group, 5-ethoxycarbonyl-4-phenylthiazol-2-yl group, 5-ethoxycarbonyl-4-(pentafluorophenyl)thiazol-2-yl group, 5-methylcarbamoyl-4-phenylthiazol-2-yl group, 5-ethylcarbamoyl-4-phenylthiazol-2-yl group, 5-isopropylcarbamoyl-4-phenylthiazol-2-yl group, 5-(2-phenylethyl)carbamoyl-4-phenylthiazol-2-yl group, 5-ethoxycarbonyl-4-(trifluoromethyl)thiazol-2-yl group, 5-carboxy-4-[(1,1-dimethyl)ethyl]thiazol-2-yl group, 5-(ethoxycarbonyl)methyl-4-phenylthiazol-2-yl group, 5-carboxy-4-phenylthiazol-2-yl group, 5-propylcarbamoyl-4-phenylthiazol-2-yl group
   [Substituent Group γ -2z] a halogen atom, nitro group, cyano group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group, 3-thienyl group, 1-pyrrolyl group, 2-methylthiazol-4-yl group, imidazo[1,2-a]pyridin-2-yl group, 2-pyridyl group, acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group
(8) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a group selected from the group consisting of the aforementioned substituent group δ -3e, substituent group δ -5e, and substituent group δ -8e,
   the following partial formula (Iz-1) in the general formula (I) containing ring Z 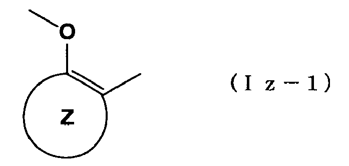 is the following formula (Iz-2): 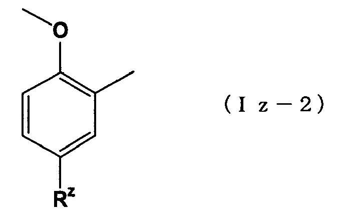 wherein R^{z} represents a halogen atom;
(9) the aforementioned medicament which comprises as an active ingredient a substance selected from the group consisting of the compound and a pharmacologically acceptable salt thereof, and a hydrate thereof and a solvate thereof, wherein A is hydrogen atom,
   E is 2,5-bis(trifluoromethyl)phenyl group, 3,5-bis(trifluoromethyl)phenyl group, or 4-[(1,1-dimethyl)ethyl]-5-[(2,2-dimethyl)propionyl]thiazol-2-yl group,
   the following partial formula (Iz-1) in the general formula (I) containing ring Z 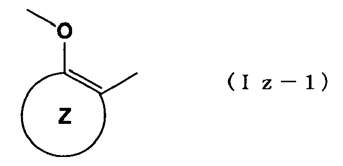 is the following formula (Iz-2): 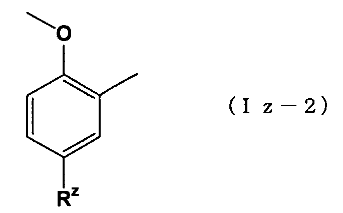 wherein R^{z} represents a halogen atom;
(10) the aforementioned medicament which comprises as an active ingredient a substance selected from the group consisting of the compound and a pharmacologically acceptable salt thereof, and a hydrate thereof and a solvate thereof, wherein A is hydrogen atom,
   E is 3,5-bis(trifluoromethyl)phenyl group,
   the following partial formula (Iz-1) in the general formula (I) containing ring Z 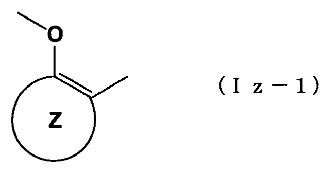 is the following formula (Iz-2): 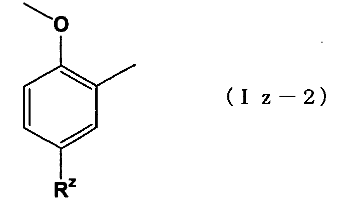 wherein R^{z} represents a halogen atom;
(11) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a C₆ to C₁₀ aryl group which may be substituted or a 5- to 13-membered heteroaryl group which may be substituted,
   the following partial formula (Iz-1) in the general formula (I) containing ring Z 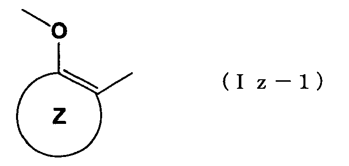 is the following formula (Iz-2): 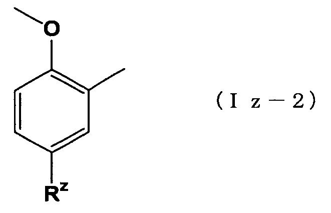 wherein R^{z} represents a halogen atom;
(12) the aforementioned medicament which comprises as an active ingredient a substance selected from the group consisting of the compound and a pharmacologically acceptable salt thereof, and a hydrate thereof and a solvate thereof, wherein A is hydrogen atom,
   E is a 2,5-di-substituted phenyl group, a 3,5-di-substituted phenyl group, or a 4,5-di-substituted thiazol-2-yl group,
   the following partial formula (Iz-1) in the general formula (I) containing ring Z 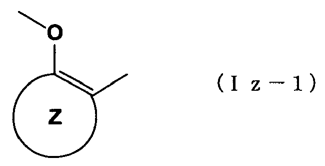 is the following formula (Iz-2): 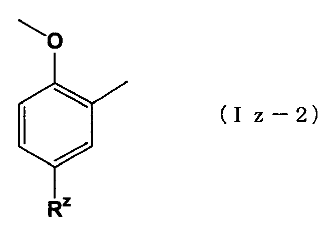 wherein R^{z} represents a halogen atom;
(13) the aforementioned medicament which comprises as an active ingredient a substance selected from the group consisting of the compound and a pharmacologically acceptable salt thereof, and a hydrate thereof and a solvate thereof, wherein A is hydrogen atom,
   E is 3,5-bis(trifluoromethyl)phenyl group,
   ring Z is a C₆ to C₁₀ arene which may have one or more substituents in addition to the group represented by formula -O-A wherein A has the same meaning as that defined above and the group represented by formula -X-E wherein each of X and E has the same meaning as that defined above, or a 5- to 13-membered heteroarene which may have one or more substituents in addition to the group represented by formula - O-A wherein A has the same meaning as that defined above and the group represented by formula -X-E wherein each of X and E has the same meaning as that defined above;
(14) the aforementioned medicament which comprises as an active ingredient a substance selected from the group consisting of the compound and a pharmacologically acceptable salt thereof, and a hydrate thereof and a solvate thereof, wherein the compound represented by the general formula (I) is a compound selected from the group consisting of the compounds described in the pamphlet of International Patent Publication WO03/103647 as Compound No. 1 to 555; and
(15) the aforementioned medicament which comprises as an active ingredient a substance selected from the group consisting of the compound and a pharmacologically acceptable salt thereof, and a hydrate thereof and a solvate thereof, wherein the compound represented by the general formula (I) is a compound selected from the group consisting of the compounds described in the pamphlet of International Patent Publication WO03/103647 as Compound No. 18 to 223 and Compound No. 322 to 555.

From another aspect, the present invention provides use of each of the substances for manufacture of the medicament according to the aforementioned (1) to (15).

The present invention further provides a method for preventive and/or therapeutic treatment of dermal pigmentation and/or development of skin cancer in a mammal including a human, which comprises the step of administering a preventively and/or therapeutically effective amount of the aforementioned substance to a mammal including a human.

The present invention further provides an inhibitor against transformation and/or proliferation of melanocytes caused by ultraviolet irradiation. The medicaments of the present invention may be used for preventive and/or therapeutic treatment of, for example, pigmentation by sunburn, pigmentation by transformation and/or proliferation of melanocytes accompanied by dermatitis such as atopic dermatitis, pigmentation by transformation and proliferation of melanocytes caused by proliferation-inducing stimulation, and pigmentation in diseases with melanocyte proliferation.

From further another aspect, the present invention provides a cosmetic, which comprises the aforementioned substance as an ingredient for skin whitening.

Furthermore, the compounds of the present invention have an inhibitory activity against secretion of cell proliferation-inducing cytokines. Therefore, they are useful for preventive and/or therapeutic treatment of cell proliferative dermatoses such as keloid and psoriasis.

### Best Mode for Carrying out the Invention

References to the disclosures in the pamphlets of International Publication WO02/49632, International Publication WO03/103654 and International Publication WO03/103647 are useful for better understanding of the present invention. The entire disclosures in the aforementioned pamphlets of International Publication WO02/49632, International Publication WO03/103654, and International Publication WO03/103647 are incorporated by reference in the disclosures of the specification.

The compounds encompassed within the general formula (I) as active ingredients of the medicaments of the present invention are disclosed, for example, in the pamphlets of International Publication WO02/49632, International Publication WO03/103654, and International Publication WO03/103647,

In the pamphlets of International Publication WO02/49632,
(1) preferred embodiments of the compounds which are encompassed within the general formula (I) as active ingredients of the medicaments of the present invention (pp.38-109 and pp.119-120);
(2) preferred examples of the compounds which are encompassed within the general formula (I) as active ingredients of the medicaments of the present invention (pp.120-156);
(3) general preparation methods of the compounds which are encompassed within the general formula (I) as active ingredients of the medicaments of the present invention (pp.157-162); and
(4) examples of the preparation of the compounds which are encompassed within the general formula (I) as active ingredients of the medicaments of the present invention (pp.167-289)
   are disclosed. The aforementioned entire disclosures are incorporated by reference in the disclosures of the specification.

In the pamphlets of International Publication WO03/103654,
(1) preferred embodiments of the compounds which are encompassed within the general formula (I) as active ingredients of the medicaments of the present invention (pp.7-74);
(2) preferred examples of the compounds which are encompassed within the general formula (I) as active ingredients of the medicaments of the present invention (pp.74-111);
(3) general preparation methods of the compounds which are encompassed within the general formula (I) as active ingredients of the medicaments of the present invention (pp.112-114); and
(4) examples of the preparation of the compounds which are encompassed within the general formula (I) as active ingredients of the medicaments of the present invention (pp.121-253)
   are disclosed. The aforementioned entire disclosures are incorporated by reference in the disclosures of the specification.

In the pamphlets of International Publication WO03/103647,
(1) preferred embodiments of the compounds which are encompassed within the general formula (I) as active ingredients of the medicaments of the present invention (pp.6-81);
(2) preferred examples of the compounds which are encompassed within the general formula (I) as active ingredients of the medicaments of the present invention (pp.81-150);
(3) general preparation methods of the compounds which are encompassed within the general formula (I) as active ingredients of the medicaments of the present invention (pp.151-164); and
(4) examples of the preparation of the compounds which are encompassed within the general formula (I) as active ingredients of the medicaments of the present invention (pp.168-382)
   are disclosed. The aforementioned entire disclosures are incorporated by reference in the disclosures of the specification.

In the following, compounds represented by the aforementioned general formula (I) are explained in details.

"Connecting group whose number of atoms of main chain is 2 to 5" in the definition of X means connecting groups wherein 2 to 5 atoms in a main chain link together between rings Z and E. The aforementioned "number of atoms of the main chain" is counted so as to minimize the number of connecting atoms existing between the rings Z and E, regardless of the presence or absence of hetero atom(s). For example, the number of atoms of 1,2-cyclopentylene is counted as 2, the number of atoms of 1,3-cyclopentylene is counted as 3, the number of atoms of 1,4-phenylene is counted as 4, and the number of atoms of 2,6-pyridine-diyl is counted as 3.

The aforementioned "connecting group whose number of atoms of main chain is 2 to 5" is formed by one functional group selected from the following group of divalent group ζ -1, or formed by combining 2 to 4 functional groups of 1 to 4 kinds selected from the following divalent group ζ -2.
[Divalent Group ζ -1] the following formulas: 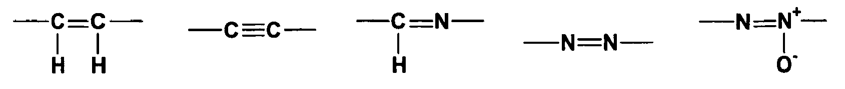 [Divalent Group ζ-2] the following formulas:

―O― ―S―

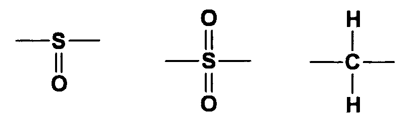

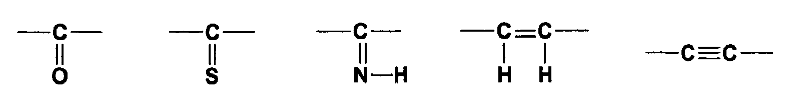

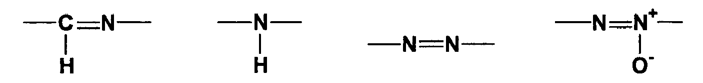

When two or more divalent groups combine, each group may be the same or different.

The aforementioned "connecting group wherein the number of atoms of the main chain is 2 to 5," is preferably a group selected from the following connecting group α.
[Connecting group α] the following formulas: 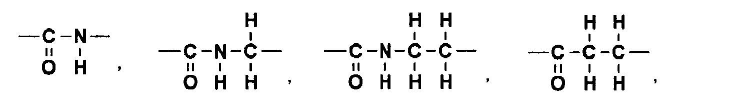 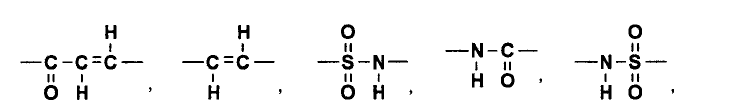 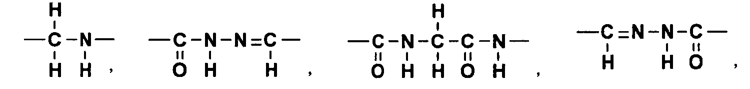 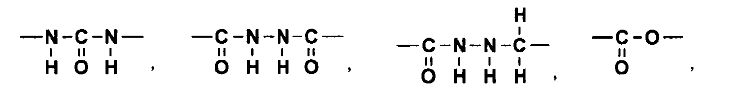 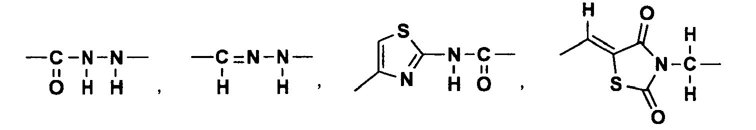 wherein a bond at the left end binds to ring Z and a bond at the right end binds to E.

The group represented by the following formula is most preferred: 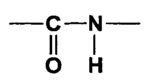 wherein the bond at the left end binds to ring Z and the bond at the right end binds to E.

Examples of the substituent, according to "connecting group which may be substituted" in the definition of "a connecting group whose number of atoms of the main chain is 2 to 5," include similar groups to the substituents in the definition of the aforementioned "which may be substituted." A C₁ to C₆ alkyl group is preferred, and a methyl group is more preferred. The substituent may combine with a substituent of the ring E or Z, together with atoms to which they bind, to form a cyclic group which may be substituted. Examples include the compounds represented by the general formula (I) being those represented by the following formulas:

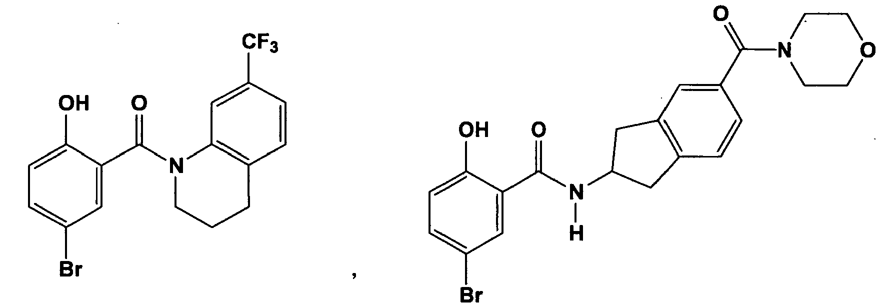

In the aforementioned general formula (I), examples of A include hydrogen atom or acetyl group, and hydrogen atom is preferred.

Examples of the "arene" in "an arene which may have one or more substituents in addition to the group represented by formula -O-A wherein A has the same meaning as that defined above and the group represented by formula -X-E wherein each of X and E has the same meaning as that defined above" in the definition of ring Z include a monocyclic or fused heterocyclic aromatic hydrocarbon, and include, for example, benzene ring, naphthalene ring, anthracene ring, phenanthrene ring, and acenaphylene ring. C₆ to C₁₀ arenes such as benzene ring, naphthalene ring and the like are preferred, benzene ring and naphthalene ring are more preferred, and benzene ring is most preferred.

Examples of the substituent in the definition of "an arene which may have one or more substituents in addition to the group represented by formula -O-A wherein A has the same meaning as that defined above and the group represented by formula ―X―E wherein each of X and E has the same meaning as that defined above" in the aforementioned definition of ring Z include similar groups to the substituent explained for the definition "which may be substituted." The position of substituents existing on the arene is not particularly limited, and when two or more substituents exist, they may be the same or different.

When "an arene which may have one or more substituents in addition to the group represented by formula -O-A wherein A has the same meaning as that defined above and the group represented by formula -X-E wherein each of X and E has the same meaning as that defined above" in the aforementioned definition of ring Z is "a benzene ring which may have one or more substituents in addition to the group represented by formula ― O―A wherein A has the same meaning as that defined above and the group represented by formula -X-E wherein each of X and E has the same meaning as that defined above," "a benzene ring which has one to three substituents in addition to the group represented by formula ―O―A wherein A has the same meaning as that defined above and the group represented by formula ―X―E wherein each of X and E has the same meaning as that defined above" is preferred, and "a benzene ring which has one substituent in addition to the group represented by formula ―O―A wherein A has the same meaning as that defined above and the group represented by formula ―X―E wherein each of X and E has the same meaning as that defined above" is more preferred. Preferred examples of the said substituents include groups selected from the following substituent group γ-1z. Halogen atom and tert-butyl group [(1,1-dimethyl)ethyl group] are more preferred, and halogen atom is most preferred.
[Substituent Group γ -1z] halogen atoms, nitro group, cyano group, hydroxy group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group [thiophen-2-yl group], 3-thienyl group [thiophen-3-yl group], 1-pyrrolyl group [pyrrol-1-yl group], 2-methylthiazol-4-yl group, imidazo[1,2-a]pyridin-2-yl group, 2-pyridyl group [pyridin-2-yl group], acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group

When "an arene which may have one or more substituents in addition to the group represented by formula ― O―A wherein A has the same meaning as that defined above and the group represented by formula ―X―E wherein each of X and E has the same meaning as that defined above" in the aforementioned definition of ring Z is "a benzene ring which may have one or more substituents in addition to the group represented by formula -O-A wherein A has the same meaning as that defined above and the group represented by formula -X-E wherein each of X and E has the same meaning as that defined above," it is most preferable that one substituent exists and locates on the position of R^{z} when the following partial formula (Iz-1) in the general formula containing ring Z 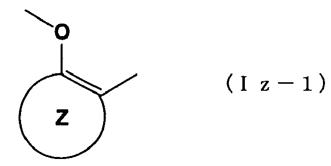 is represented by the following formula (Iz-2). 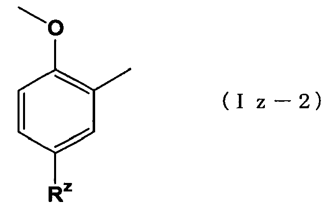 At this time, the said substituents can be defined as R^{z}. Preferred examples of R^{z} include a group selected from the following substituent group γ -2z. Halogen atom and tert-butyl group are more preferred, and halogen atom is most preferred.
[Substituent Group γ -2z] halogen atoms, nitro group, cyano group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group, 3-thienyl group, 1-pyrrolyl group, 2-methylthiazol-4-yl group, imidazo[1,2-a]pyridin-2-yl group, 2-pyridyl group, acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group

When "an arene which may have one or more substituents in addition to the group represented by formula ―O―A wherein A has the same meaning as that defined above and the group represented by formula ―X―E wherein each of X and E has the same meaning as that defined above" in the aforementioned definition of ring Z is "a naphthalene ring which may have one or more substituents in addition to the group represented by formula ― O― A wherein A has the same meaning as that defined above and the group represented by formula ―X― E wherein each of X and E has the same meaning as that defined above," naphthalene ring is preferred.

Examples of the "hetero arene" in "a hetero arene which may have one or more substituents in addition to the group represented by formula -O-A wherein A has the same meaning as that defined above and the group represented by formula -X-E wherein each of X and E has the same meaning as that defined above" in the aforementioned definition of ring Z include a monocyclic or a fused polycyclic aromatic heterocyclic rings containing at least one of 1 to 3 kinds of heteroatoms selected from oxygen atom, sulfur atom and nitrogen atom and the like as ring-constituting atoms (ring forming atoms), and include, for example, furan ring, thiophene ring, pyrrole ring, oxazole ring, isoxazole ring, thiazole ring, isothiazole ring, imidazole ring, pyrazole ring, 1,2,3-oxadiazole ring, 1,2,3-thiadiazole ring, 1,2,3-triazole ring, pyridine ring, pyridazine ring, pyrimidine ring, pyrazine ring, 1,2,3-triazine ring, 1,2,4-triazine ring, 1H-azepine ring, 1,4-oxepine ring, 1,4-thiazepine ring, benzofuran ring, isobenzofuran ring, benzo[b]thiophene ring, benzo[c]thiophene ring, indole ring, 2H-isoindole ring, 1H-indazole ring, 2H-indazole ring, benzoxazole ring, 1,2-benzisoxazole ring, 2,1-benzisoxazole ring, benzothiazole ring, 1,2-benzisothiazole ring, 2,1-benzisothiazole ring, 1,2,3-benzoxadiazol ring, 2,1,3-benzoxadiazol ring, 1,2,3-benzothiadiazole ring, 2,1,3-benzothiadiazole ring, 1H-benzotriazole ring, 2H-benzotriazole ring, quinoline ring, isoquinoline ring, cinnoline ring, quinazoline ring, quinoxaline ring, phthalazine ring, naphthyridine ring, 1H-1,5-benzodiazepine ring, carbazole ring, α -carboline ring, β -carboline ring, γ -carboline ring, acridine ring, phenoxazine ring, phenothiazine ring, phenazine ring, phenanthridine ring, phenanthroline ring, thianthrene ring, indolizine ring, and phenoxathiine ring, which are 5 to 14-membered monocyclic or fused polycyclic aromatic heterocyclic rings. 5 to 13-membered monocyclic or fused polycyclic aromatic heterocyclic rings are preferred, and thiophene ring, pyridine ring, indole ring, quinoxaline ring, and carbazole ring are more preferred.

Examples of the substituent in the definition of "a hetero arene which may have one or more substituents in addition to the group represented by formula -O-A wherein A has the same meaning as that defined above and the group represented by formula ―X―E wherein each of X and E has the same meaning as that defined above" in the aforementioned definition of ring Z include similar groups to the substituent explained for the aforementioned definition "which may be substituted." The position of substituents existing on the hetero arene is not particularly limited, and when two or more substituents exist, they may be the same or different.

Halogen atom is preferred as the substituent in the definition of "a hetero arene which may have one or more substituents in addition to the group represented by formula ―O―A wherein A has the same meaning as that defined above and the group represented by formula ―X―E wherein each of X and E has the same meaning as that defined above" in the aforementioned definition of ring Z.

Examples of the aryl group of "an aryl group which may be substituted" in the definition of E include similar groups to the aryl group in the definition of the aforementioned "hydrocarbon group," and C₆ to C₁₀ aryl groups such as phenyl group, 1-naphthyl group, 2-naphthyl group and the like are preferred, and phenyl group is most preferred.

Examples of the substituent in the definition of "an aryl group which may be substituted" in the definition of E include similar groups to the substituent explained for the definition "which may be substituted." The position of substituents existing on the aryl group is not particularly limited, and when two or more substituents exist, they may be the same or different.

When "an aryl group which may be substituted" in the aforementioned definition of E is "a phenyl group which may be substituted," "a mono-substituted phenyl group," "a di-substituted phenyl group," and "a phenyl group which has three or more substituents" are preferred, and "a di-substituted phenyl group" is more preferred.

When "an aryl group which may be substituted" in the aforementioned definition of E is "a di-substituted phenyl group," preferred examples of the group include groups represented by the following substituent group δ -1e.
[Substituent Group δ-1e] 3,5-bis(trifluoromethyl)phenyl group, 3,4-propylenedioxyphenyl group, 3,5-dichlorophenyl group, 2,4-dihydroxyphenyl group, 2,5-dimethoxyphenyl group, 2-chloro-5-(trifluoromethyl)phenyl group, 3,5-bis[(1,1-dimethyl)ethyl]phenyl group, 2,5-bis(trifluoromethyl)phenyl group, 4-chloro-2-(trifluoromethyl)phenyl group, 2-fluoro-3-(trifluoromethyl)phenyl group, 4-fluoro-3-(trifluoromethyl)phenyl group, 4-chloro-3-(trifluoromethyl)phenyl group, 3-fluoro-5-(trifluoromethyl)phenyl group, 3-bromo-5-(trifluoromethyl)phenyl group, 2-fluoro-5-(trifluoromethyl)phenyl group, 4-nitro-3-(trifluoromethyl)phenyl group, 2-nitro-5-(trifluoromethyl)phenyl group, 4-cyano-3-(trifluoromethyl)phenyl group, 2-methyl-3-(trifluoromethyl)phenyl group, 4-methyl-3-(trifluoromethyl)phenyl group, 2-methyl-5-(trifluoromethyl)phenyl group, 4-methoxy-3-(trifluoromethyl)phenyl group, 3-methoxy-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-chloro-4-(trifluoromethyl)phenyl group, 2,5-dichlorophenyl group, 3,4-dichlorophenyl group, 3,5-difluorophenyl group, 3,5-dinitrophenyl group, 2,5-bis[(1,1-dimethyl)ethyl]phenyl group, 5-[(1,1-dimethyl)ethyl]-2-methoxyphenyl group, 3,5-dimethylphenyl group, 4-methoxybiphenyl-3-yl group, 3,5-dimethoxyphenyl group, 3,5-bis(methoxycarbonyl)phenyl group, 2-bromo-5-(trifluoromethyl)phenyl group, 3-methoxycarbonyl-5-(trifluoromethyl)phenyl group, 3-carboxy-5-(trifluoromethyl)phenyl group, 2 - (2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 3,5-dicarboxyphenyl group, 5-isopropyl-2-methylphenyl group, 2,5-diethoxyphenyl group, 2,5-dimethylphenyl group, 5-chloro-2-cyano group, 5-diethylsulfamoyl-2-methoxyphenyl group, 2-chloro-5-nitrophenyl group, 2-methoxy-5-(phenylcarbamoyl)phenyl group, 5-acetylamino-2-methoxyphenyl group, 5-methoxy-2-methylphenyl group, 2,5-dibutoxyphenyl group, 2,5-diisopentyloxy group, 5-carbamoyl-2-methoxyphenyl group, 5-[(1,1-dimethyl)propyl]-2-phenoxyphenyl group, 2-hexyloxy-5-methanesulfonyl group, 5-(2,2-dimethylpropionyl)-2-methylphenyl group, 5-methoxy-2-(1-pyrrolyl)phenyl group, 5-chloro-2-(p-toluenesulfonyl)phenyl group, 2-chloro-5-(p-toluenesulfonyl)phenyl group, 2-fluoro-5-methanesulfonyl group, 2-methoxy-5-phenoxy group, 4-methylbiphenyl-3-yl group, 2-methoxy-5-(1-methyl-1-phenylethyl)phenyl group, 5-morpholino-2-nitrophenyl group, 5-fluoro-2-(1-imidazolyl)phenyl group, 2-butyl-5-nitrophenyl group, 5-[(1,1-dimethyl)]propyl-2-hydroxyphenyl group, 2-methoxy-5-methylphenyl group, 2,5-difluorophenyl group, 4-isopropyl-2-(trifluoromethyl)phenyl group, 2-nitro-4-(trifluoromethyl)phenyl group, 4-bromo-3-(trifluoromethyl)phenyl group, 4-bromo-2-(trifluoromethyl)phenyl group, 2-bromo-4-(trifluoromethyl)phenyl group, 4-fluoro-2-(trifluoromethyl)phenyl group, 4-isopropoxy-2-(trifluoromethyl)phenyl group, 4-cyano-2-(trifluoromethyl)phenyl group, 2,6-diisopropylphenyl group, 2,6-dimethylphenyl group, 3,4-dimethylphenyl group, 2,4-dichlorophenyl group, 2,3-dimethylphenyl group, indan-5-yl group, 2,4-dimethylphenyl group, 2,6-dichlorophenyl group, 4-bromo-2-(trifluoromethoxy)phenyl group, 3,4-ethylenedioxyphenyl group, 3-chloro-4-cyanophenyl group, 3-chloro-4-(trifluoromethoxy)phenyl group, 2-chloro-4-cyanophenyl group, 2,3-dichlorophenyl group, 4-isopropyl-3-methylphenyl group, 4-[(1,1-dimethyl)propyl]-2-hydroxyphenyl group, 3-chloro-2-cyanophenyl group, 2-cyano-4-methylphenyl group, 2,2-difluoro-1,3-benzodioxol-4-yl group, 2,2,3,3-tetrafluoro-1,4-benzodioxen-5-yl group, 3-chloro-4-(trifluoromethylsulfanyl)phenyl group, 2-nitro-4-(trifluoromethoxy)phenyl group, 2,2-difluoro-1,3-benzodioxol-5-yl group, 2-methyl-4-(trifluoromethoxy)phenyl group, 4-bromo-2-fluorophenyl group, 2,4-bis(methanesulfonyl)phenyl group, 2,2,3,3-tetrafluoro-1,4-benzodioxen-6-yl group, 2-benzoyl-4-chlorophenyl group, 2-bromo-4-fluorophenyl group, 3,4-dimethoxyphenyl group, 3,4-difluorophenyl group, 3-chloro-4-methoxyphenyl group, 2-chloro-4-nitrophenyl group, 2,4-difluorophenyl group, 2-benzoyl-5-methylphenyl group, 2-bromo-4-(trifluoromethoxy)phenyl group, 3,4-dihexyloxyphenyl group, 2,4-bis(trifluoromethyl)phenyl group, 4-cyano-2-(trifluoromethoxy)phenyl group, 2-(4-cyanophenoxy)-5-(trifluoromethyl)phenyl group, 2-(4-methoxyphenoxy)-5-(trifluoromethyl)phenyl group

When "an aryl group which may be substituted" in the aforementioned definition of E is "a di-substituted phenyl group," "a 2,5-di-substituted phenyl group," and "a 3,5-di-substituted phenyl group" are preferred.

When "an aryl group which may be substituted" in the aforementioned definition of E is "a 2,5-di-substituted phenyl group," preferred examples of the group include groups represented by the following substituent group δ -2e.
[Substituent Group δ -2e] 2,5-dimethoxyphenyl group, 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2,5-dichlorophenyl group, 2,5-bis[(1,1-dimethyl)ethyl]phenyl group, 5-[(1,1-dimethyl)ethyl]-2-methoxyphenyl group, 4-methoxybiphenyl-3-yl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 5-isopropyl-2-methylphenyl group, 2,5-diethoxyphenyl group, 2,5-dimethylphenyl group, 5-chloro-2-cyano group, 5-diethylsulfamoyl-2-methoxyphenyl group, 2-chloro-5-nitrophenyl group, 2-methoxy-5-(phenylcarbamoyl)phenyl group, 5-acetylamino-2-methoxyphenyl group, 5-methoxy-2-methylphenyl group, 2,5-dibutoxyphenyl group, 2,5-diisopentyloxy group, 5-carbamoyl-2-methoxyphenyl group, 5-[(1,1-dimethyl)propyl]-2-phenoxyphenyl group, 2-hexyloxy-5-methanesulfonyl group, 5-(2,2-dimethylpropionyl)-2-methylphenyl group, 5-methoxy-2-(1-pyrrolyl)phenyl group, 5-chloro-2-(p-toluenesulfonyl)phenyl group, 2-chloro-5-(p-toluenesulfonyl)phenyl group, 2-fluoro-5-methanesulfonyl group, 2-methoxy-5-phenoxy group, 2-methoxy-5-(1-methyl-I-phenylethyl)phenyl group, 5-morpholino-2-nitrophenyl group, 5-fluoro-2-(1-imidazolyl)phenyl group, 2-butyl-5-nitrophenyl group, 5-[(1,1-dimethyl)propyl]-2-hydroxyphenyl group, 2-methoxy-5-methylphenyl group, 2,5-difluorophenyl group, 2-benzoyl-5-methylphenyl group, 2-(4-cyanophenoxy)-5-(trifluoromethyl)phenyl group, 2-(4-methoxyphenoxy)-5-(trifluoromethyl)phenyl group

When "an aryl group which may be substituted" in the aforementioned definition of E is "a 2,5-di-substituted phenyl group," "a 2,5-di-substituted phenyl group wherein at least one of the said substituents is trifluoromethyl group" is more preferred, a group selected from the following substituent group δ -3e is further preferred, and 2,5-bis(trifluoromethyl)phenyl group is most preferred.
[Substituent Group δ -3e] 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 2-(4-cyanophenoxy)-5-(trifluoromethyl)phenyl group, 2-(4-methoxyphenoxy)-5-(trifluoromethyl)phenyl group

When "an aryl group which may be substituted" in the aforementioned definition of E is "a 3,5-di-substituted phenyl group," preferred examples of the group include groups represented by the following substituent group δ -4e.
[Substituent Group δ -4e] 3,5-bis(trifluoromethyl)phenyl group, 3,5-dichlorophenyl group, 3,5-bis[(1,1-dimethyl)ethyl]phenyl group, 3-fluoro-5-(trifluoromethyl)phenyl group, 3-bromo-5-(trifluoromethyl)phenyl group, 3-methoxy-5-(trifluoromethyl)phenyl group, 3,5-difluorophenyl group, 3,5-dinitrophenyl group, 3,5-dimethylphenyl group, 3,5-dimethoxyphenyl group, 3,5-bis(methoxycarbonyl)phenyl group, 3-methoxycarbonyl-5-(trifluoromethyl)phenyl group, 3-carboxy-5-(trifluoromethyl)phenyl group, 3,5-dicarboxyphenyl group

When "an aryl group which may be substituted" in the aforementioned definition of E is "a 3,5-di-substituted phenyl group," "a 3,5-di-substituted phenyl group wherein at least one of the said substituents is trifluoromethyl group" is more preferred, a group selected from the following substituent group δ -5e is further preferred, and 3,5-bis(trifluoromethyl)phenyl group is most preferred.
[Substituent Group δ -5e] 3,5-bis(trifluoromethyl)phenyl group, 3-fluoro-5-(trifluoromethyl)phenyl group, 3-bromo-5-(trifluoromethyl)phenyl group, 3-methoxy-5-(trifluoromethyl)phenyl group, 3-methoxycarbonyl-5-(trifluoromethyl)phenyl group, 3-carboxy-5-(trifluoromethyl)phenyl group

When "an aryl group which may be substituted" in the aforementioned definition of E is "a mono-substituted phenyl group," preferred examples of the group include groups represented by the following substituent group δ -6e.
[Substituent Group δ -6e] 4-methoxyphenyl group, 4-chlorophenyl group, 2-methoxyphenyl group, 2-(trifluoromethyl)phenyl group, 3-(trifluoromethyl)phenyl group, 4-(trifluoromethyl)phenyl group, 3-chlorophenyl group, biphenyl-3-yl group, 3-acetylphenyl group, 3-(acetylamino)phenyl group, 3-carbamoylphenyl group, 3-methylcarbomoylphenyl group, 4-methylphenyl group, 3-(trifluoromethoxy)phenyl group, 2-benzylphenyl group, 4-(trifluoromethoxy)phenyl group, 4-[(1,1-dimethyl)ethyl]phenyl group, 3-isopropoxyphenyl group, 4-isopropoxyphenyl group, 4-hexylphenyl group, 3-methylphenyl group, 4-cyclohexylphenyl group, 4-benzylphenyl group, 2-chlorophenyl group, 2-methylphenyl group, 4-butylphenyl group, 4-benzyloxyphenyl group, 3-benzylphenyl group, 4-hexyloxyphenyl group, 3-isopropylphenyl group, 4-cyanophenyl group, 3-cyanophenyl group, 4-(ethoxycarbonylmethyl)phenyl group, 3-(trifluoromethylsulfanyl)phenyl group, 4-(trifluoromethylsulfanyl)phenyl group, 4-(trifluoromethanesulfonyl)phenyl group, 3-ethynylphenyl group, 4-(1-methylpropyl)phenyl group, 3-benzoylphenyl group, 3-methoxyphenyl group, 4-(acetylamino)phenyl group, 4-sulfamoylphenyl group, 4-(difluoromethoxy)phenyl group, 3-methylsulfanylphenyl group, 4-methanesulfonylphenyl group, 3-(butylsulfamoyl)phenyl group, 3-benzyloxyphenyl group, 4-(p-toluenesulfonylamino)phenyl group, 4-morpholinophenyl group, 3-[(1,1-dimethyl)ethyl]phenyl group, 3-(5-methylfuran-2-yl)phenyl group, 3-sulfamoylphenyl group, 3-(trifluoromethanesulfonyl)phenyl group, 3-hexyloxyphenyl group, 4-acetylphenyl group, biphenyl-2-yl group, biphenyl-4-yl group, 3-[5-phenyl-3-(trifluoromethyl)pyrazol-1-yl]phenyl group, 3-{5-[(1,1-dimethyl)ethyl]-3-(trifluoromethyl)pyrazol-1-yl}phenyl group, 4-[3,5-bis(trifluoromethyl)pyrazol-1-yl]phenyl group, 3-[3,5-bis(trifluoromethyl)pyrazol-1-yl]phenyl group, 4-[5-phenyl-3-(trifluoromethyl)pyrazol-1-yl]phenyl group

When "an aryl group which may be substituted" in the aforementioned definition of E is "a phenyl group which has three or more substituents," preferred examples of the group include groups represented by the following substituent group δ -7e.
[Substituent Group δ -7e] 3,5-bis(trifluoromethyl)-2-bromophenyl group, 3,4,5-trichlorophenyl group, 3,5-dichloro-4-hydroxyphenyl group, pentafluorophenyl group, 3,5,5,8,8-pentamethyl-5,6,7,8-tetrahydronaphthalen-2-yl group, 3,5-bis(trifluoromethyl)-2-methylphenyl group, 2,6-dichloro-4-(trifluoromethyl)phenyl group, 2,4-dimethoxy-5-(trifluoromethyl)phenyl group, 2,4-difluoro-5-(trifluoromethyl)phenyl group, 4-chloro-2-(4-chlorobenzenesulfonyl)-5-(trifluoromethyl)phenyl group, 5-chloro-2-nitro-4-(trifluoromethyl)phenyl group, 2,3-difluoro-4-(trifluoromethyl)phenyl group,, 2,3,5,6-tetrafluoro-4-(trifluoromethyl)phenyl group, 2,4,6-trimethylphenyl group, 2-cyano-4,5-dimethoxyphenyl group, 2,4-dichloro-5-isopropoxyphenyl group, 2,3,5-trifluorophenyl group, 2,4,5-trichlorophenyl group, 5-ethoxy-4-fluoro-2-nitrophenyl group

When "an aryl group which may be substituted" in the aforementioned definition of E is "a naphthyl group which may be substituted," preferred examples of the group include 1-naphthyl group, 4-methoxynaphthalen-2-yl group, and 4-hydroxy-3-methylnaphthalen-1-yl group.

Examples of the "heteroaryl group" in "a heteroaryl group which may be substituted" in the definition of E include similar groups to the "monocyclic heteroaryl group" and "fused polycyclic heteroaryl group" in the definition of the aforementioned "heterocyclic group." A 5 to 13-membered heteroaryl group is preferred, and preferred examples of the group include thienyl group, pyrazolyl group, oxazolyl group, 1,3,4-thiadiazolyl group, pyridyl group, pyrimidinyl group, indolyl group, quinolyl group, carbazolyl group, thiazolyl group, and pyrazinyl group.

A 5-membered heteroaryl group is more preferred as the "heteroaryl group" in "a heteroaryl group which may be substituted" in the definition of E. Thienyl group, pyrazolyl group, oxazolyl group, 1,3,4-thiadiazolyl group, and thiazolyl group are further preferred, and thiazolyl group is most preferred.

Examples of the substituent in the definition of "a heteroaryl group which may be substituted" in the aforementioned definition of E include similar groups to the substituent explained for the definition "which may be substituted." The position of substituents existing on the heteroaryl group is not particularly limited, and when two or more substituents exist, they may be the same or different.

When "a heteroaryl group which may be substituted" in the aforementioned definition of E is "a thiazolyl group which may be substituted," "a thiazol-2-yl group which may be substituted" is preferred, and "a mono-substituted thiazol-2-yl group" and "a di-substituted thiazol-2-yl group" are more preferred. "A di-substituted thiazol-2-yl group" is further preferred, and "a 4,5-disubstituted thiazol-2-yl group" is most preferred.

When "a heteroaryl group which may be substituted" in the aforementioned definition of E is "a 4,5-disubstituted thiazol-2-yl group," a group selected from the following substituent group δ -8e is preferred, and 4-[(1,1-dimethyl)ethyl]-5-[(2,2-dimethyl)propionyl]thiazol-2-yl group is most preferred. [Substituent Group δ-8e] 5-bromo-4-[(1,1-dimethyl)ethyl]thiazol-2-yl group, 5-bromo-4-(trifluoromethyl)thiazol-2-yl group, 5-cyano-4-[(1,1-dimethyl)ethyl]thiazol-2-yl group, 5-methylthiazol-2-yl group, 4,5-dimethylthiazol-2-yl group, 5-methyl-4-phenylthiazol-2-yl group, 5-(4-fluorophenyl)-4-methylthiazol-2-yl group, 4-methyl-5-[3-(trifluoromethyl)phenyl]thiazol-2-yl group, 4-[(1,1-dimethyl)ethyl]-5-ethylthiazol-2-yl group, 4-ethyl-5-phenylthiazol-2-yl group, 4-isopropyl-5-phenylthiazol-2-yl group, 4-butyl-5-phenylthiazol-2-yl group, 4-[(1,1-dimethyl)ethyl]-5-[(2,2-dimethyl)propionyl]thiazol-2-yl group, 4-[(1,1-dimethyl)ethyl]-5-(ethoxycarbonyl)thiazol-2-yl group, 4-[(1,1-dimethyl)ethyl]-5-piperidinothiazol-2-yl group, 4-[(1,1-dimethyl)ethyl]-5-morpholinothiazol-2-yl group, 4-[(1,1-dimethyl)ethyl]-5-(4-methylpiperazin-1-yl)thiazol-2-yl group, 4-[(1,1-dimethyl)ethyl]-5-(4-phenylpiperazin-1-yl)thiazol-2-yl group, 5-carboxymethyl-4-phenylthiazol-2-yl group, 4,5-diphenylthiazol-2-yl group, 4-benzyl-5-phenylthiazol-2-yl group, 5-phenyl-4-(trifluoromethyl)thiazol-2-yl group, 5-acetyl-4-phenylthiazol-2-yl group, 5-benzoyl-4-phenylthiazol-2-yl group, 5-ethoxycarbonyl-4-phenylthiazol-2-yl group, 5-ethoxycarbonyl-4-(pentafluorophenyl)thiazol-2-yl group, 5-methylcarbamoyl-4-phenylthiazol-2-yl group, 5-ethylcarbamoyl-4-phenylthiazol-2-yl-group, 5-isopropylcarbamoyl-4-phenylthiazol-2-yl group, 5-(2-phenylethyl)carbamoyl-4-phenylthiazol-2-yl group, 5-ethoxycarbonyl-4-(trifluoromethyl)thiazol-2-yl group, 5-carboxy-4-[(1,1-dimethyl)ethyl]thiazol-2-yl group, 5-(ethoxycarbonyl)methyl-4-phenylthiazol-2-yl group, 5-carboxy-4-phenylthiazol-2-yl group, 5-propylcarbamoyl-4-phenylthiazol-2-yl group.

When "a heteroaryl group which may be substituted" in the aforementioned definition of E is "a mono-substituted thiazol-2-yl group," preferred examples of the group include groups represented by the following substituent group δ -9e. [Substituent Group δ -9e] 4-[(1,1-dimethyl)ethyl]thiazol-2-yl group, 4-phenylthiazol-2-yl group, 4-[3,5-bis(trifluoromethyl)phenyl]thiazol-2-yl group, 4-(2,4-dichlorophenyl)thiazol-2-yl group, 4-(3,4-dichlorophenyl)thiazol-2-yl group, 4-[4-(trifluoromethyl)phenyl]thiazol-2-yl group, 4-(2,5-difluorophenyl)thiazol-2-yl group, 4-(4-methoxyphenyl)thiazol-2-yl group, 4-[3-(trifluoromethyl)phenyl]thiazol-2-yl group, 4-(pentafluorophenyl)thiazol-2-yl group

The compounds represented by the aforementioned general formula (I) may form salts. Examples of pharmacologically acceptable salts include, when acidic groups exist, metal salts such as lithium salt, sodium salt, potassium salt, magnesium salt, calcium salts, or ammonium salts such as ammonium salt, methylammonium salt, dimethylammonium salt, trimethylammonium salt, dicyclohexylammonium salt, and when basic groups exist, mineral acid salts such as hydrochloride, oxalate, hydrosulfate, nitrate, phosphate, or organic acid salts such as methane sulfonate, benzene sulfonate, para-toluene sulfonate, acetate, propionate, tartrate, fumarate, maleate, malate, oxalate, succinate, citrate, benzoate, mandelate, cinnamate, lactate. Salts may sometimes be formed with amino acids such as glycine. As active ingredients of the medicament of the present invention, pharmacologically acceptable salts may also be suitably used.

The compounds or salts thereof represented by the aforementioned general formula (I) may exist as hydrates or solvates. As active ingredients of the medicament of the present invention, any of the aforementioned substances may be used. Furthermore, the compounds represented by the aforementioned general formula (I) may sometimes have one or more asymmetric carbons, and may exist as steric isomers such as optically active substance and diastereomer. As active ingredients of the medicament of the present invention, pure forms of stereoisomers, arbitrary mixture of enantiomers or diastereomers, and racemates may be used.

Furthermore, when the compounds represented by the general formula (I) has, for example, 2-hydroxypyridine form, the compounds may exist as 2-pyridone form which is a tautomer. As active ingredients of the medicament of the present invention, pure forms of tautomers or a mixture thereof may be used. When the compounds represented by the general formula (I) have olefinic double bonds, the configuration may be in either E or Z, and as active ingredients of the medicament of the present invention, geometrical isomer in either of the configurations or a mixture thereof may be used.

Examples of the compounds encompassed within the general formula (I) as active ingredients of the medicaments of the present invention are shown below. However, the active ingredients of the medicaments of the present invention are not limited to the compound set out below.

The abbreviations used in the following tables have the following meanings.
Me: methyl group, Et: ethyl group.

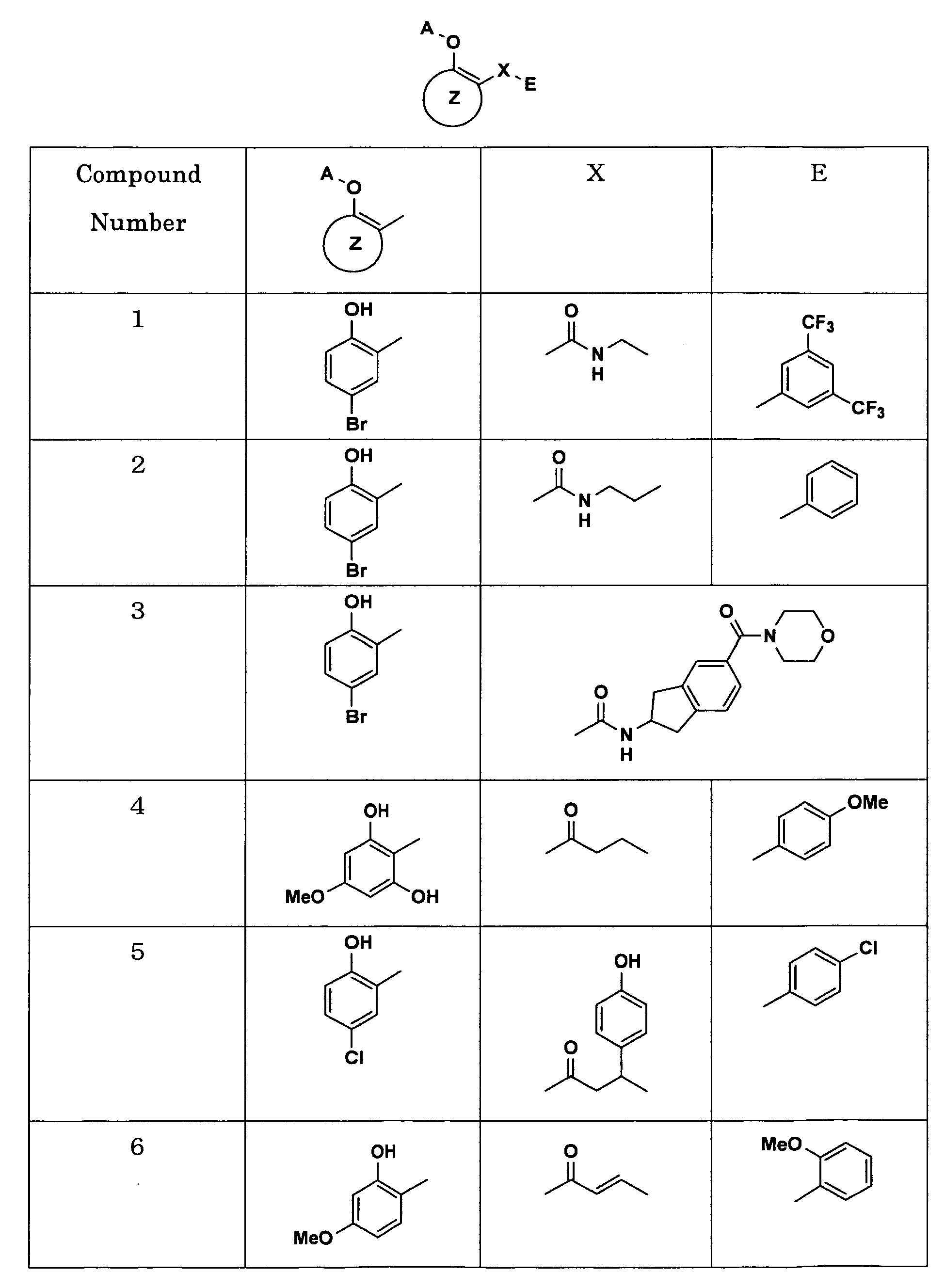

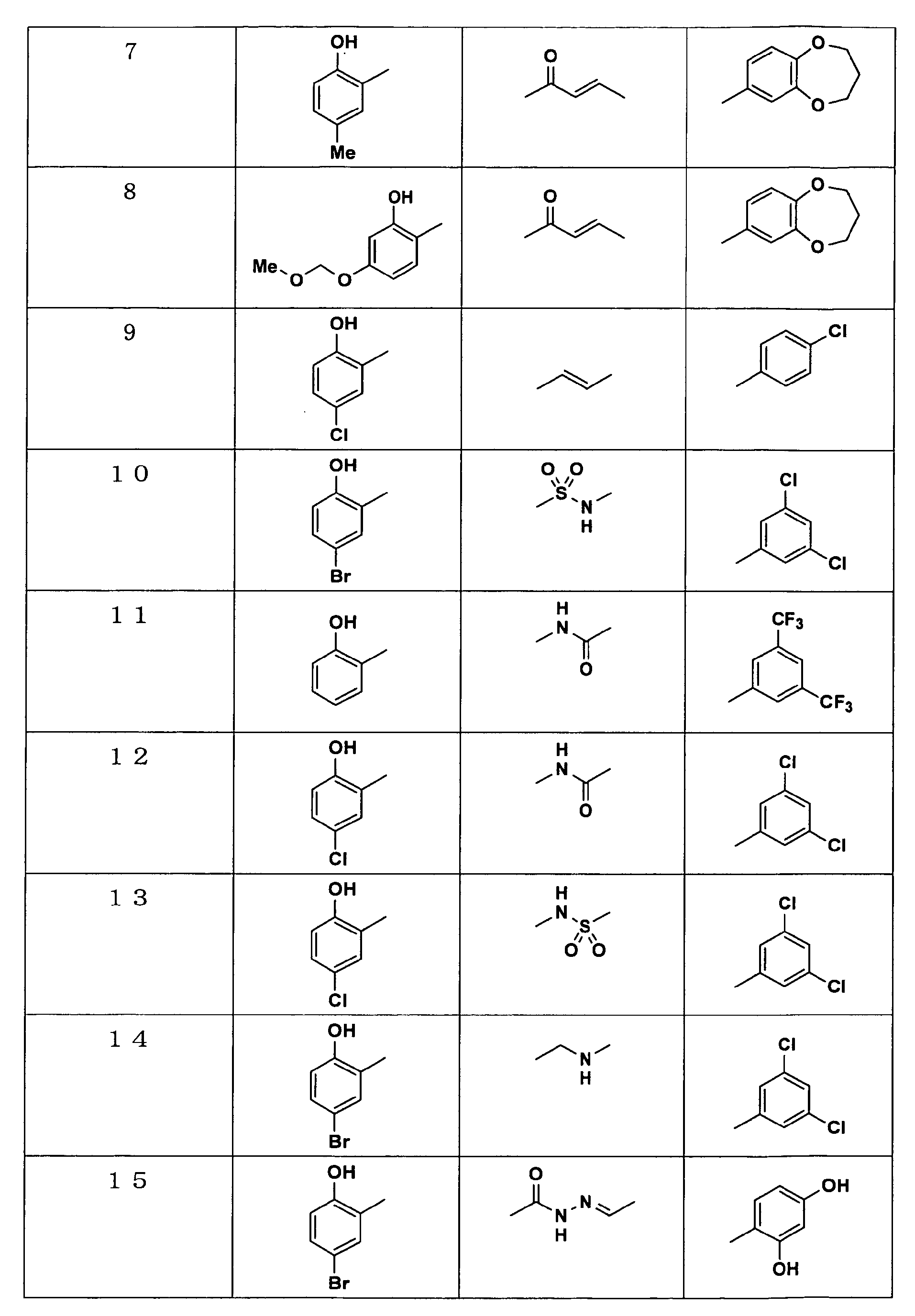

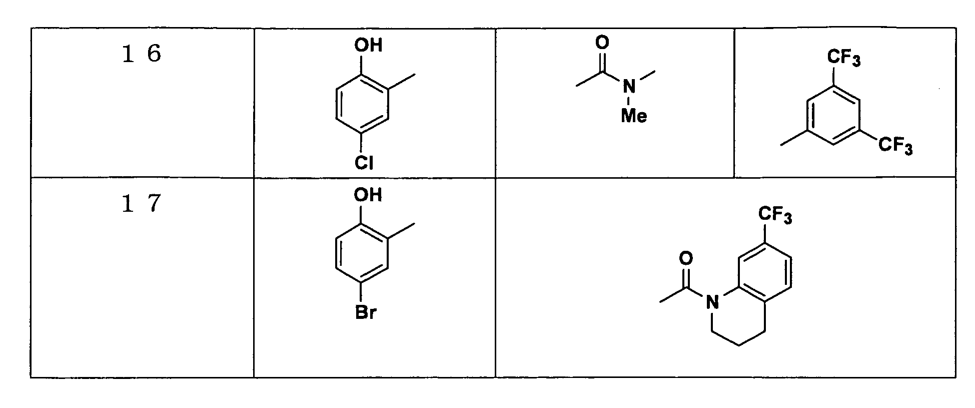

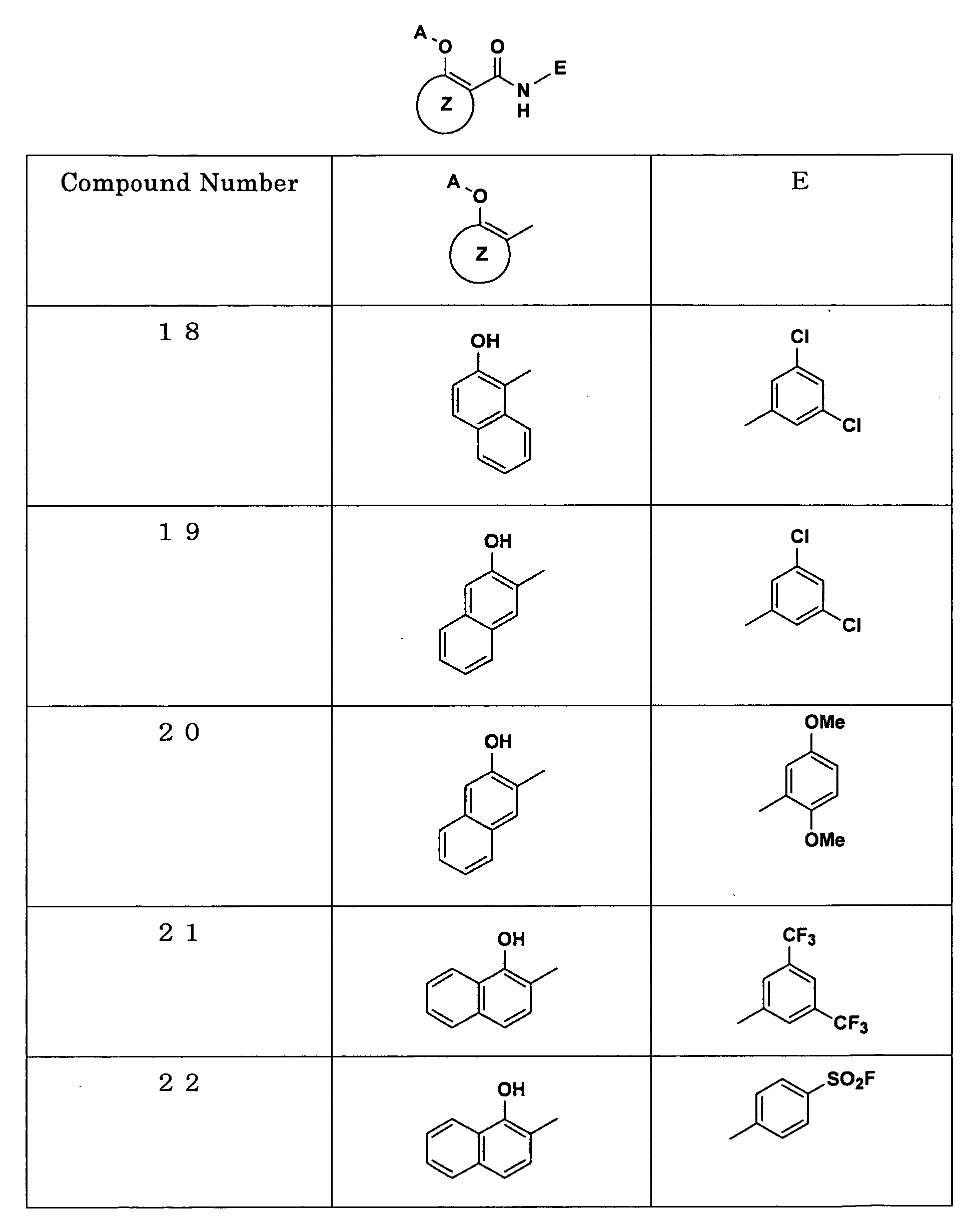

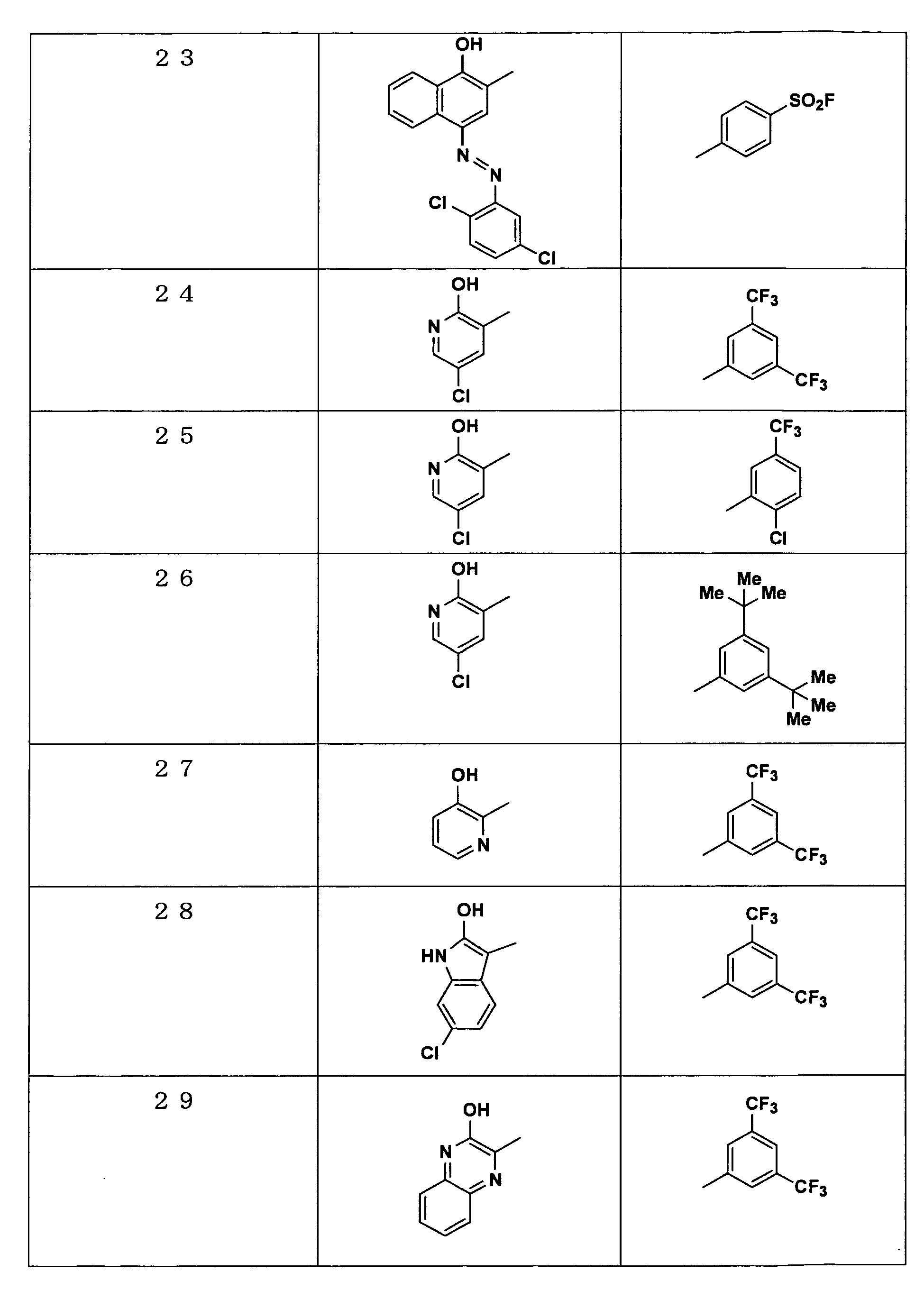

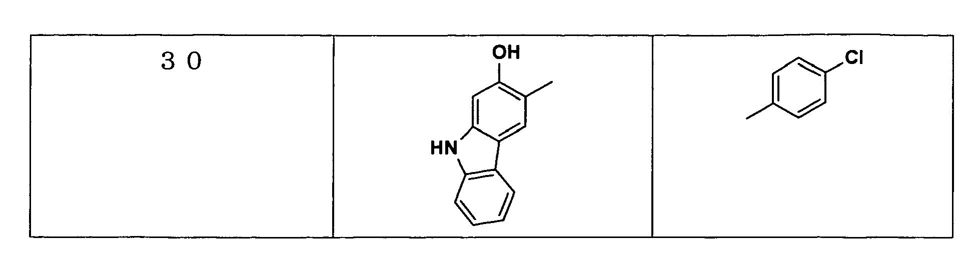

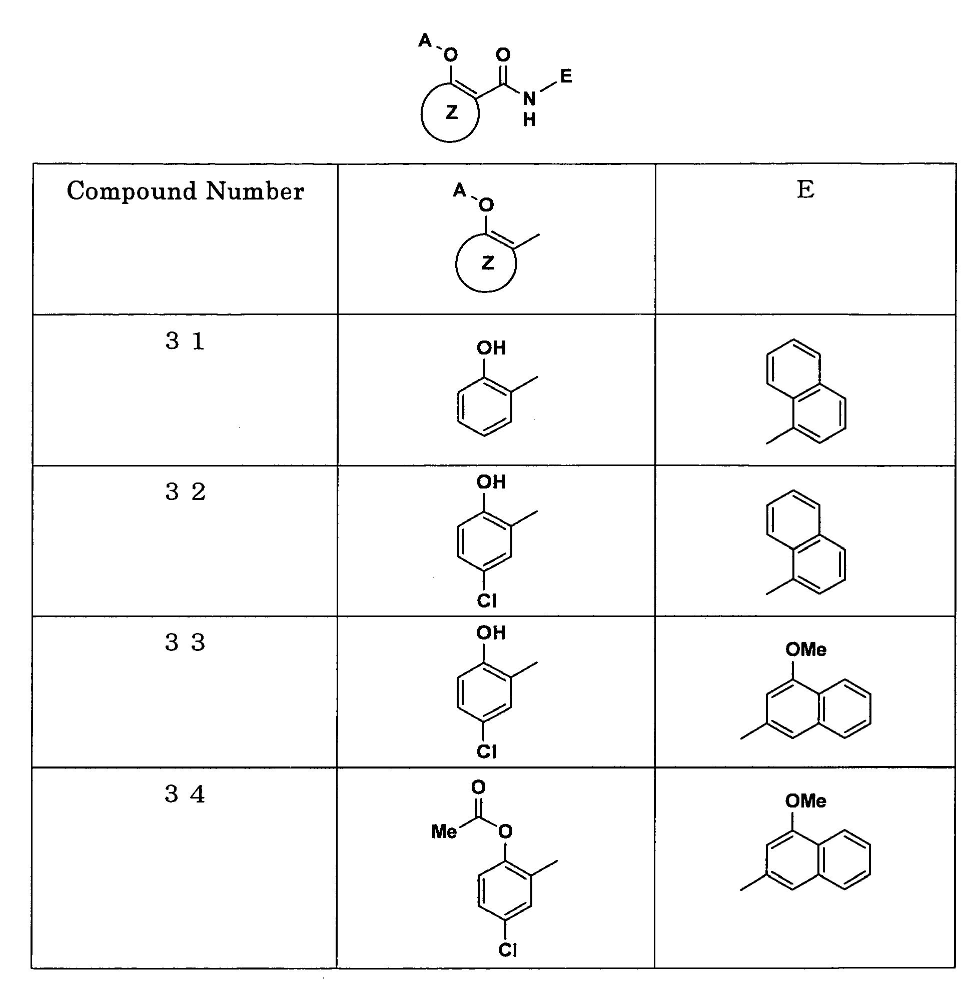

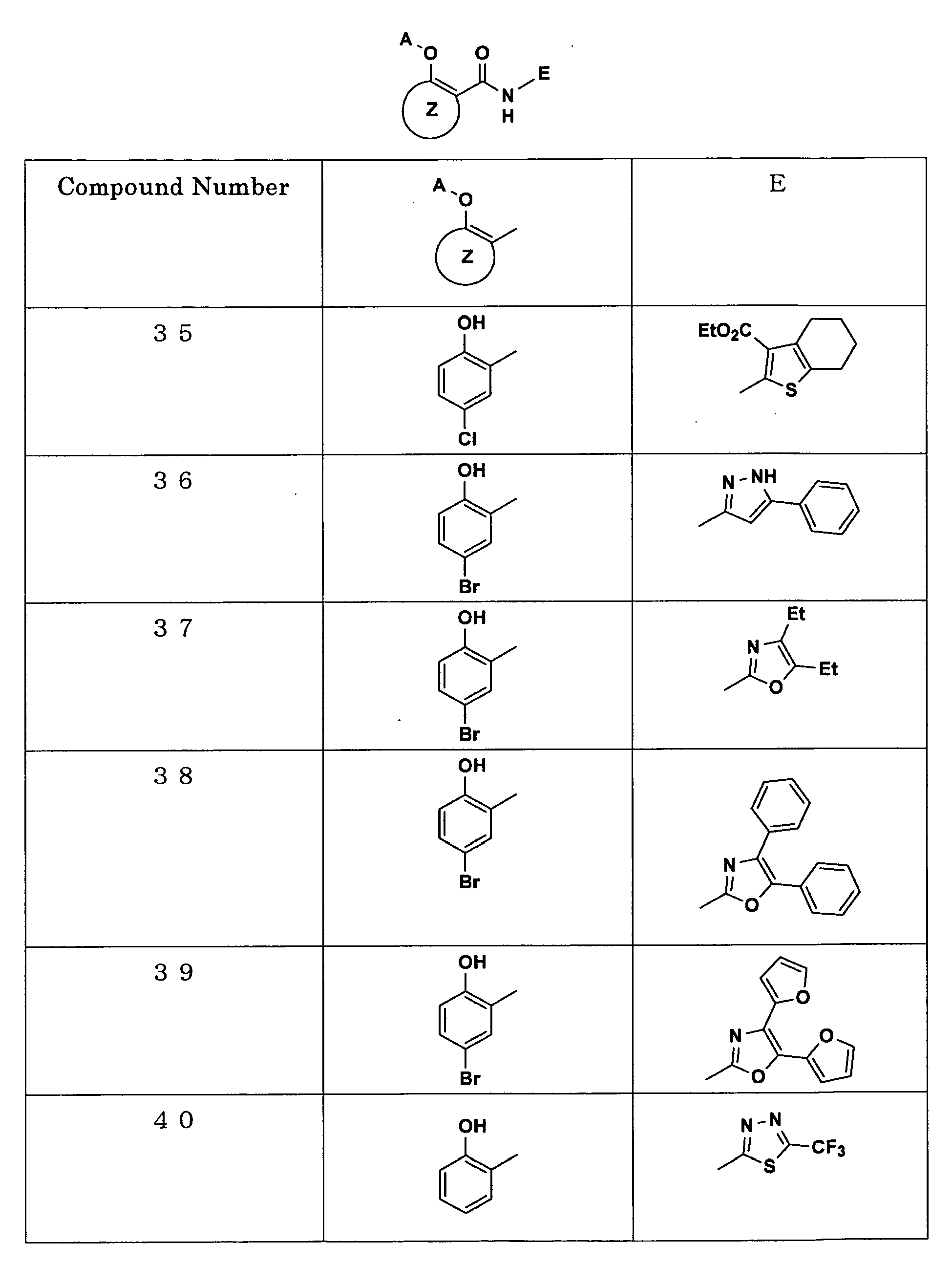

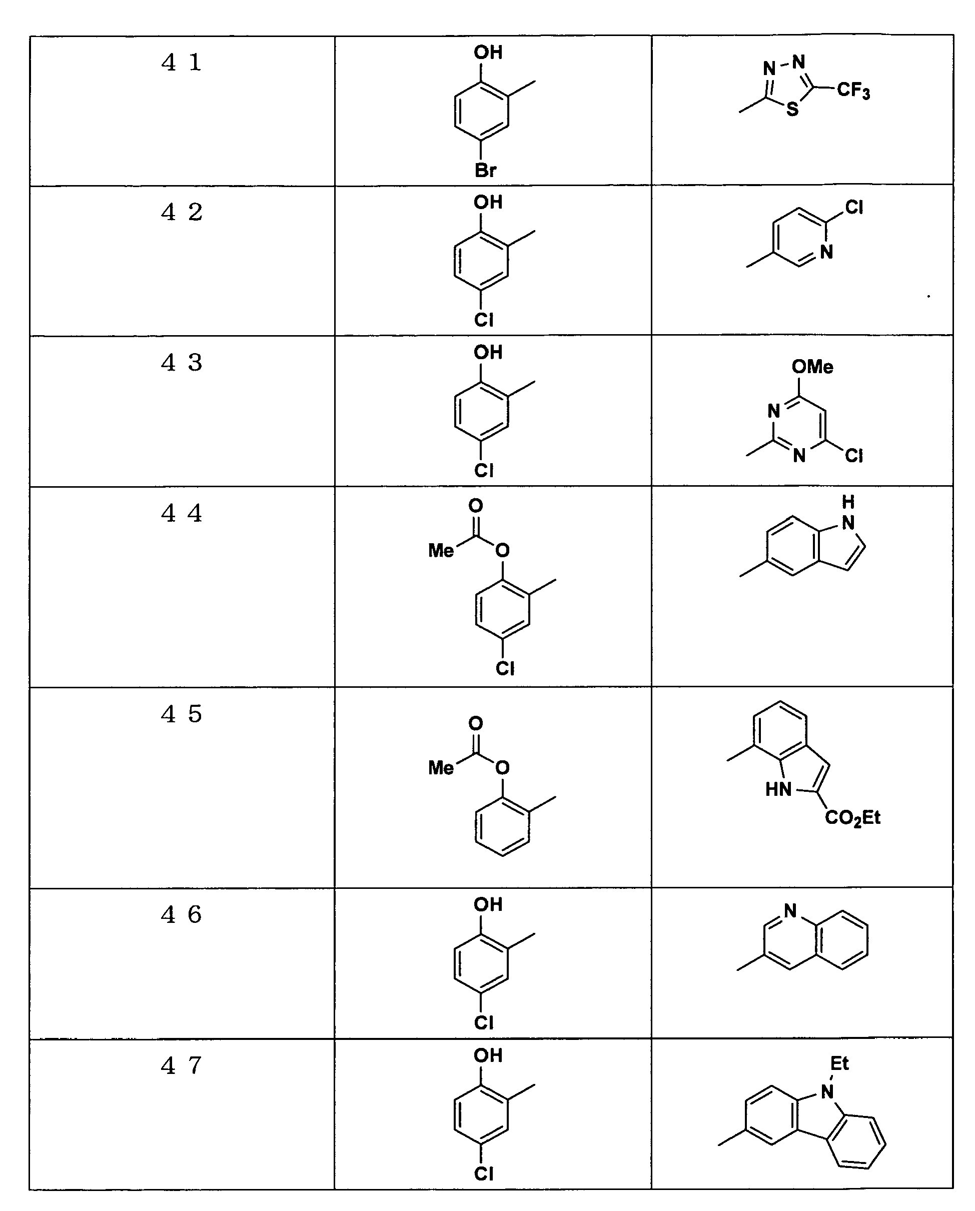

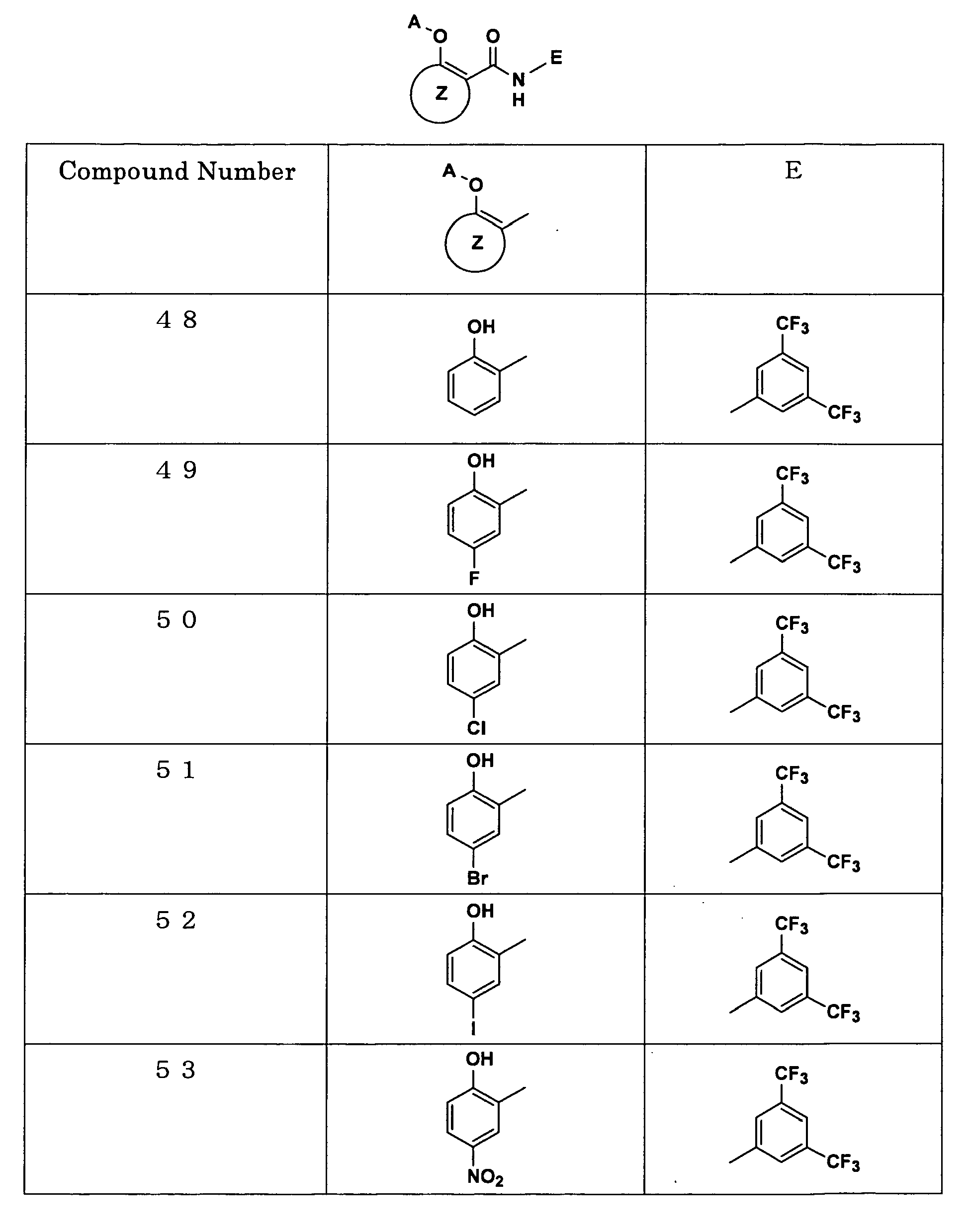

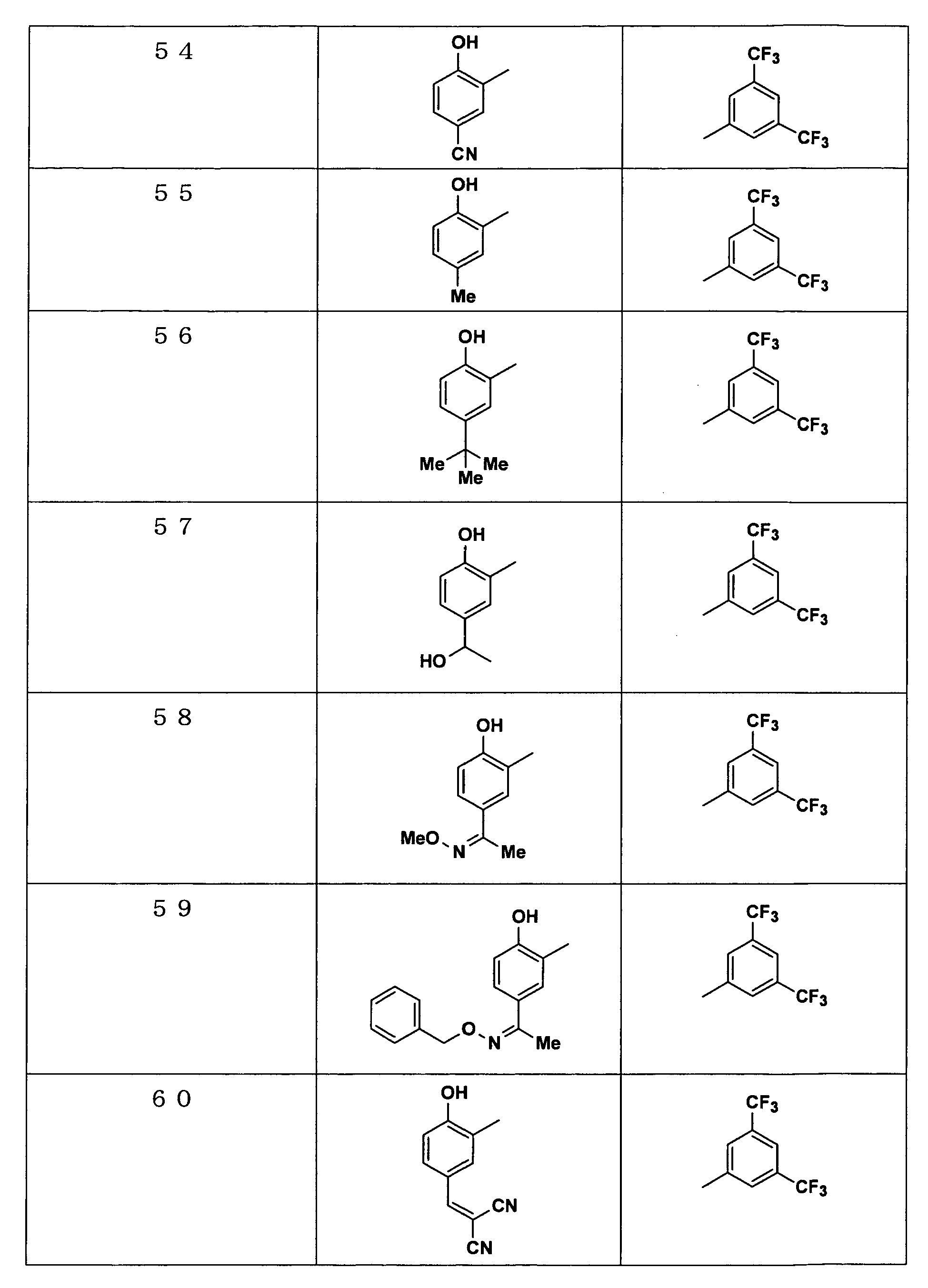

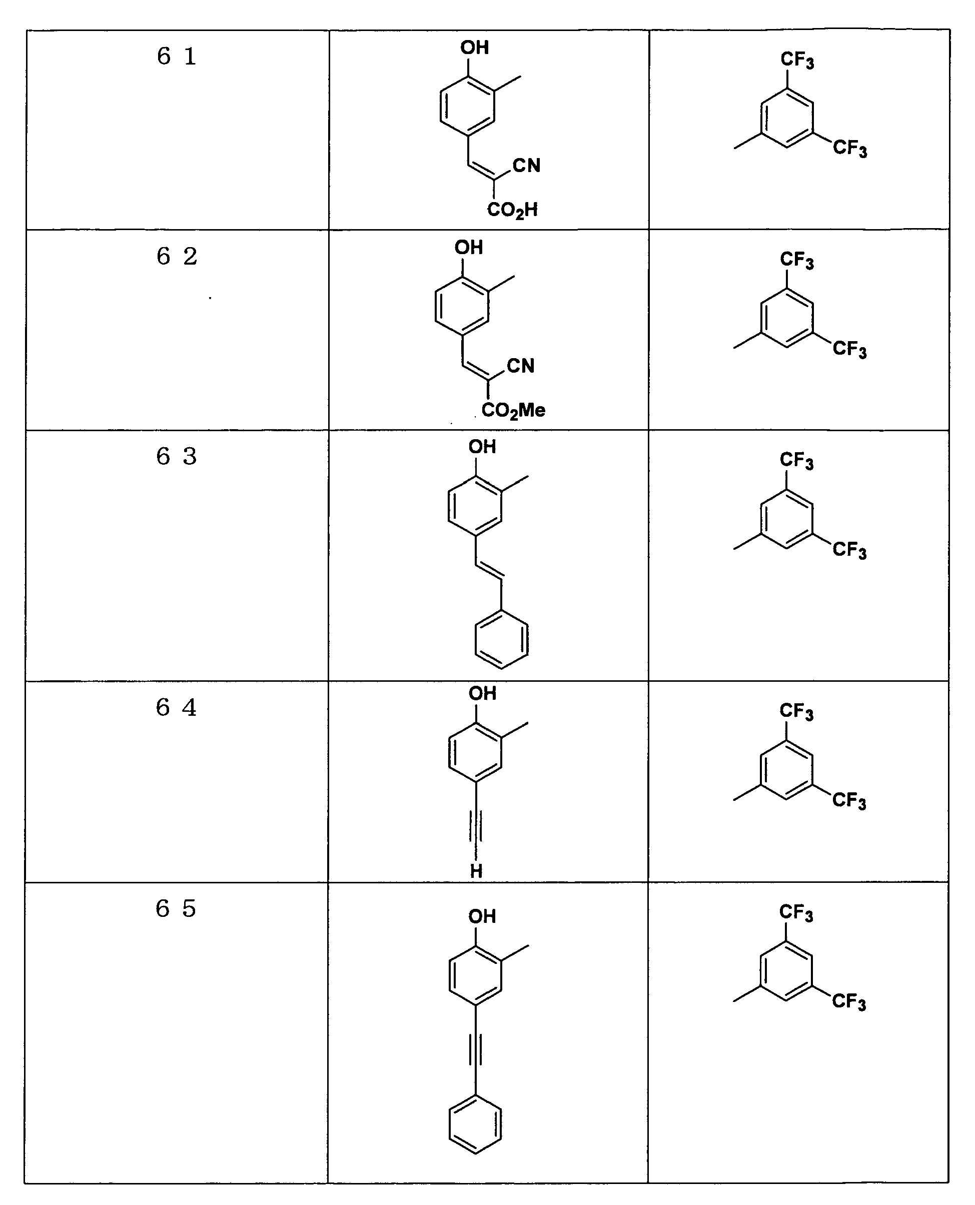

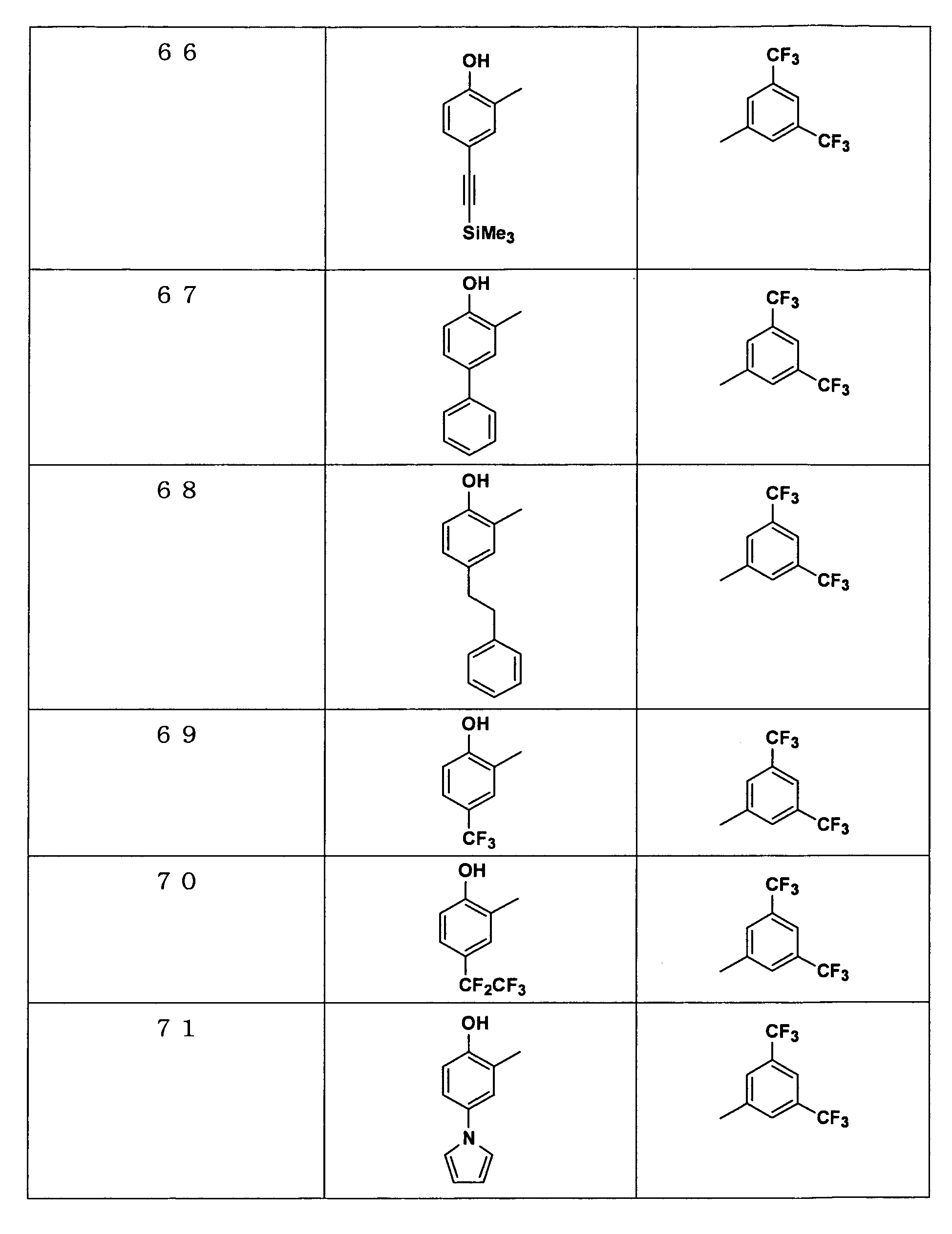

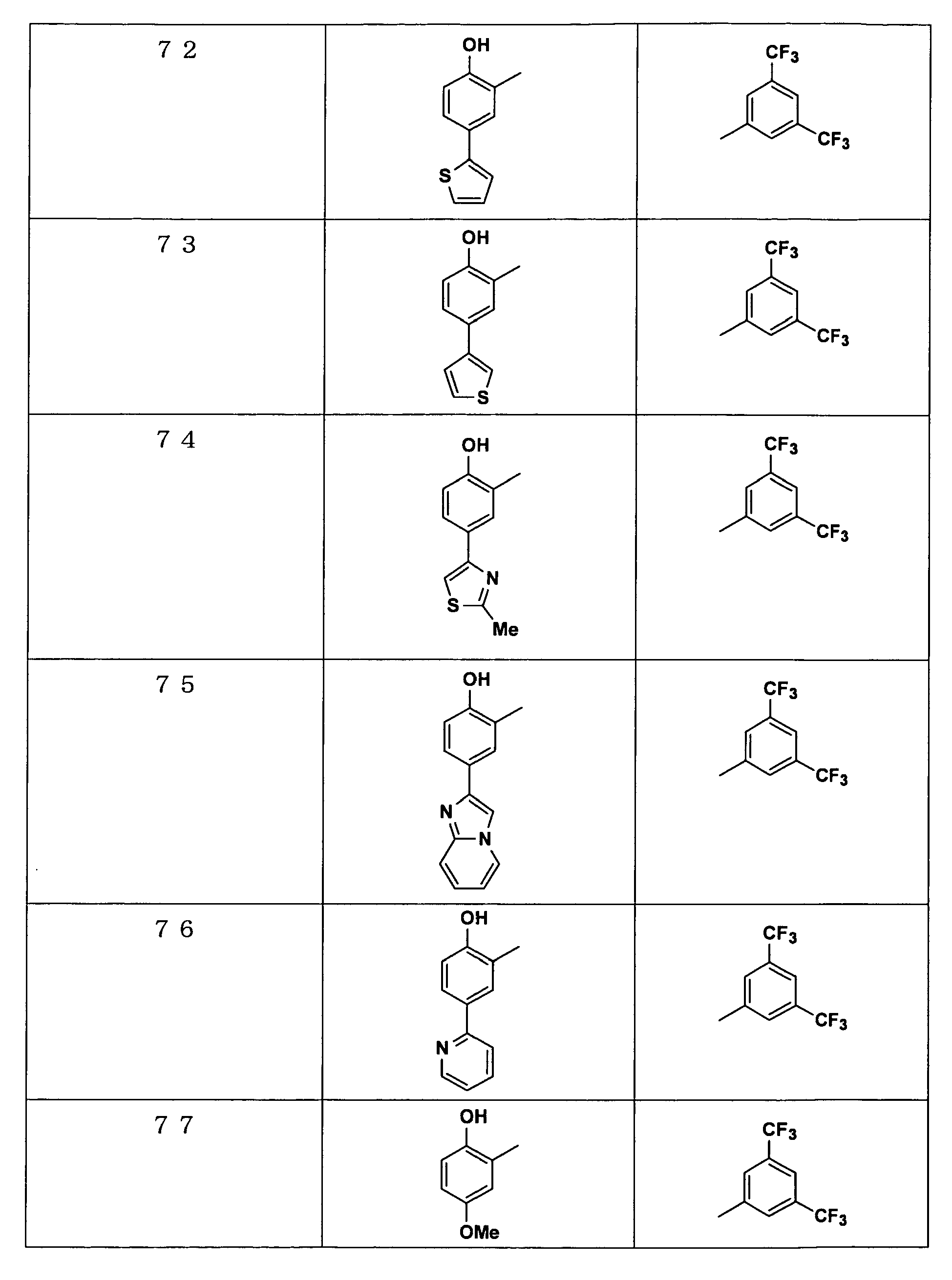

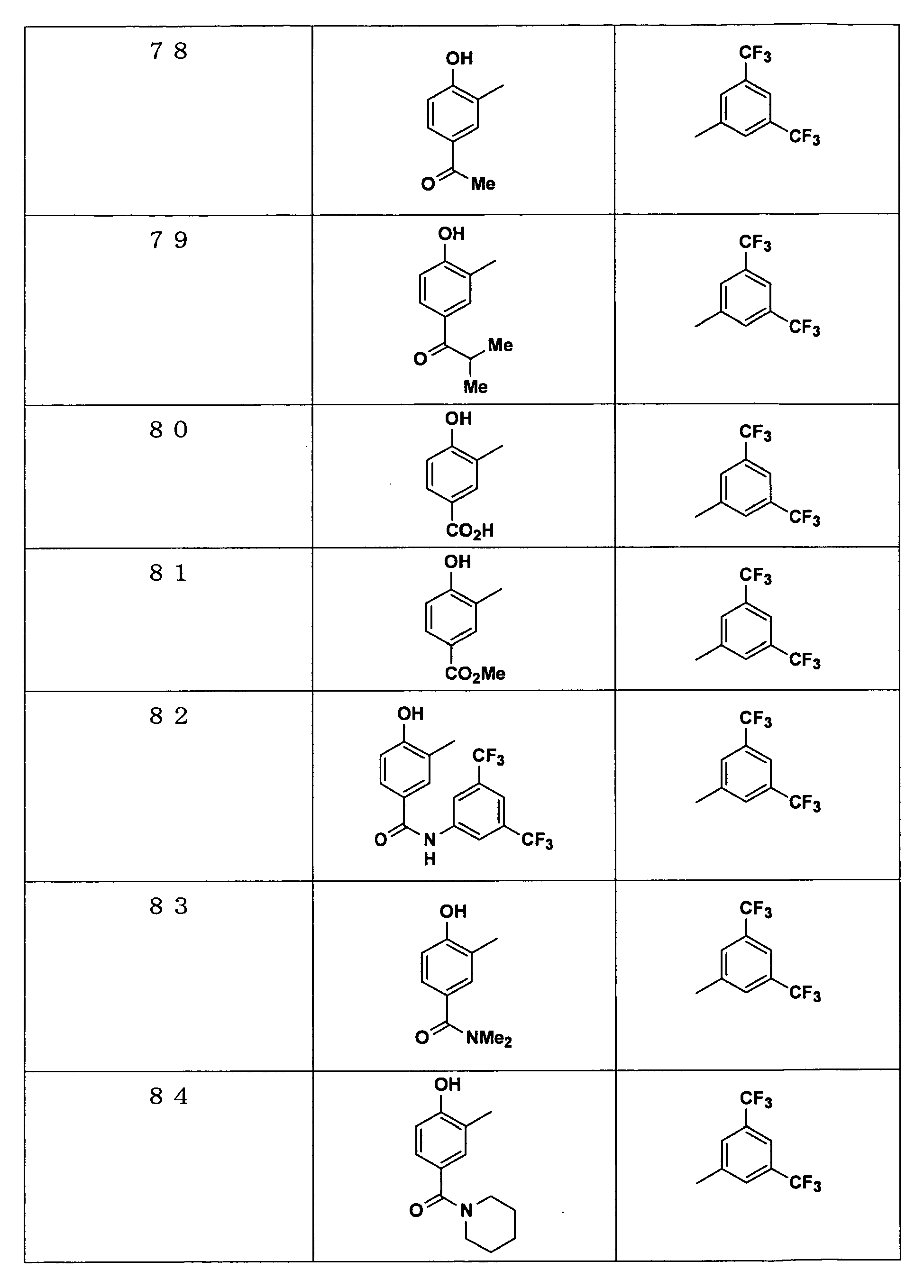

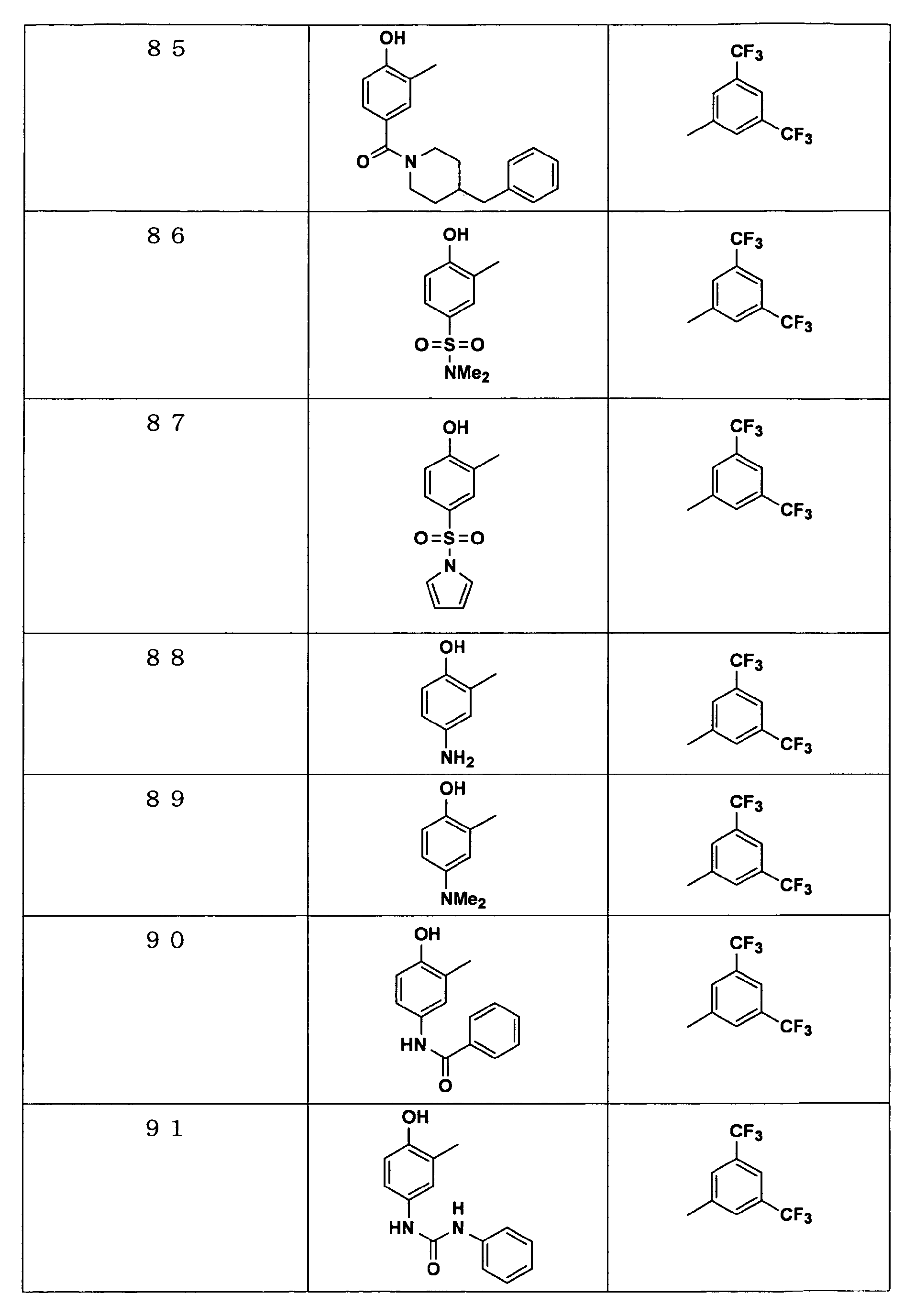

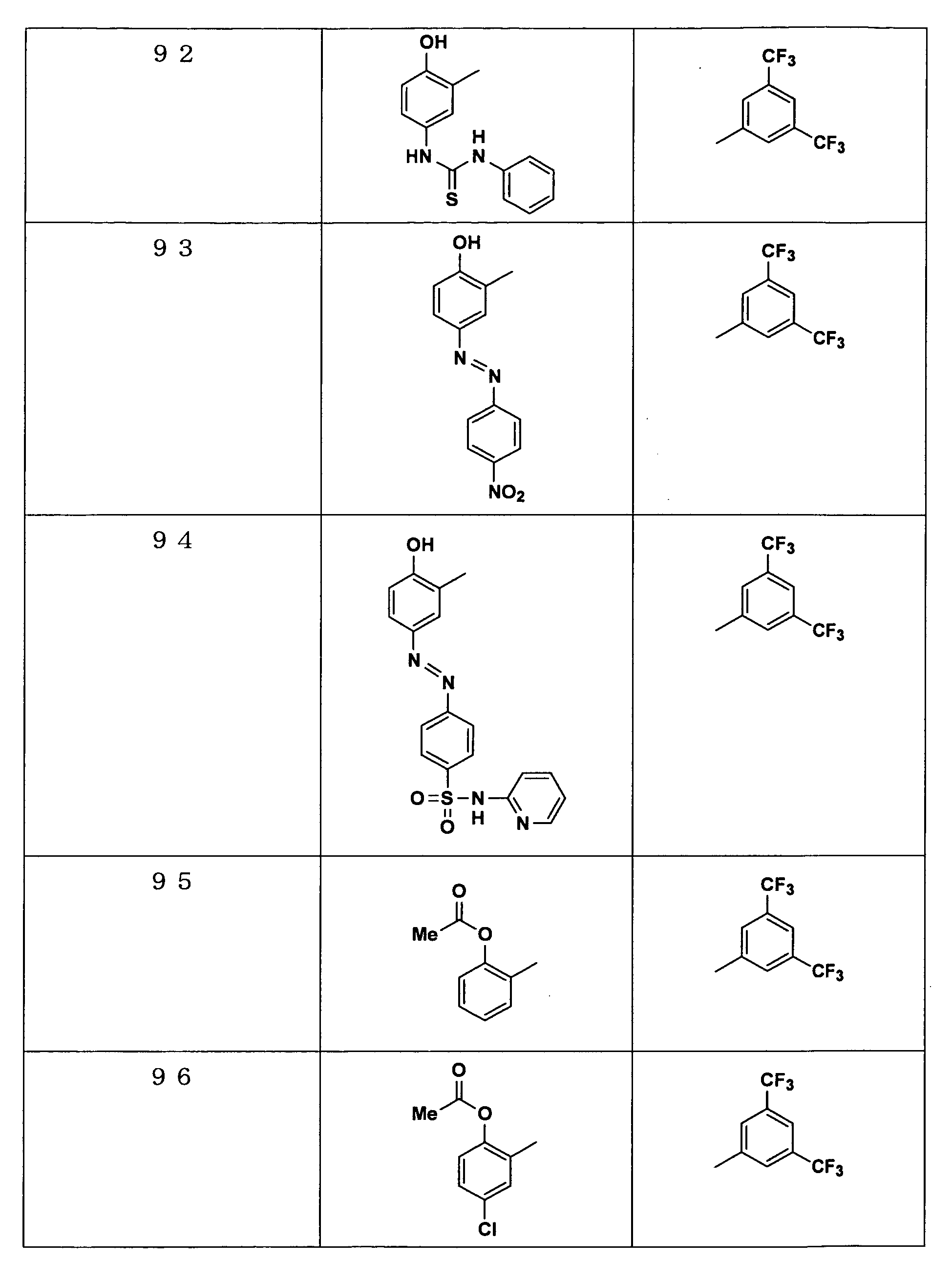

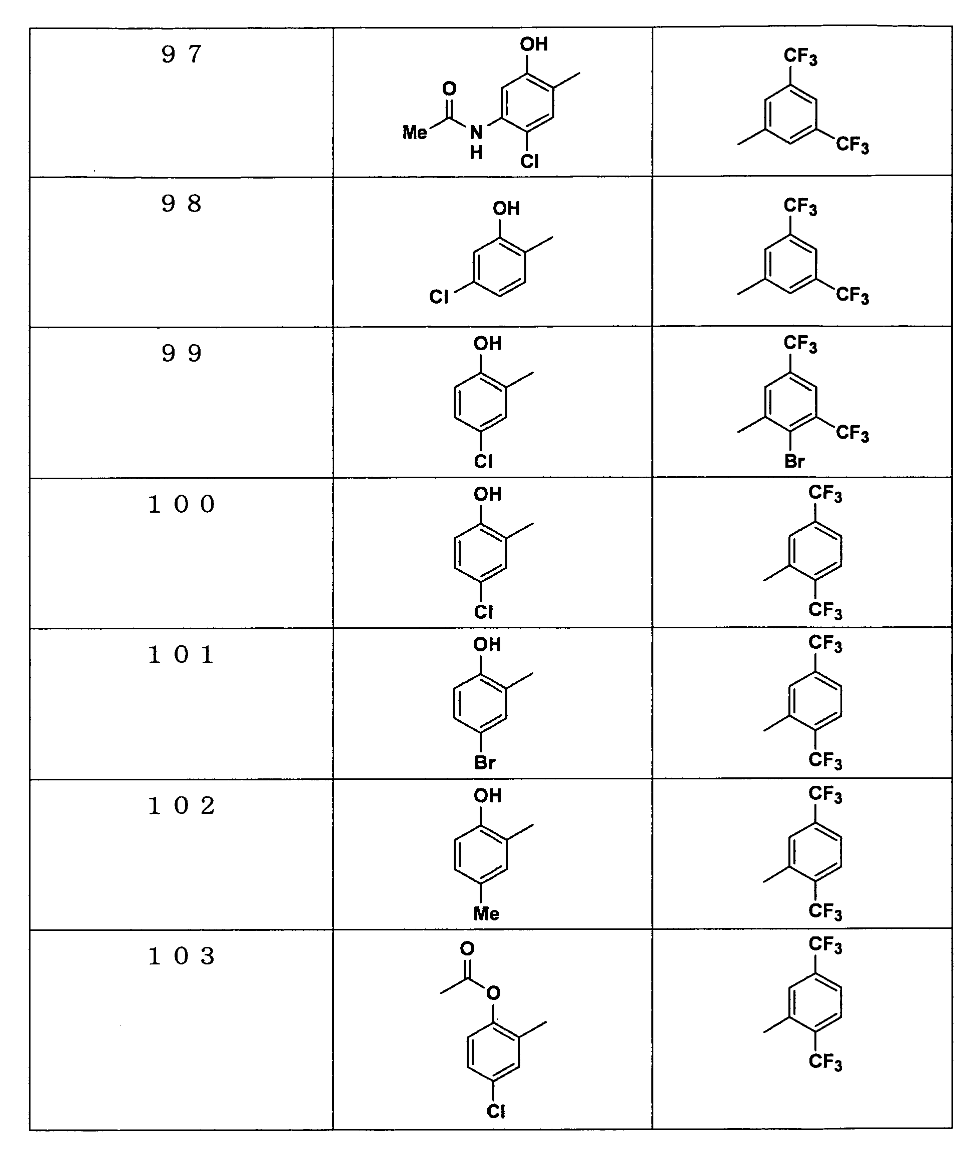

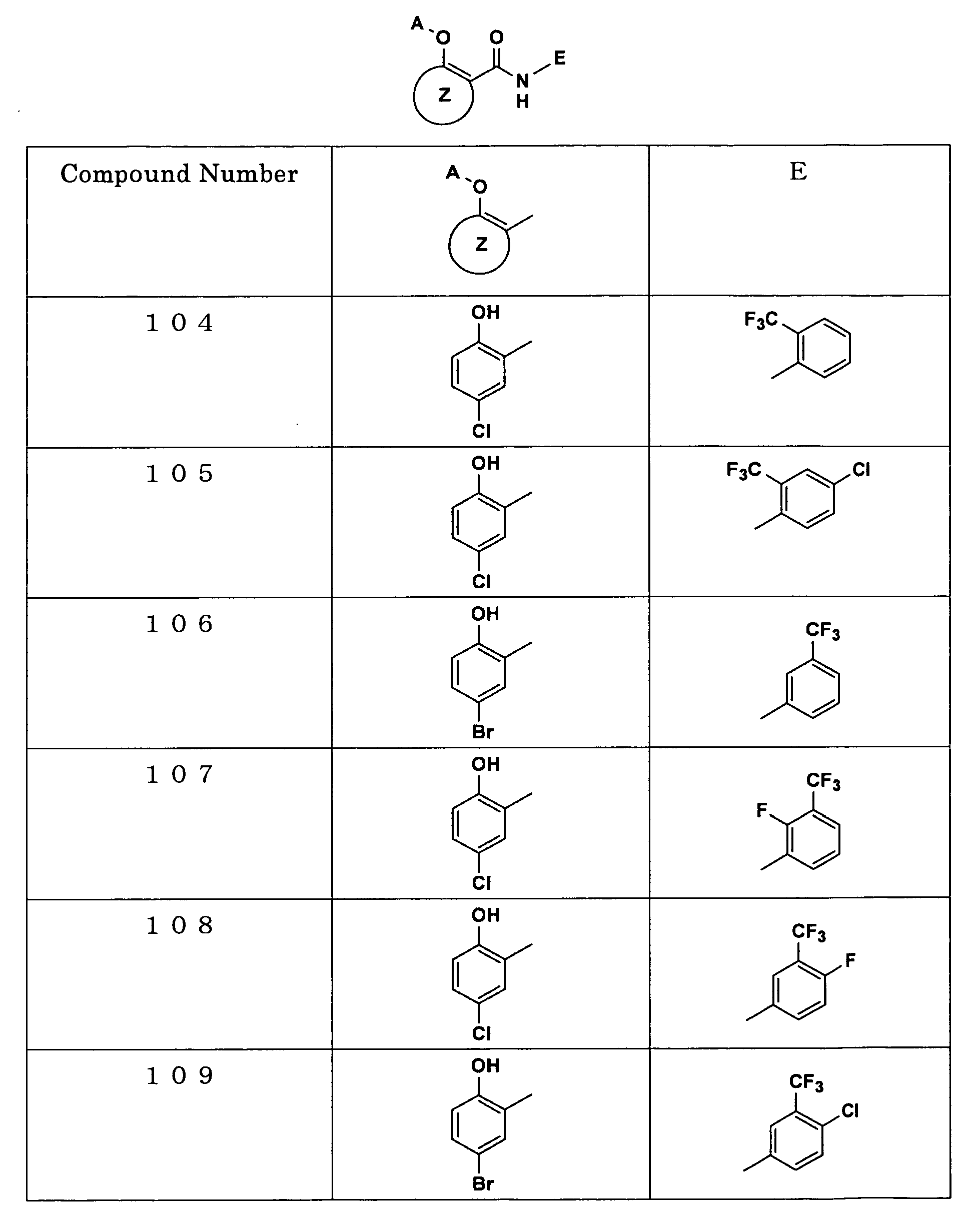

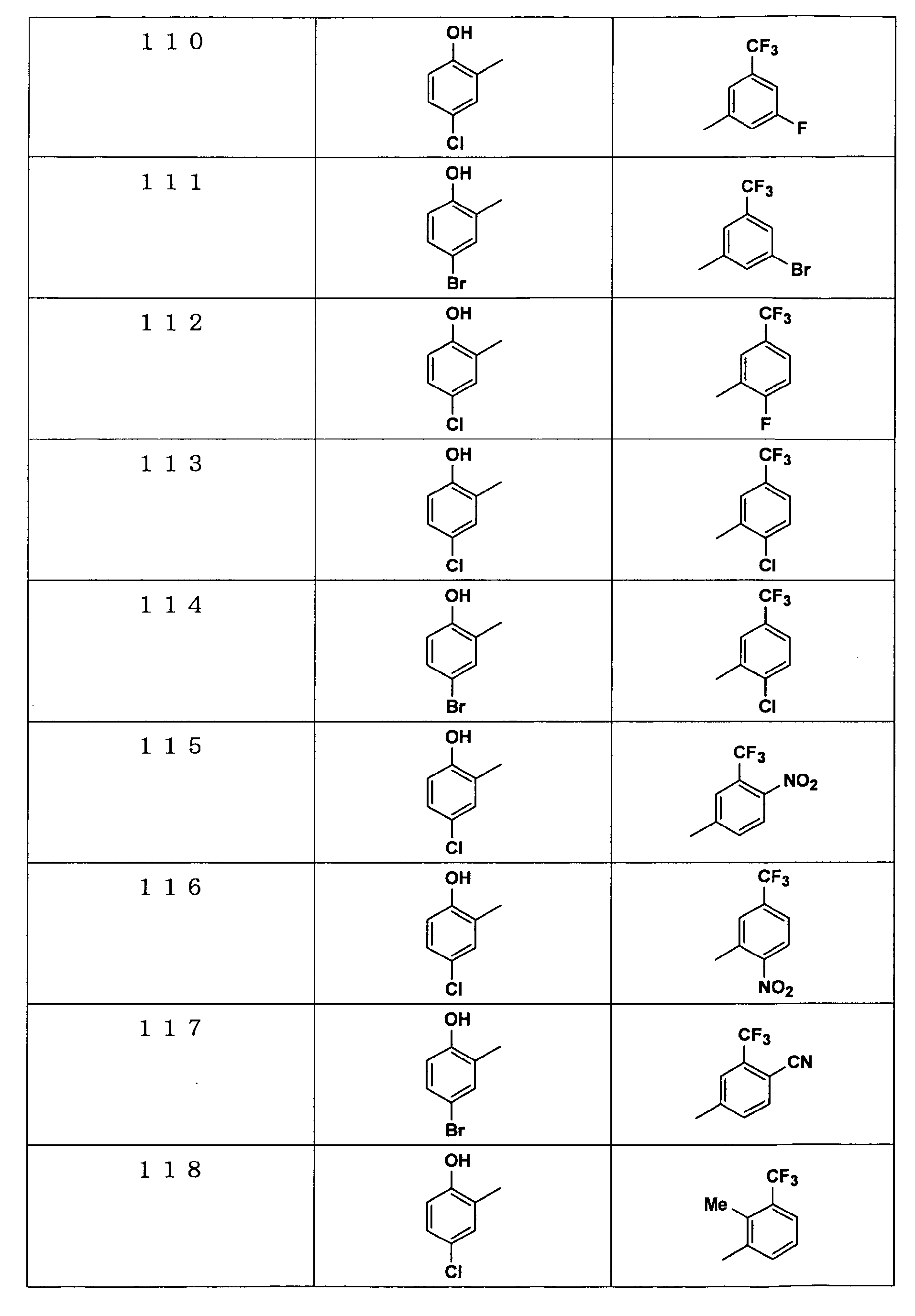

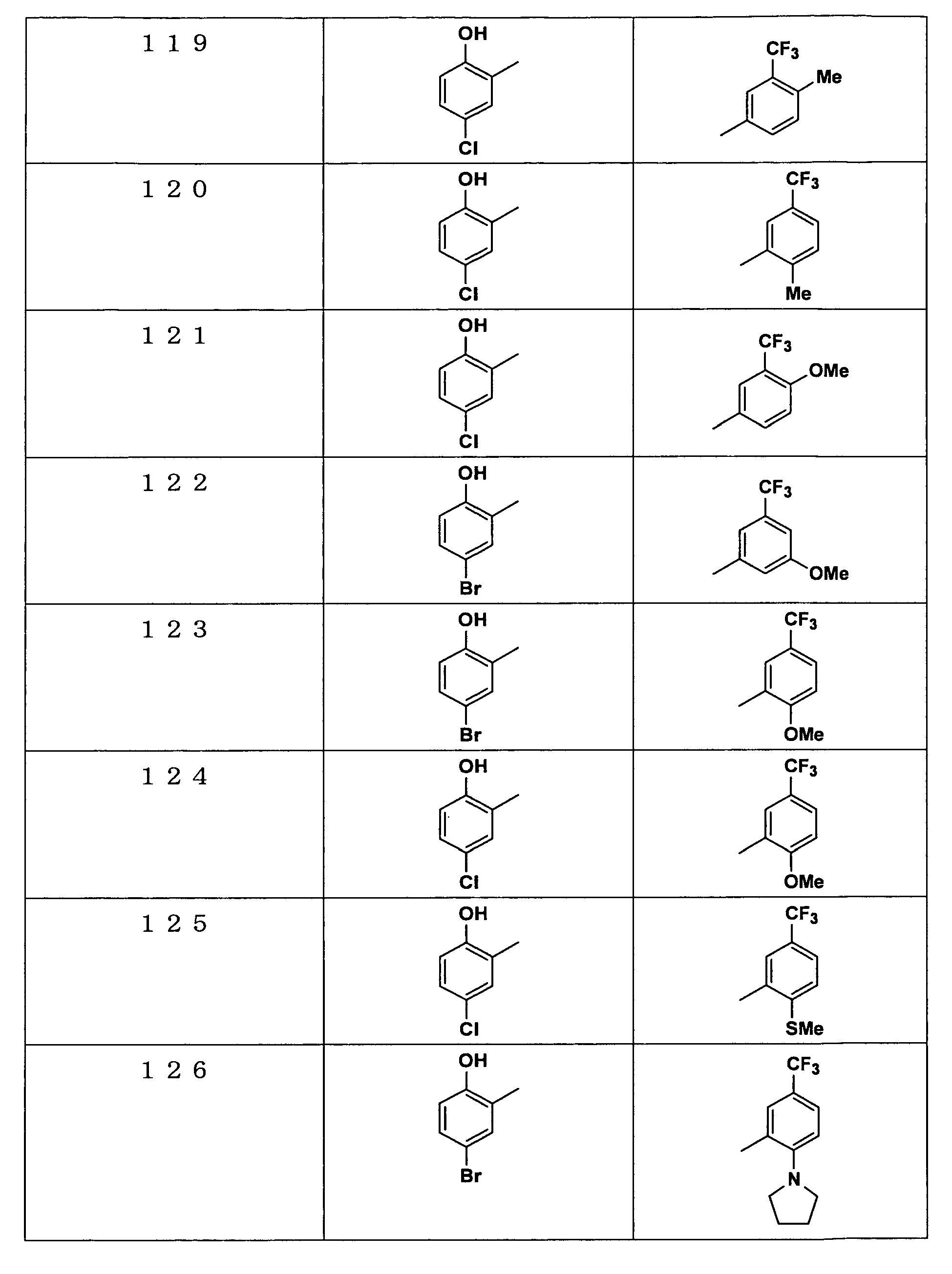

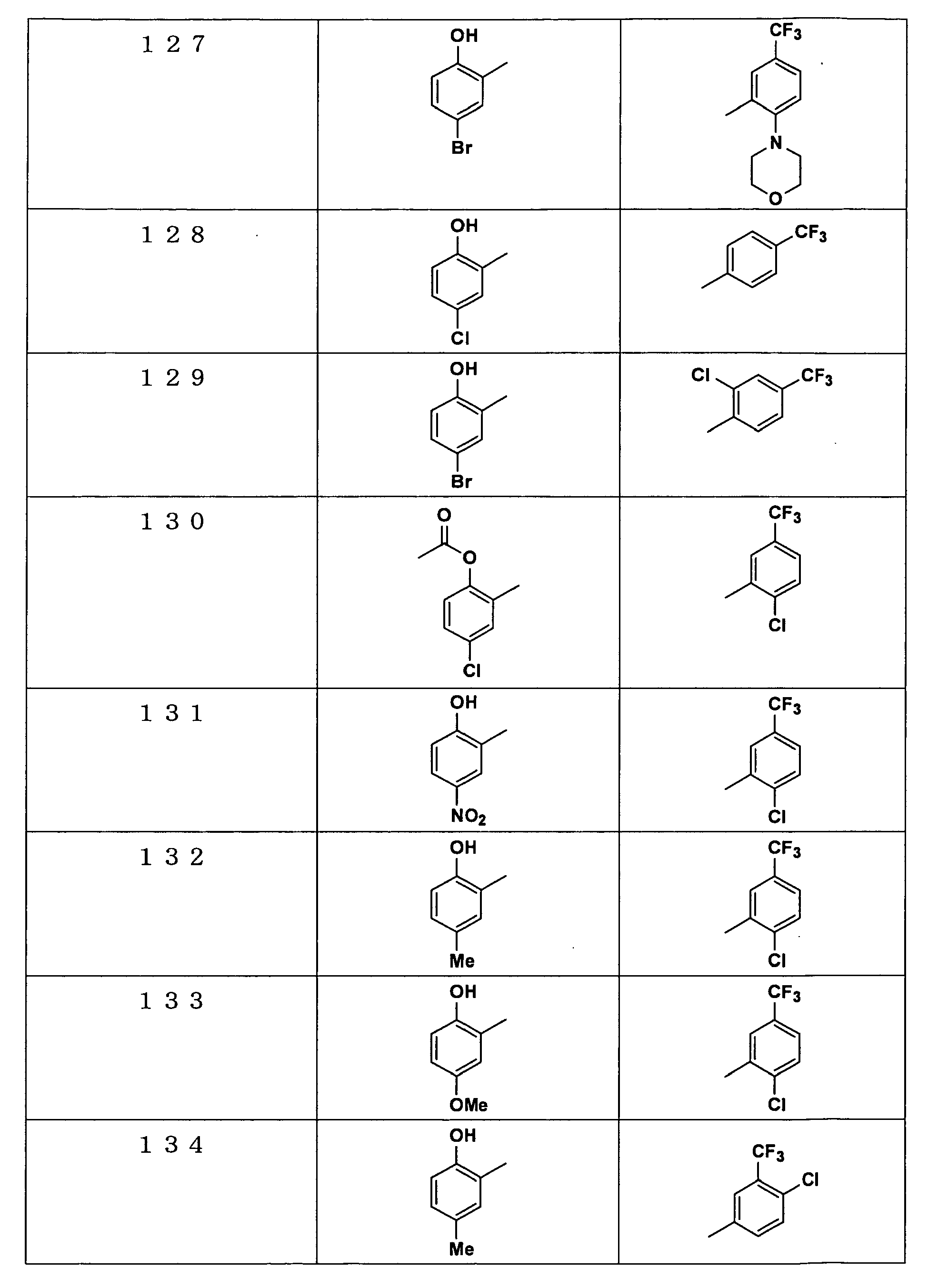

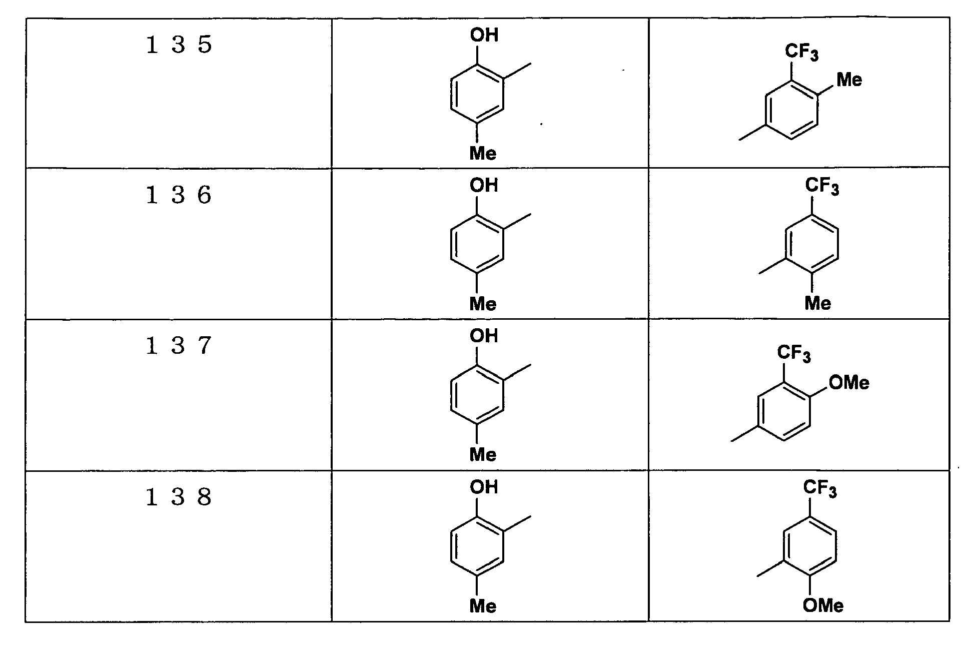

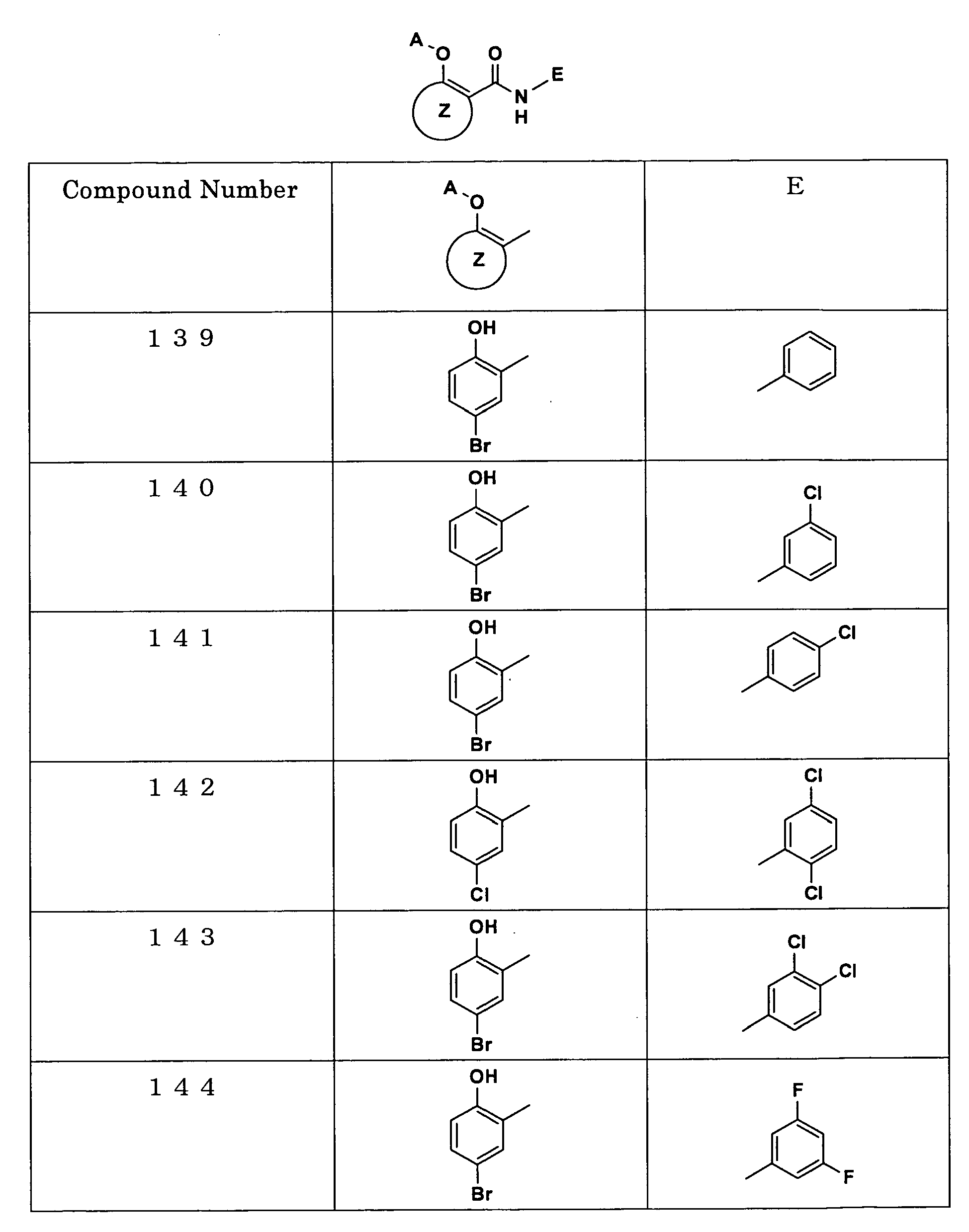

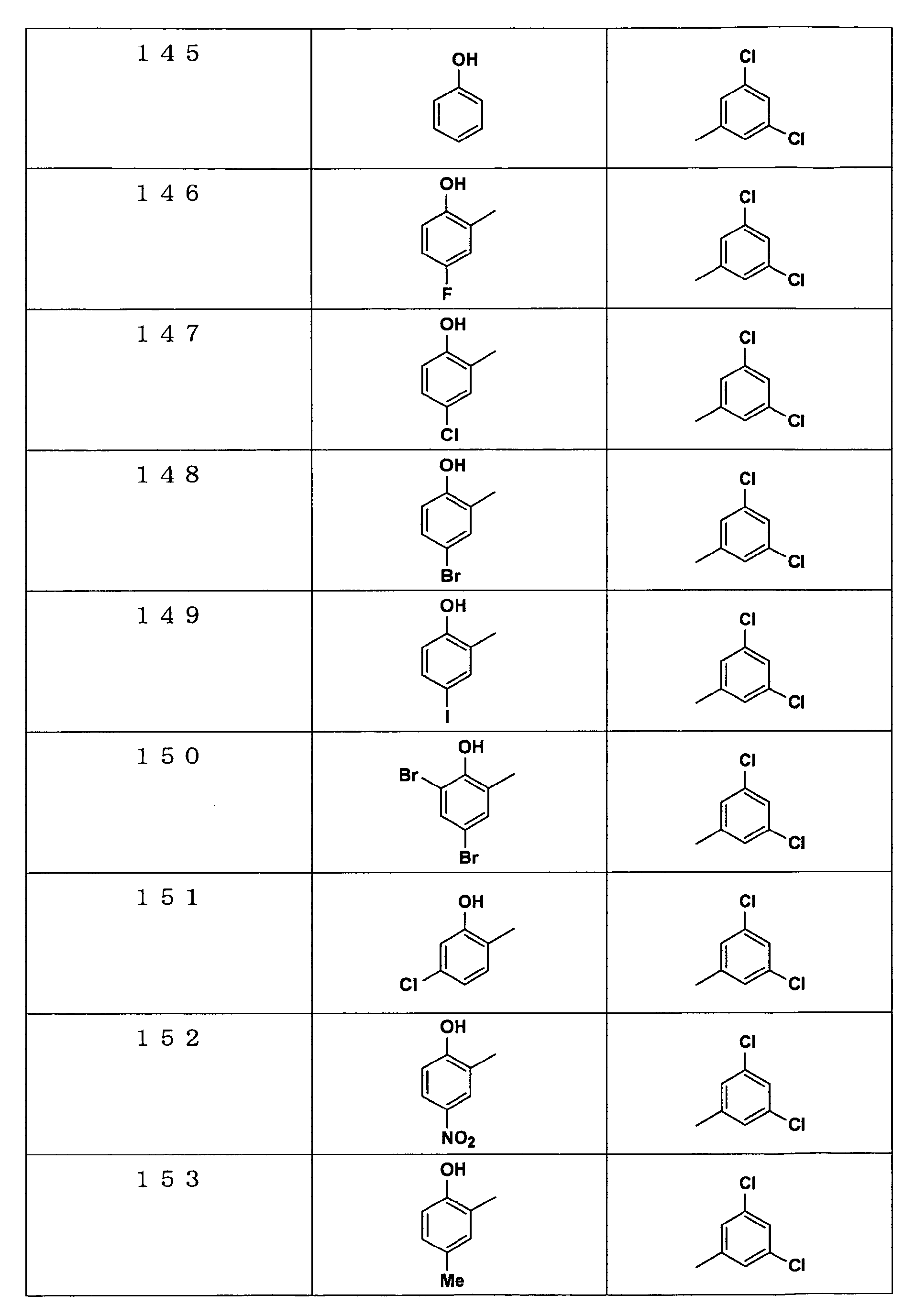

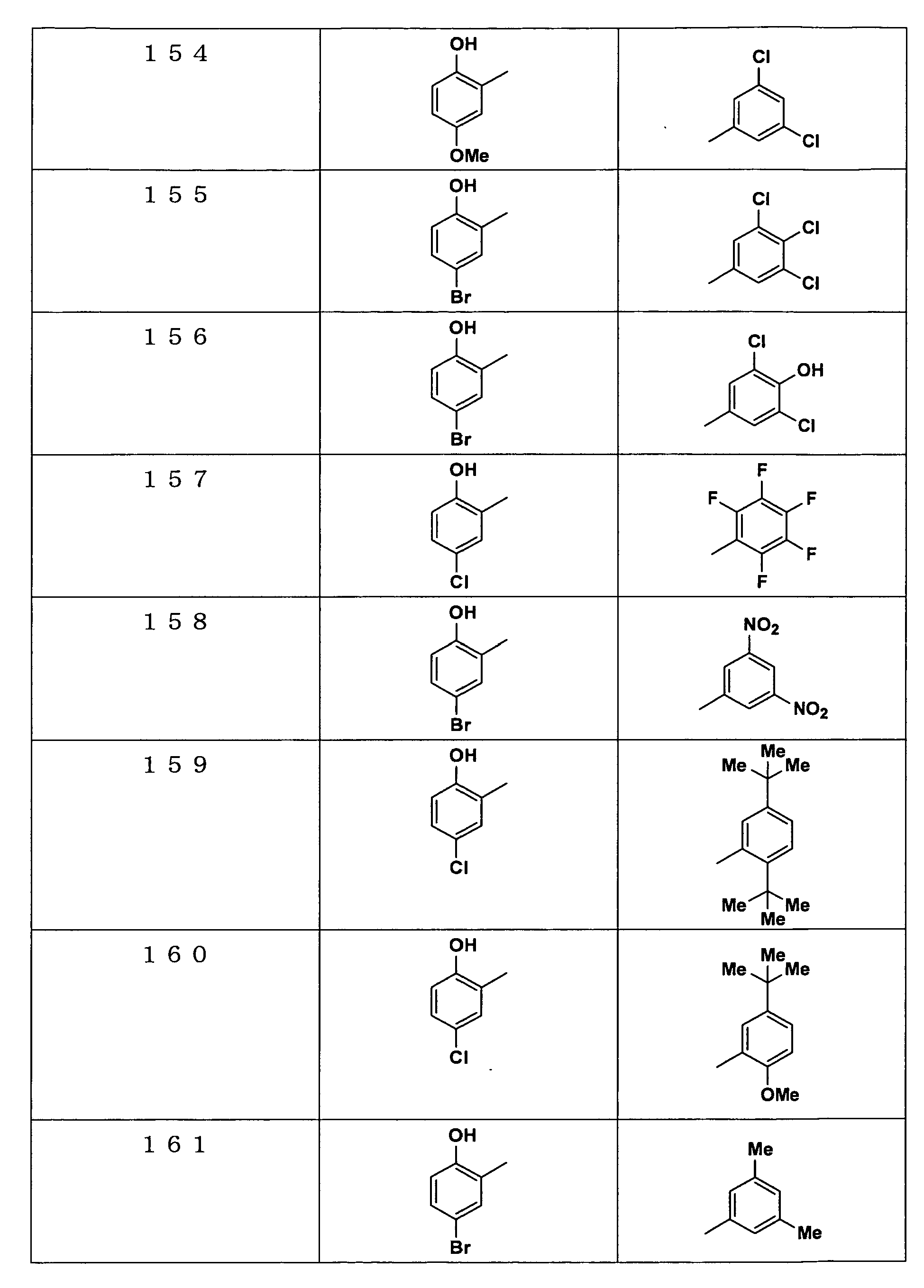

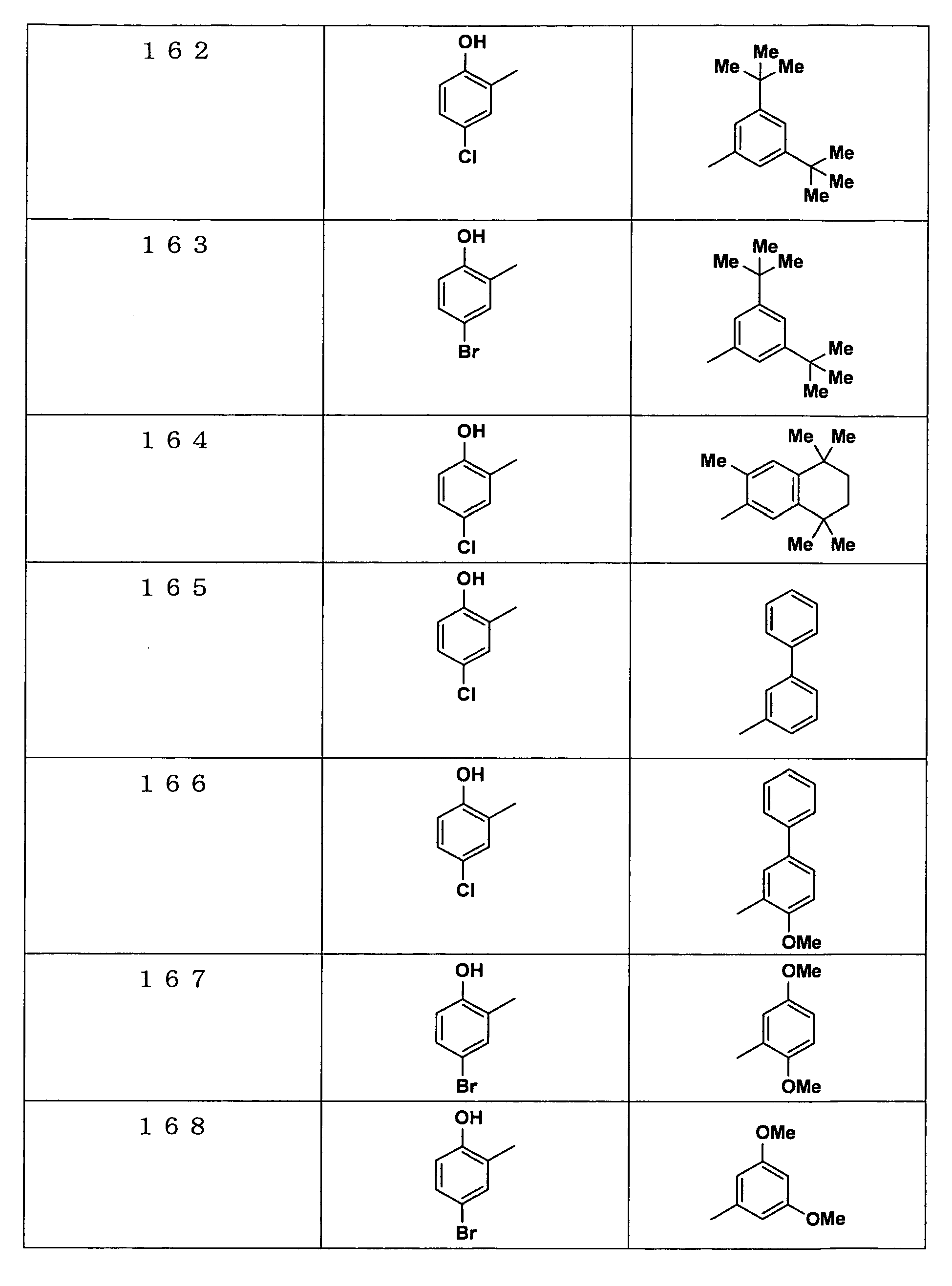

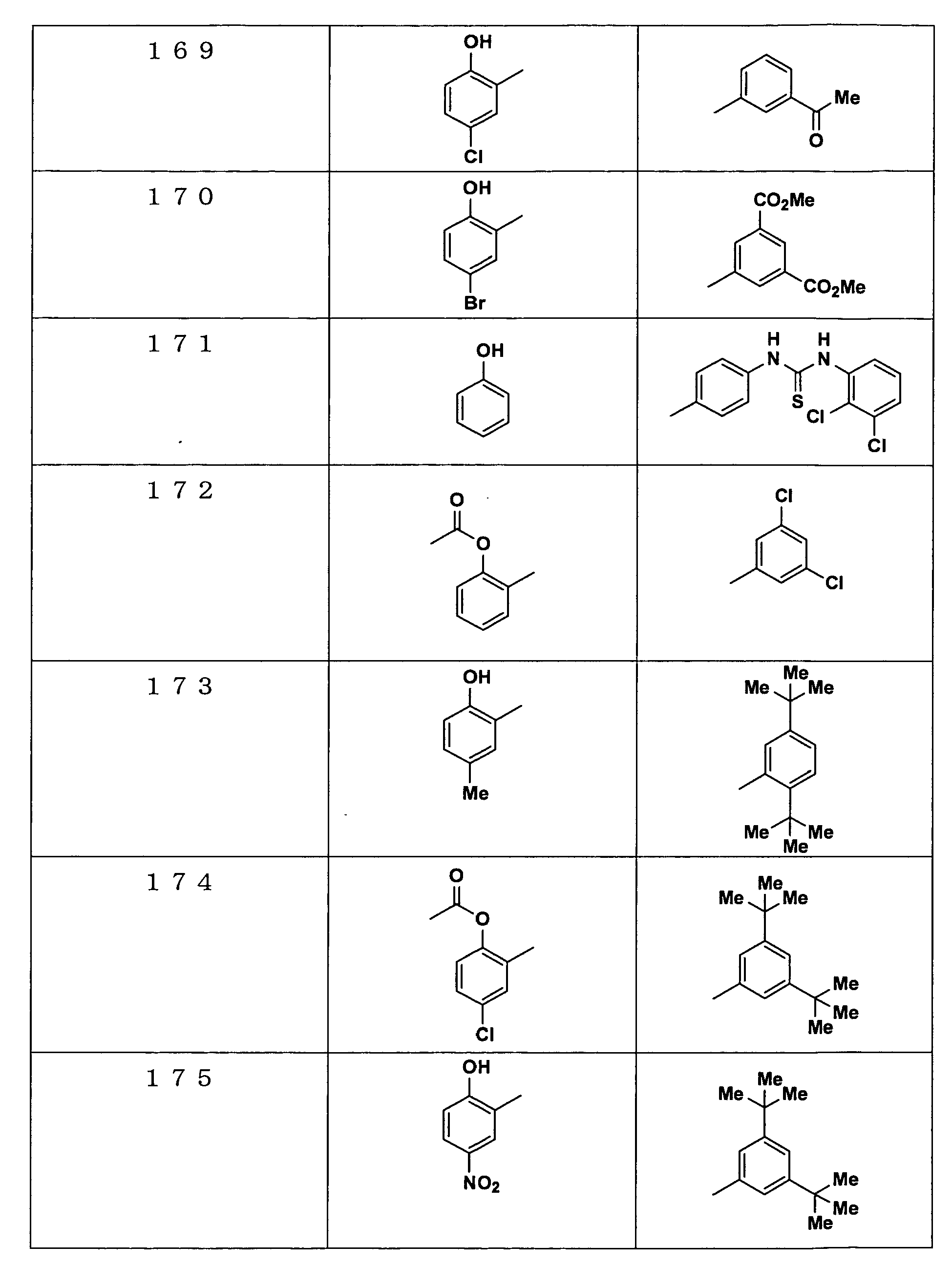

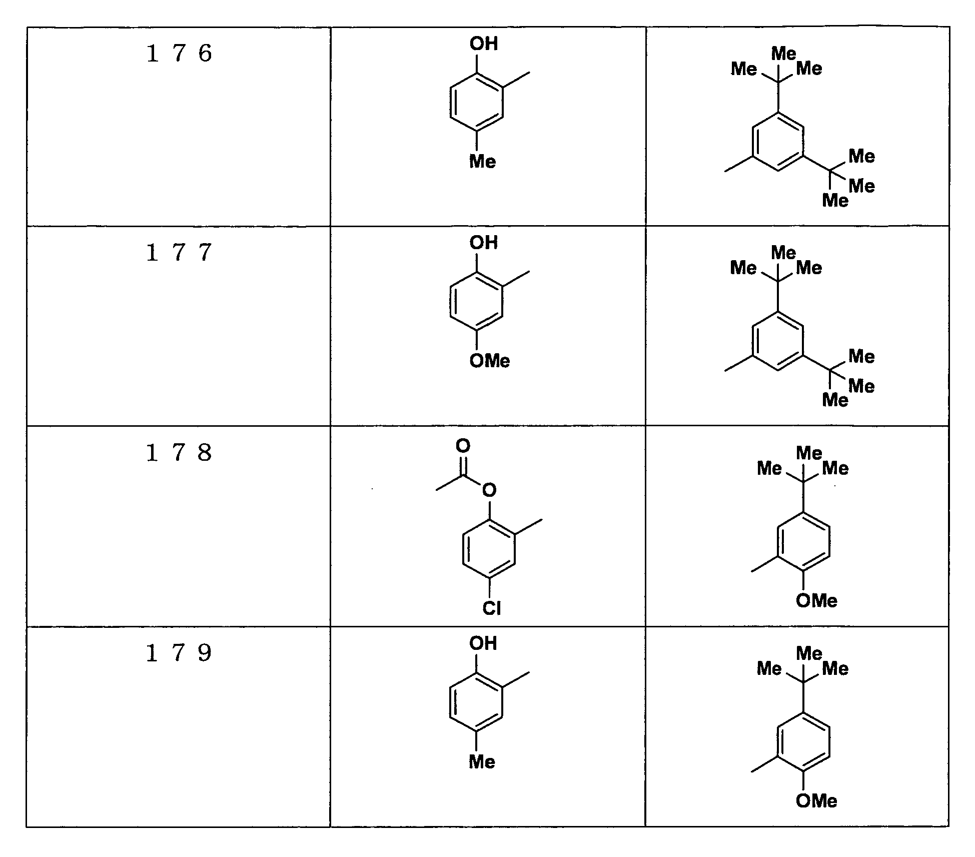

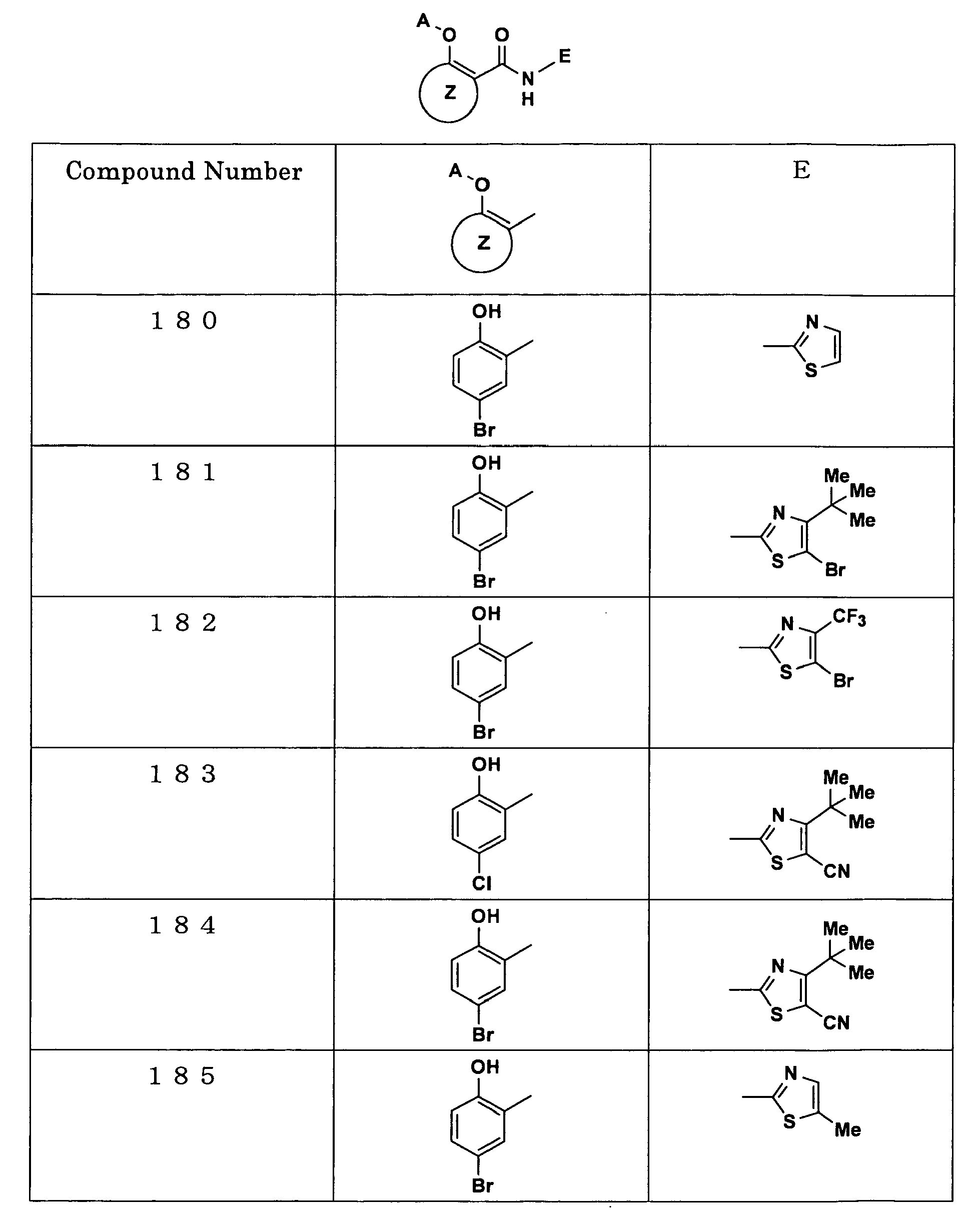

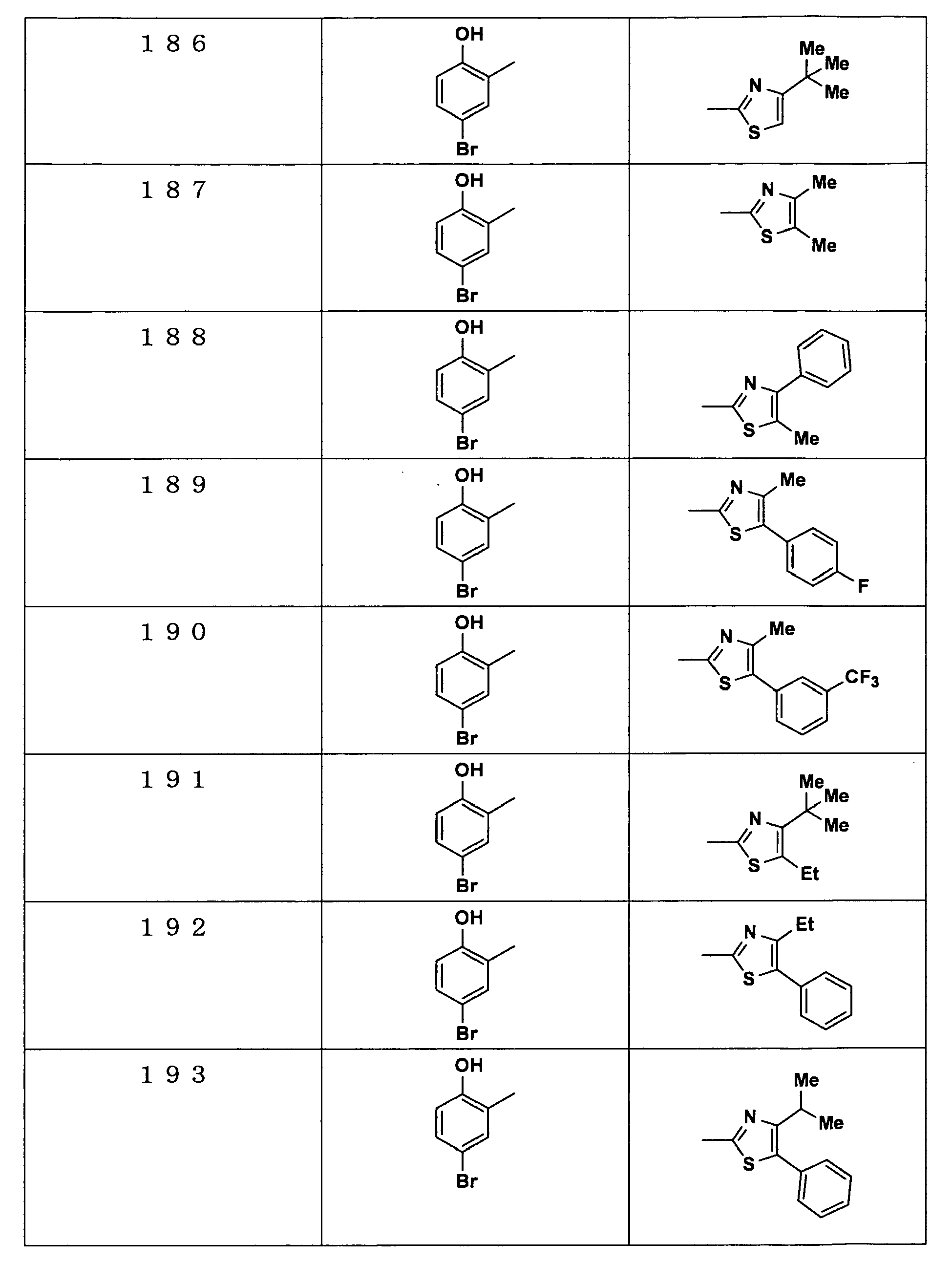

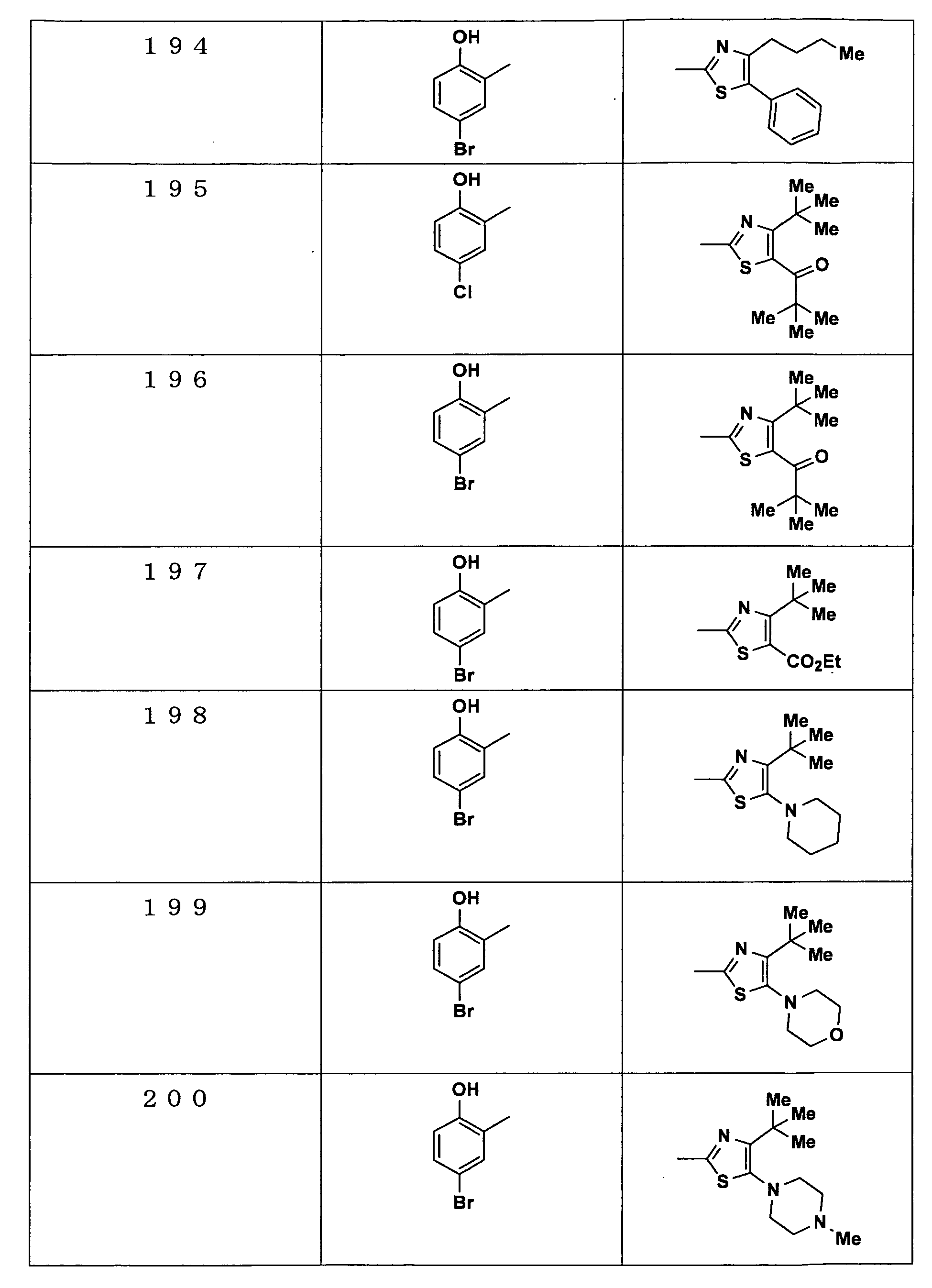

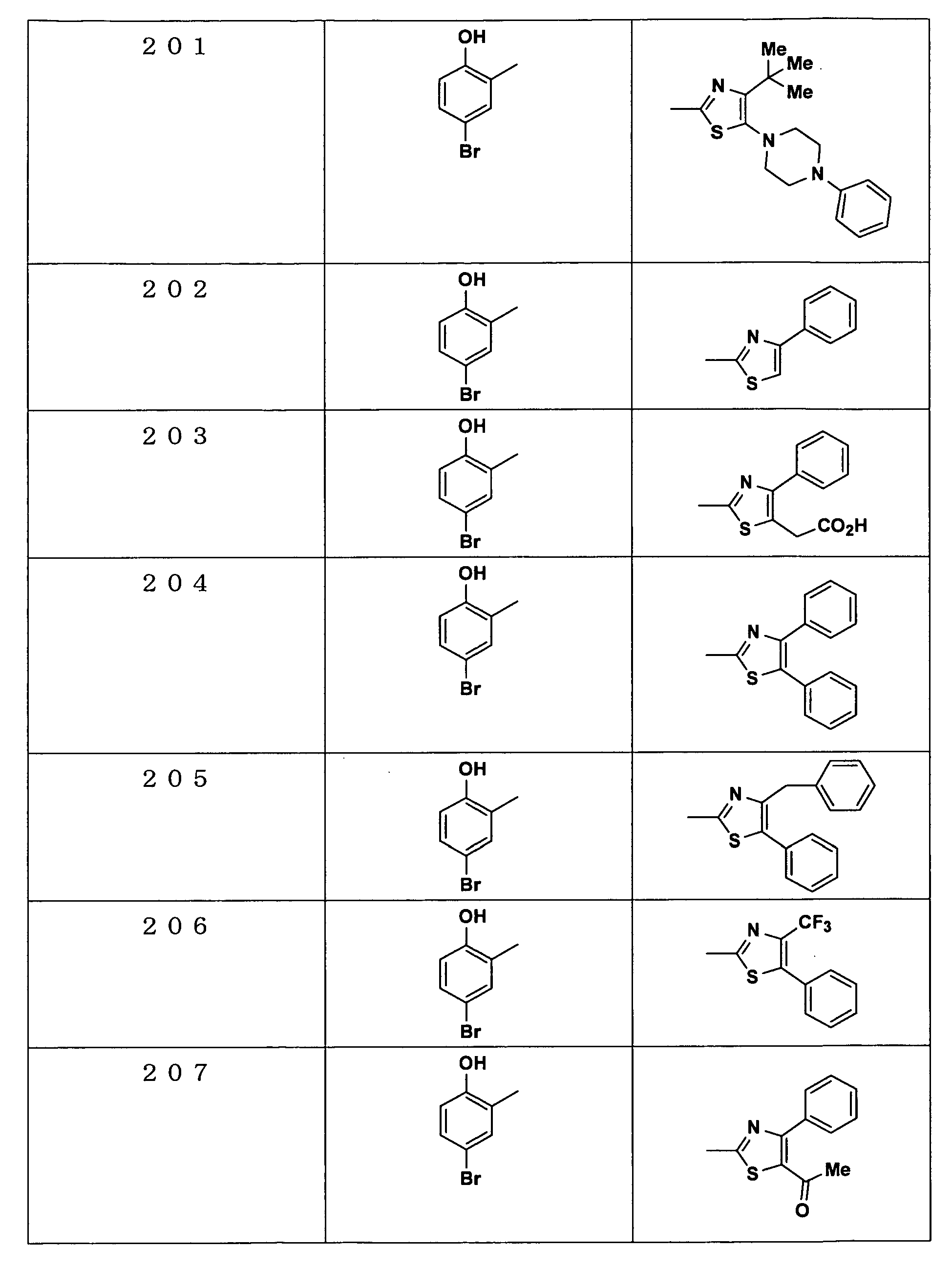

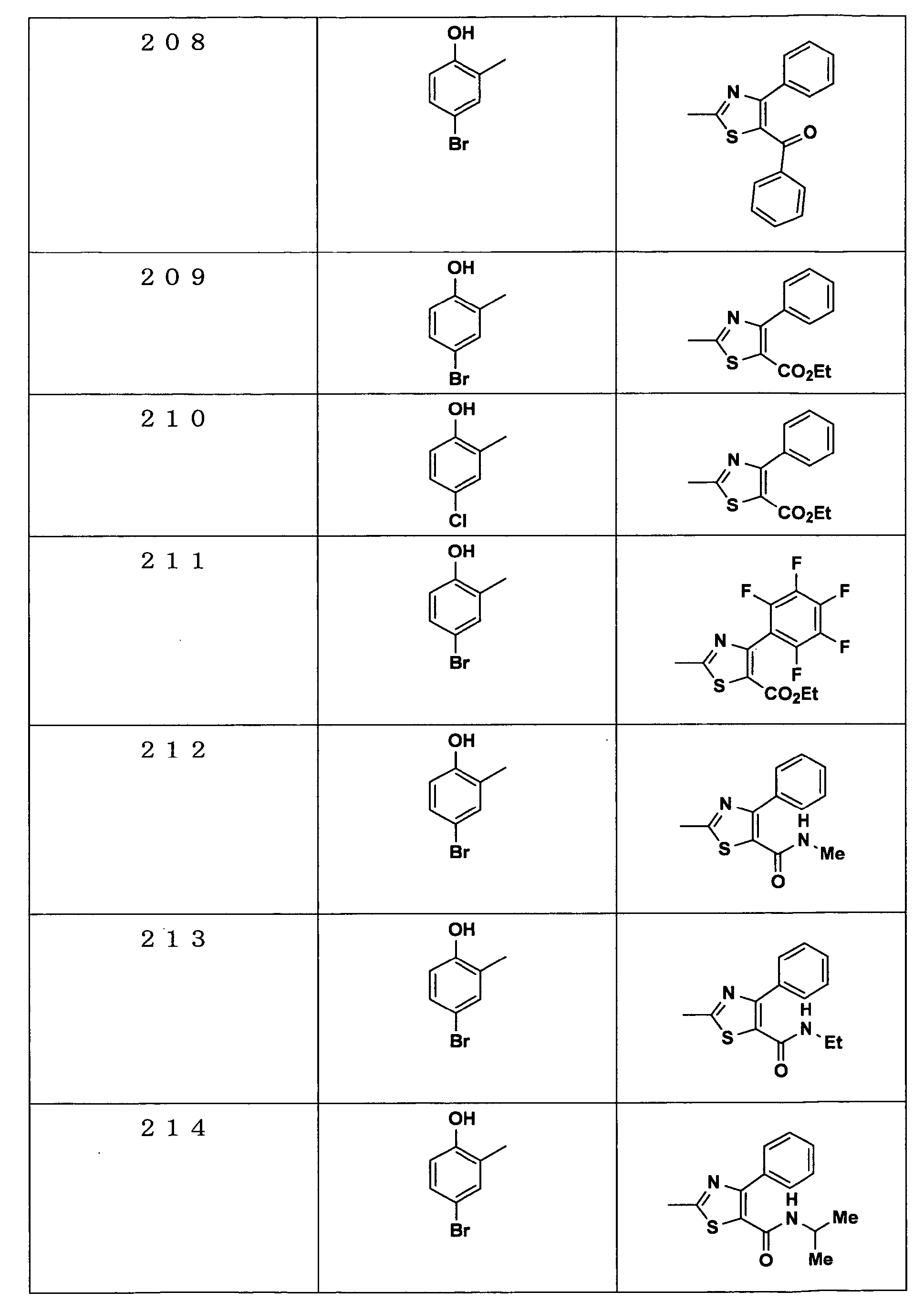

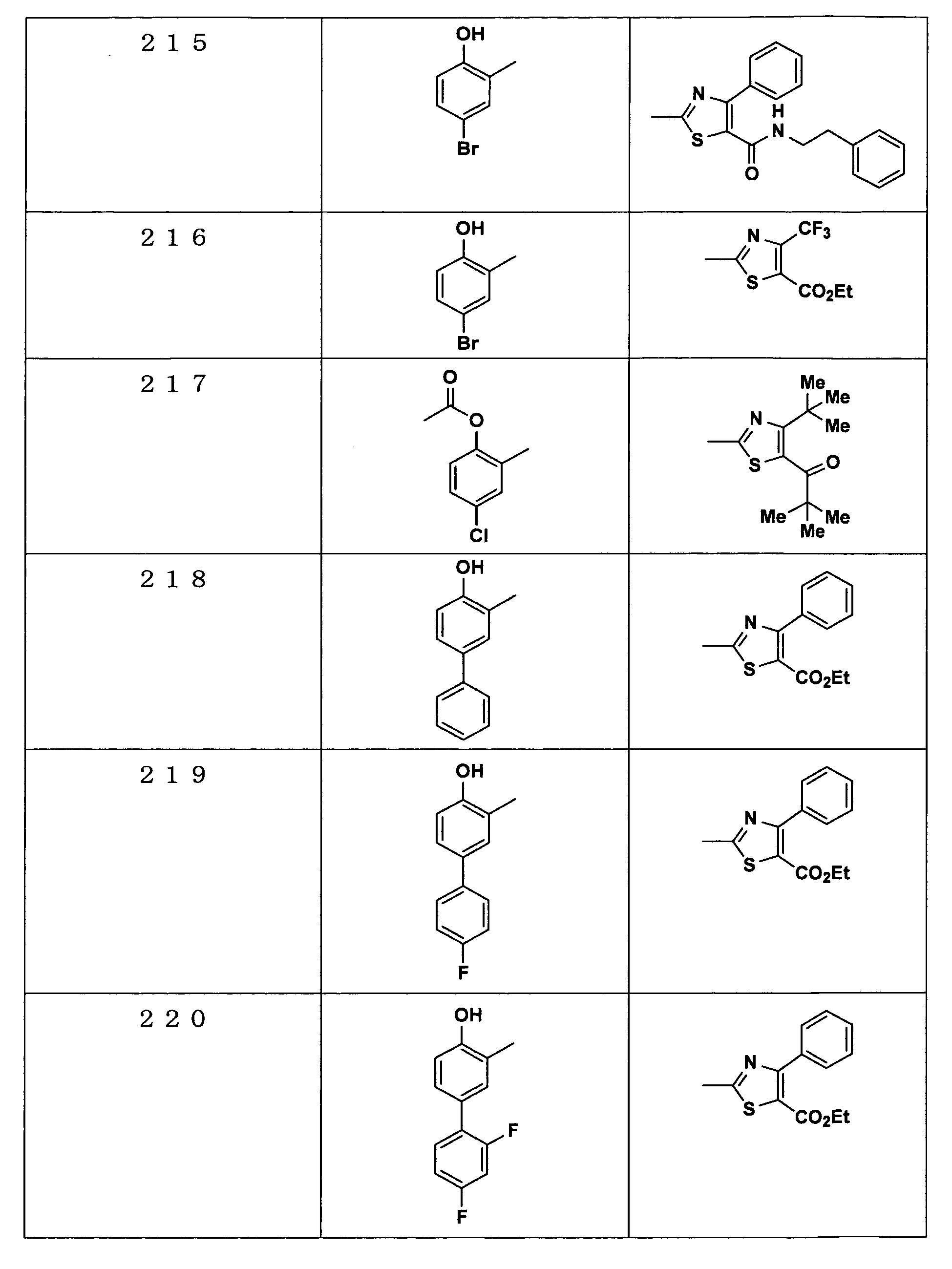

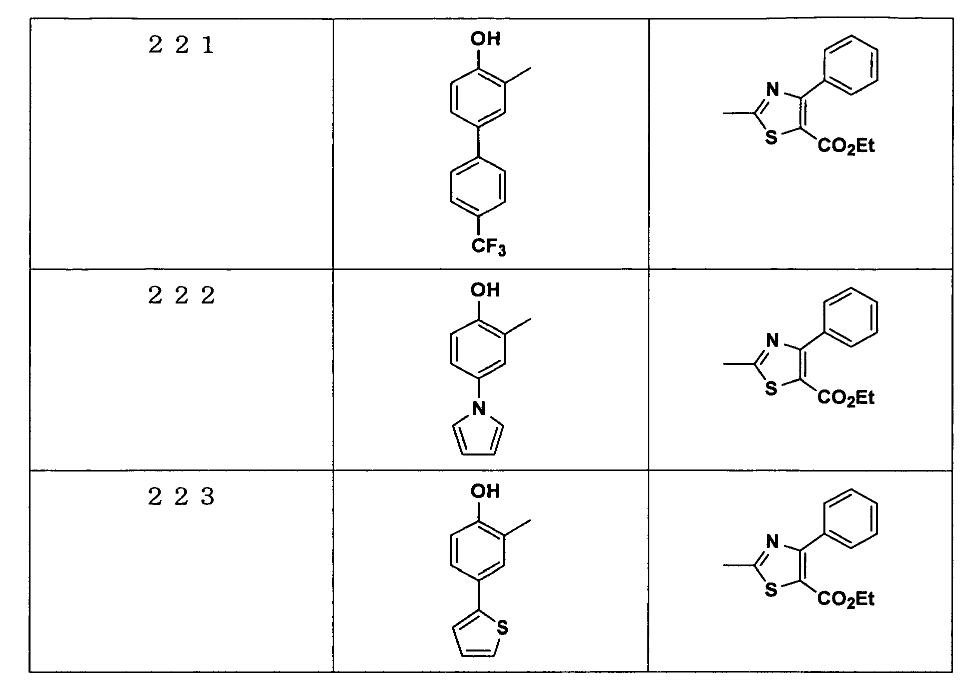

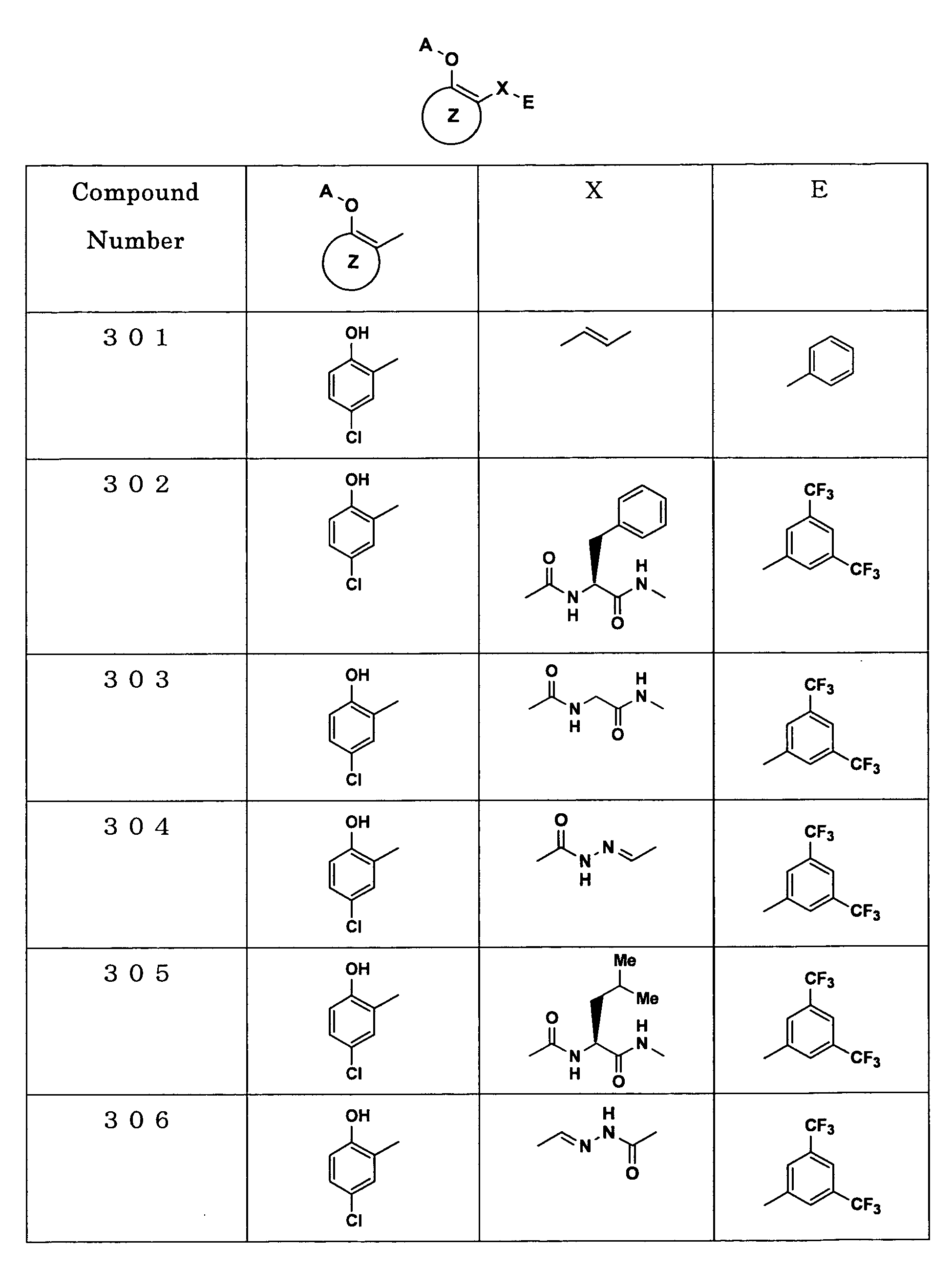

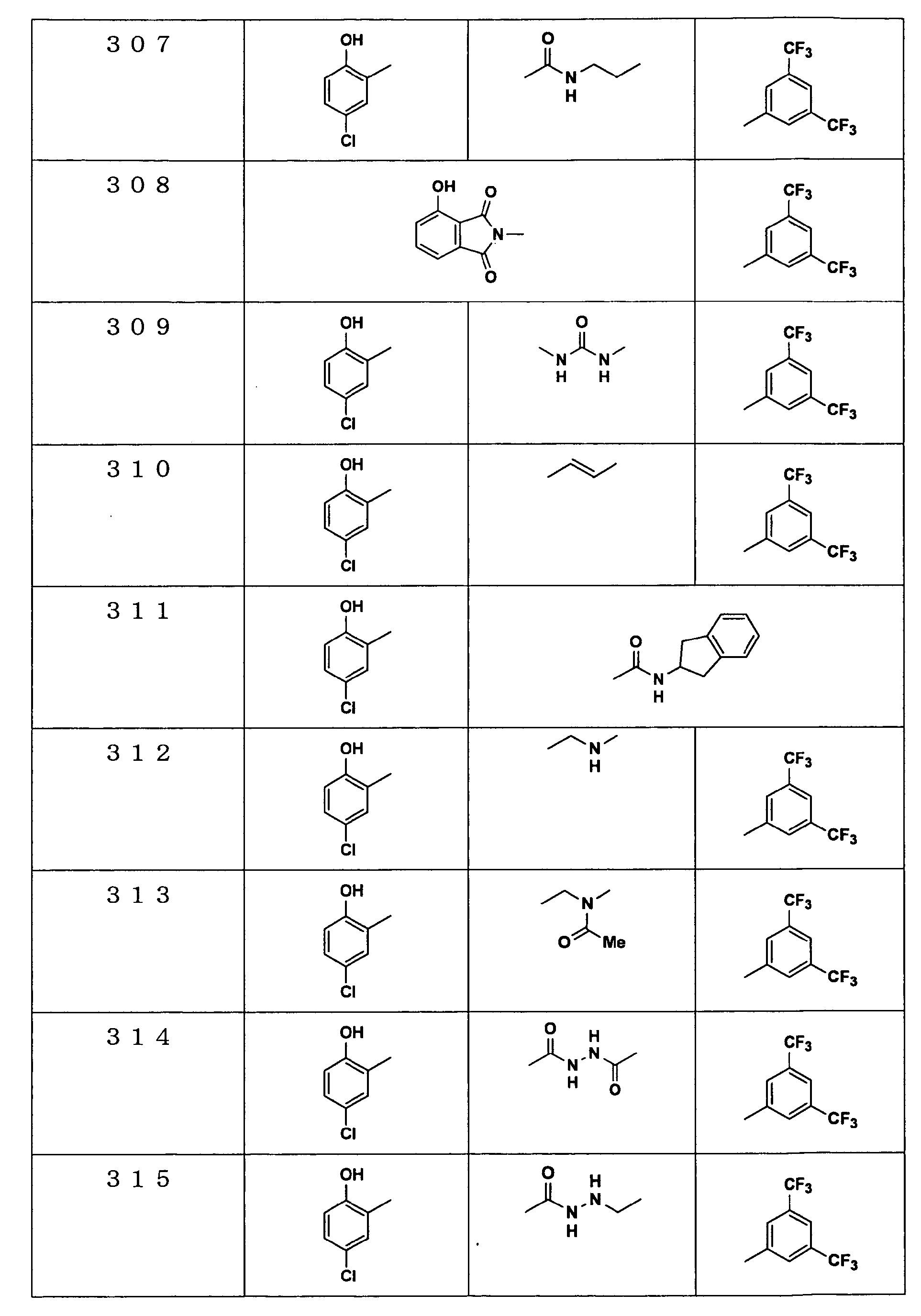

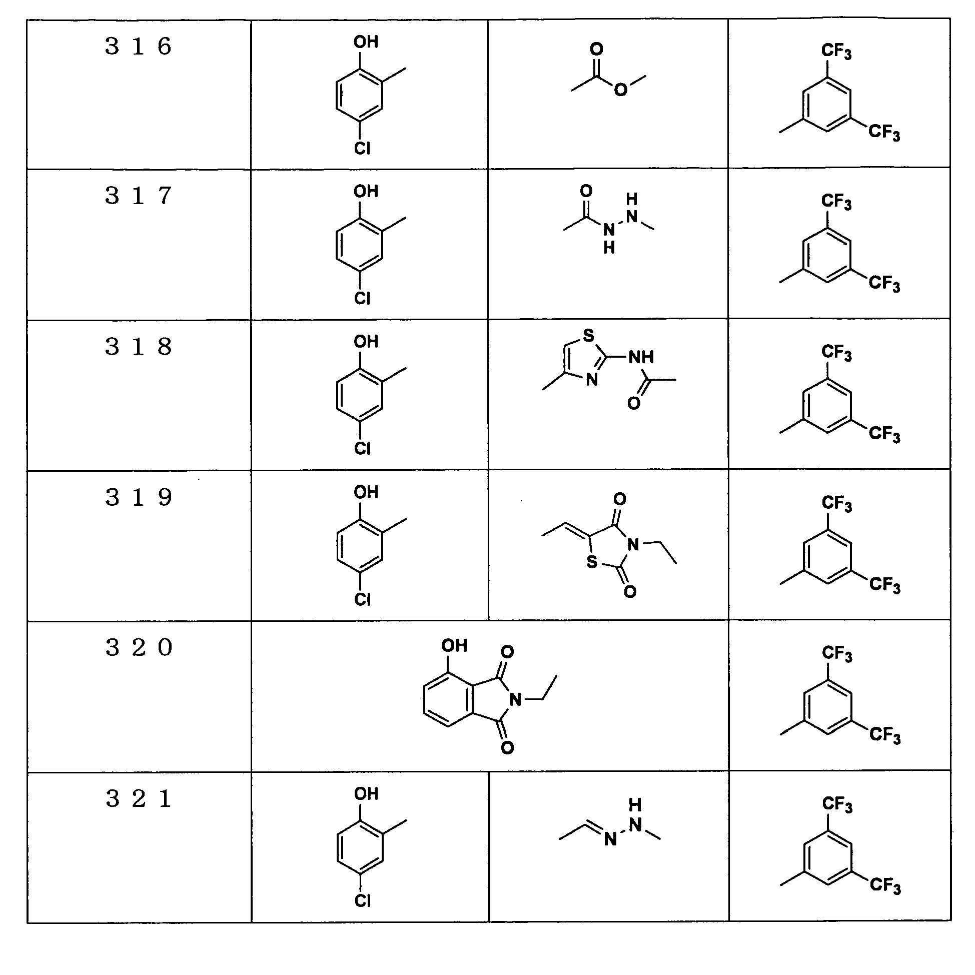

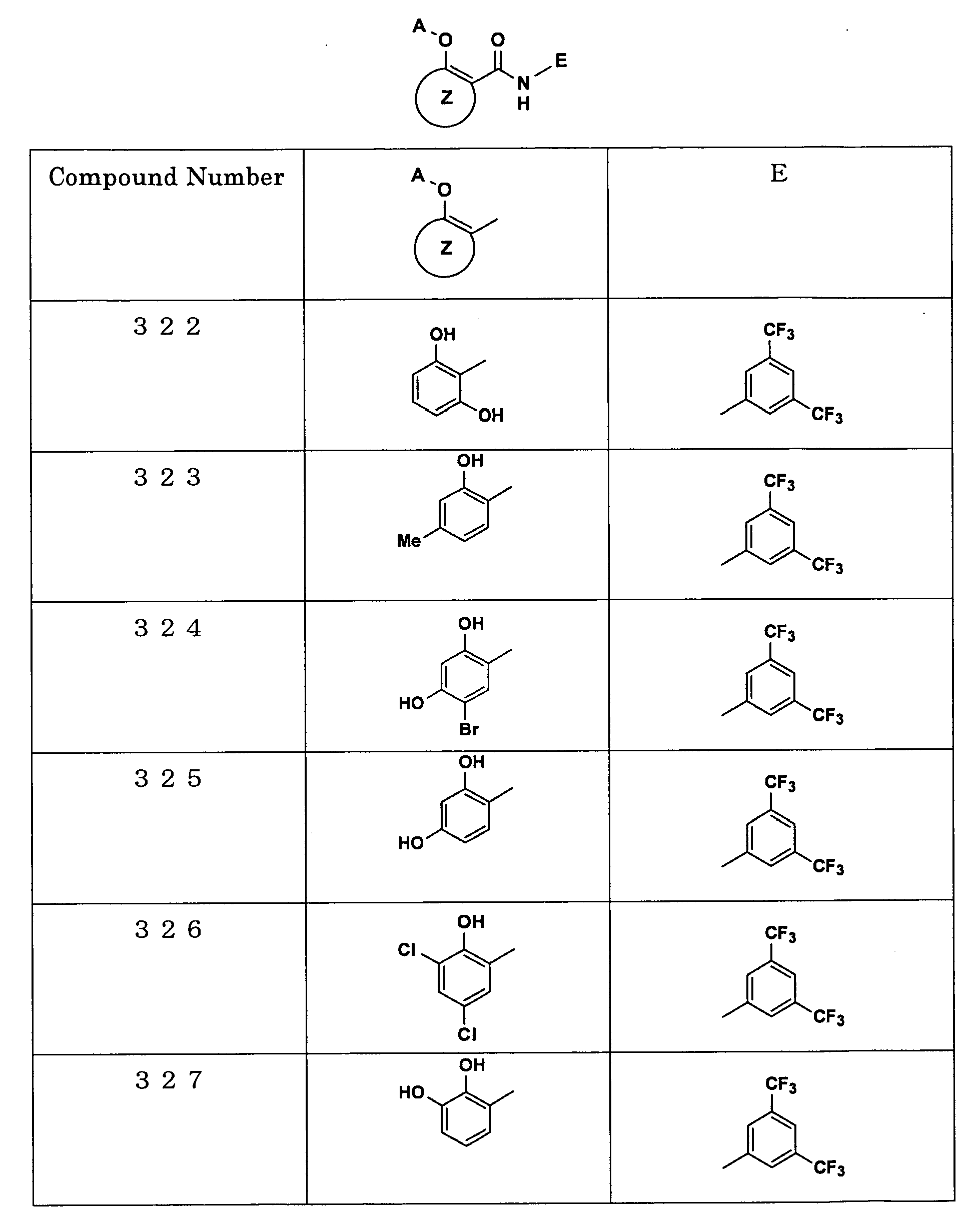

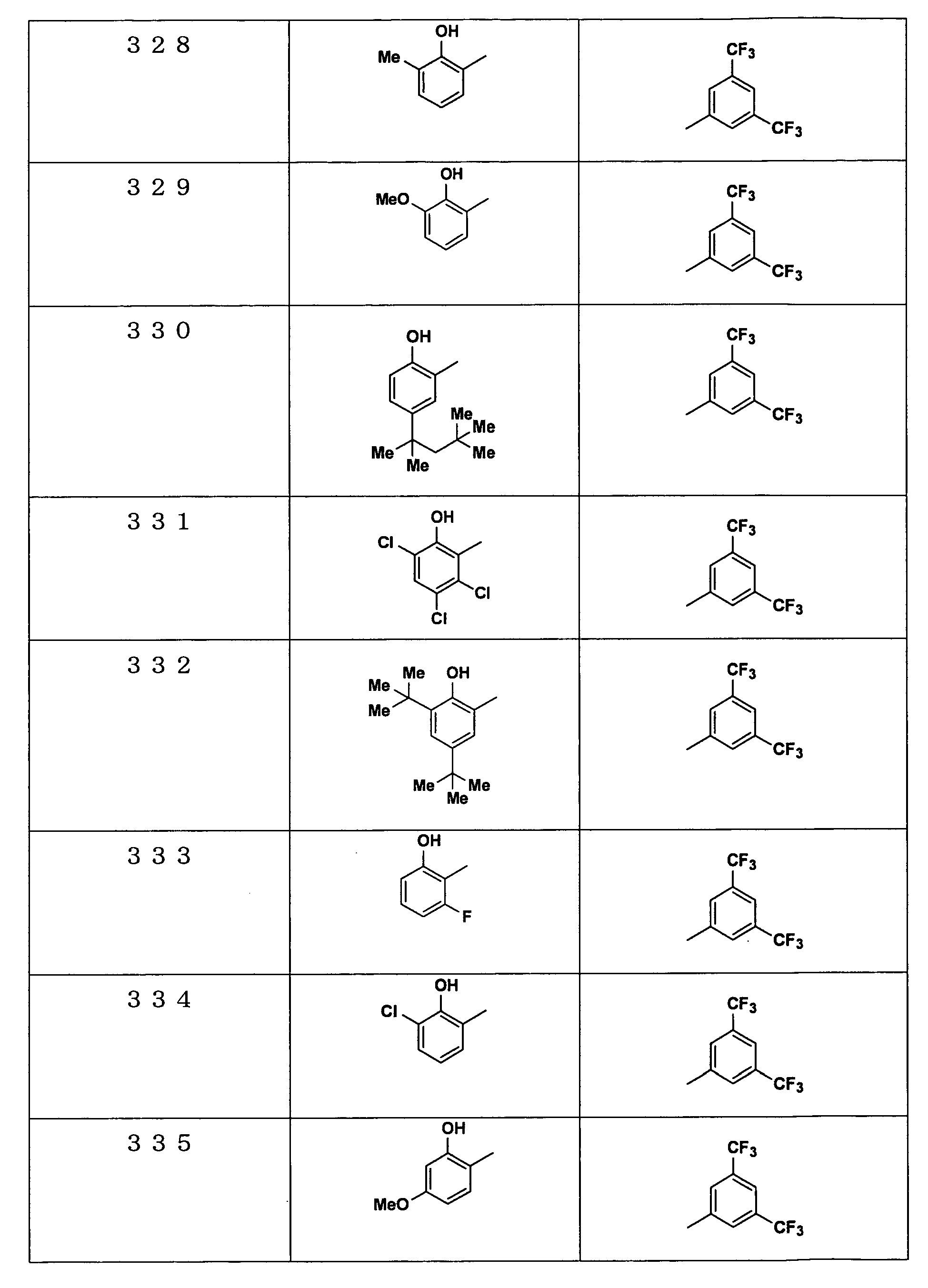

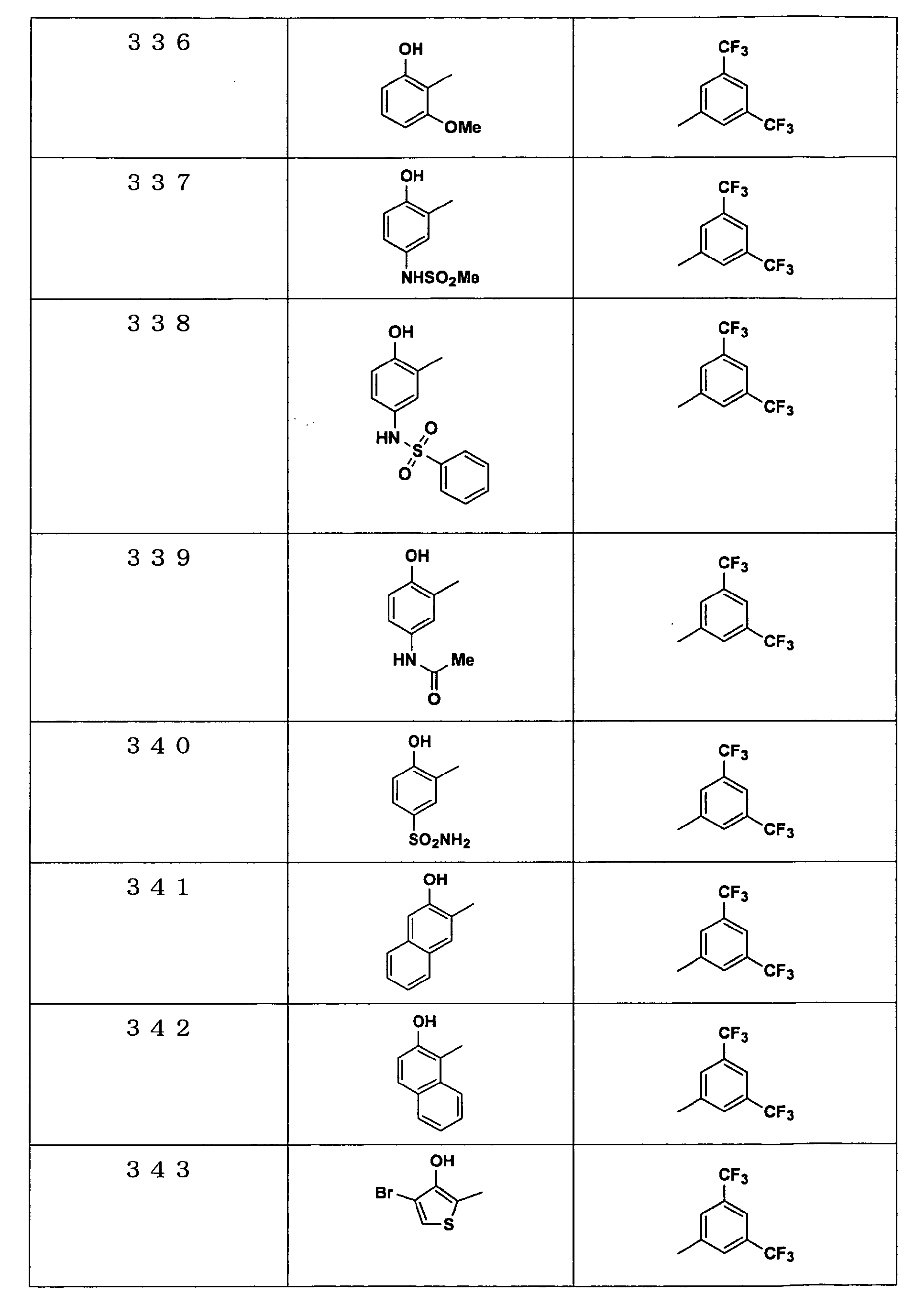

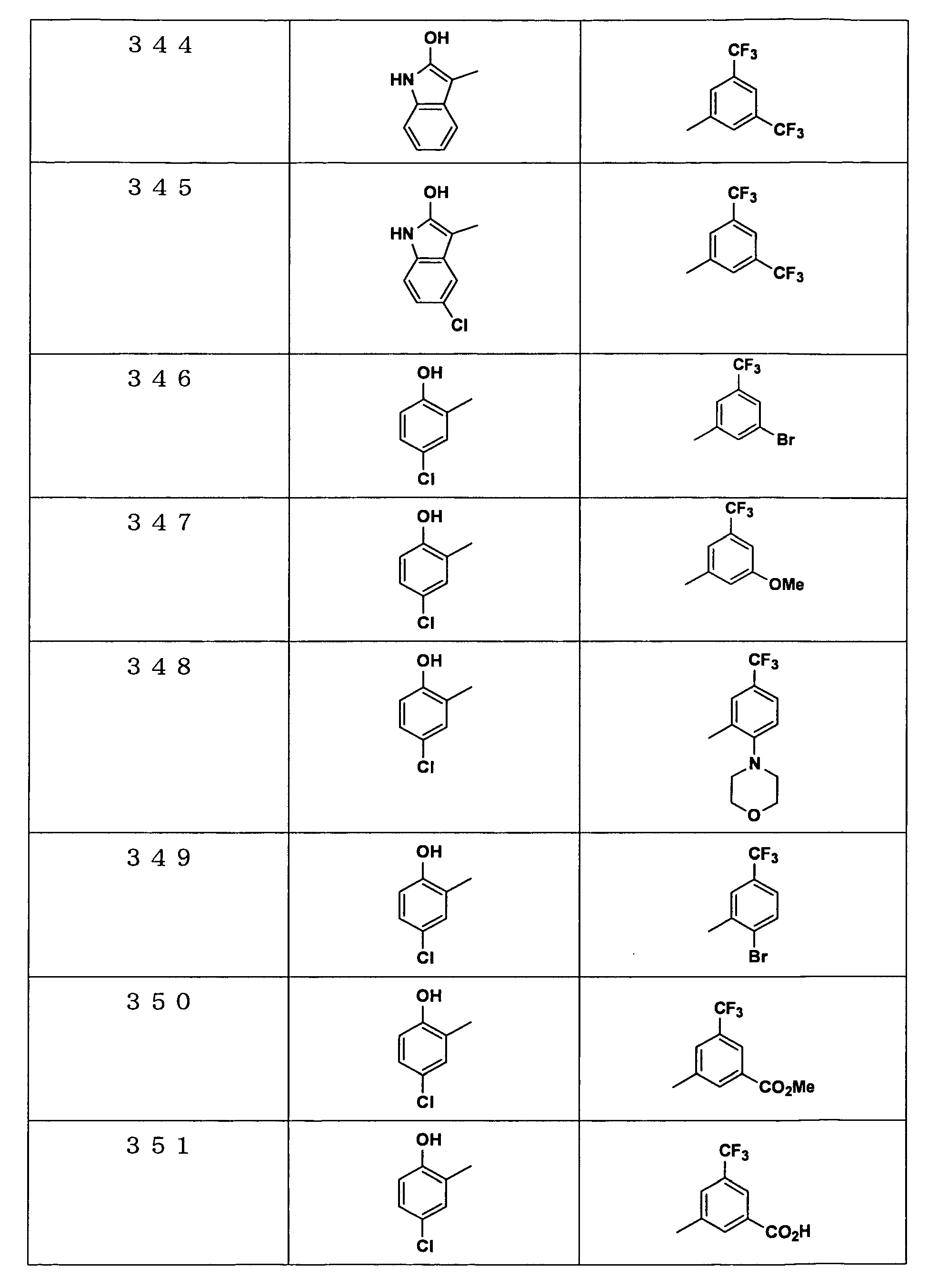

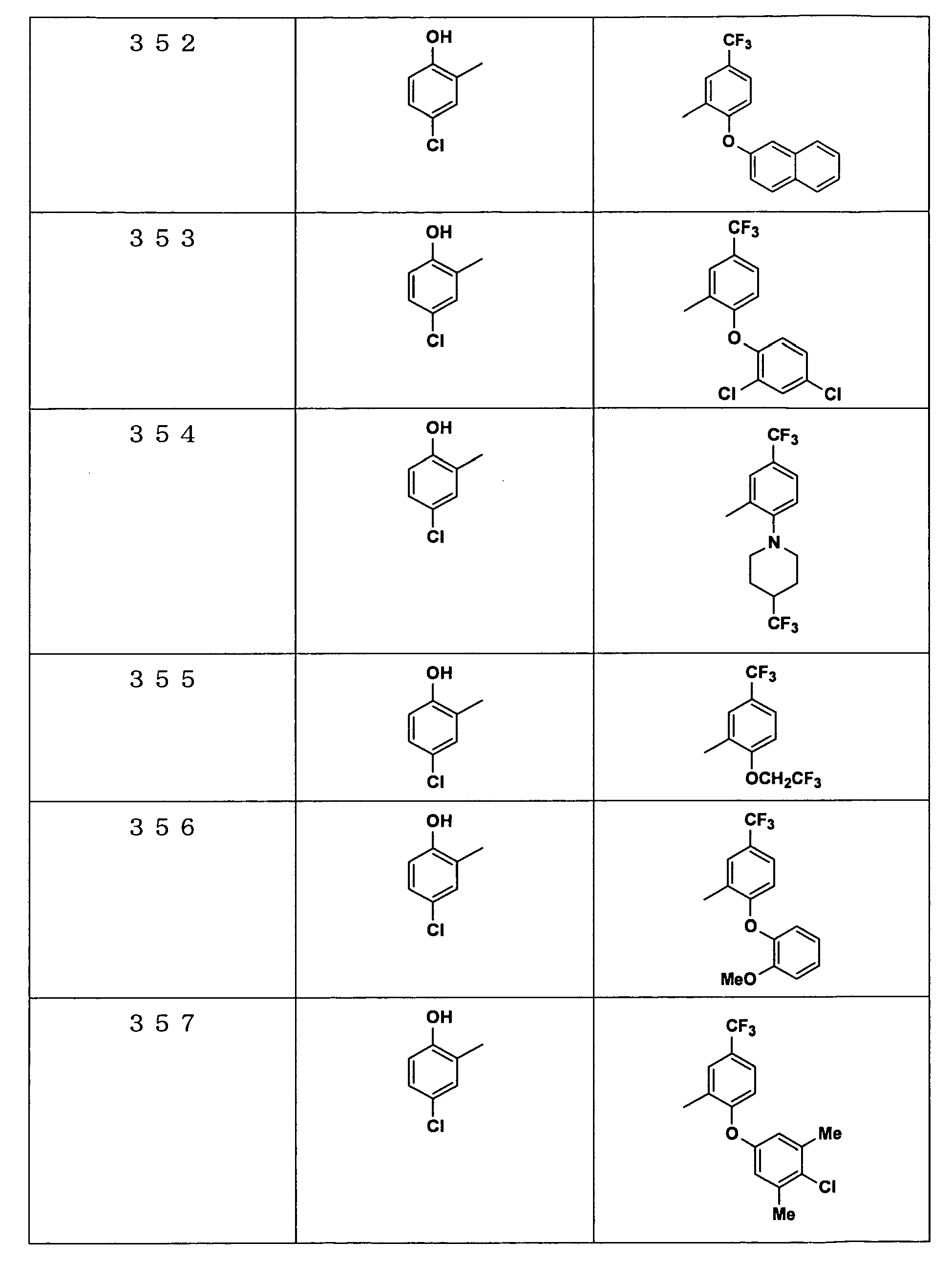

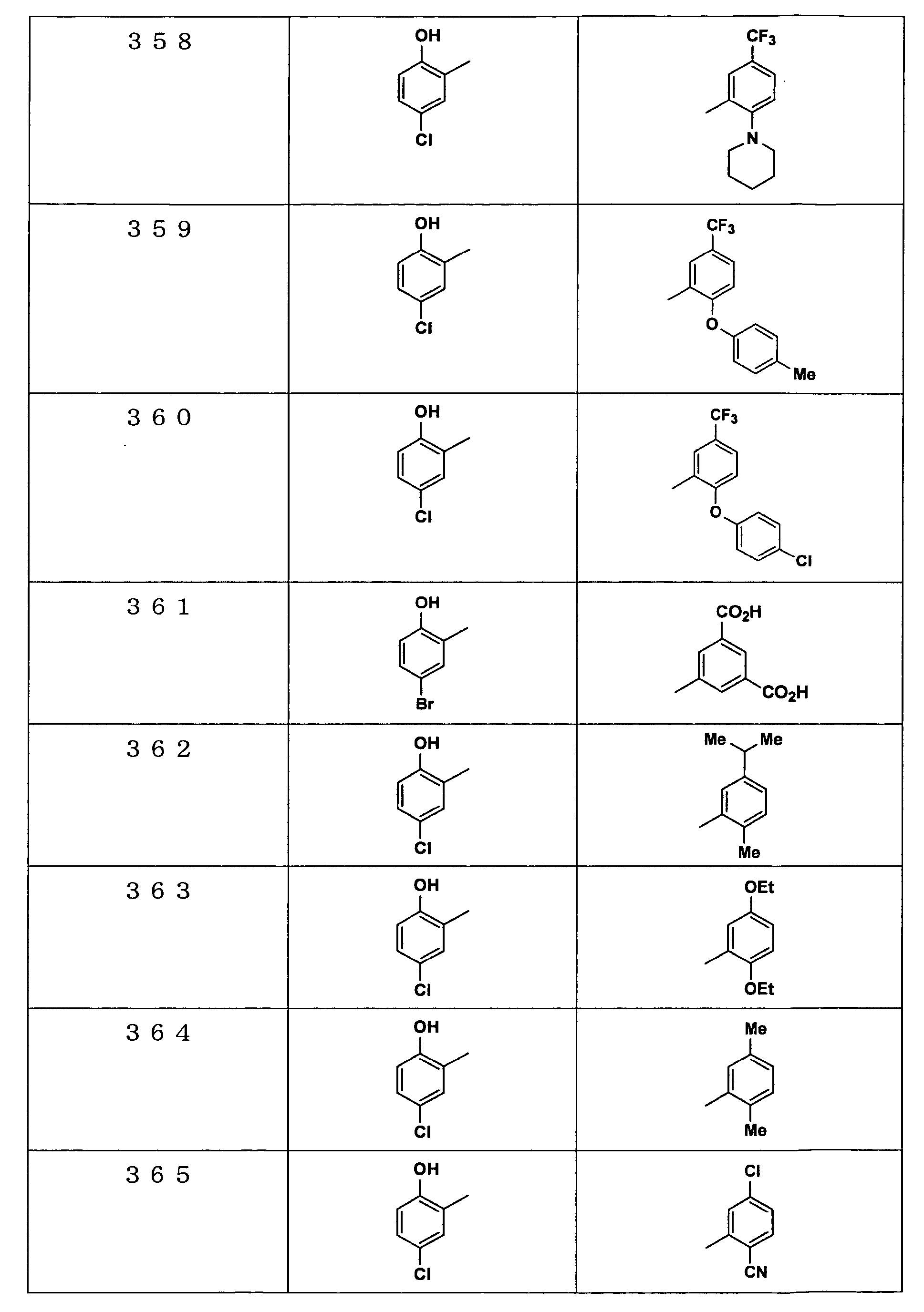

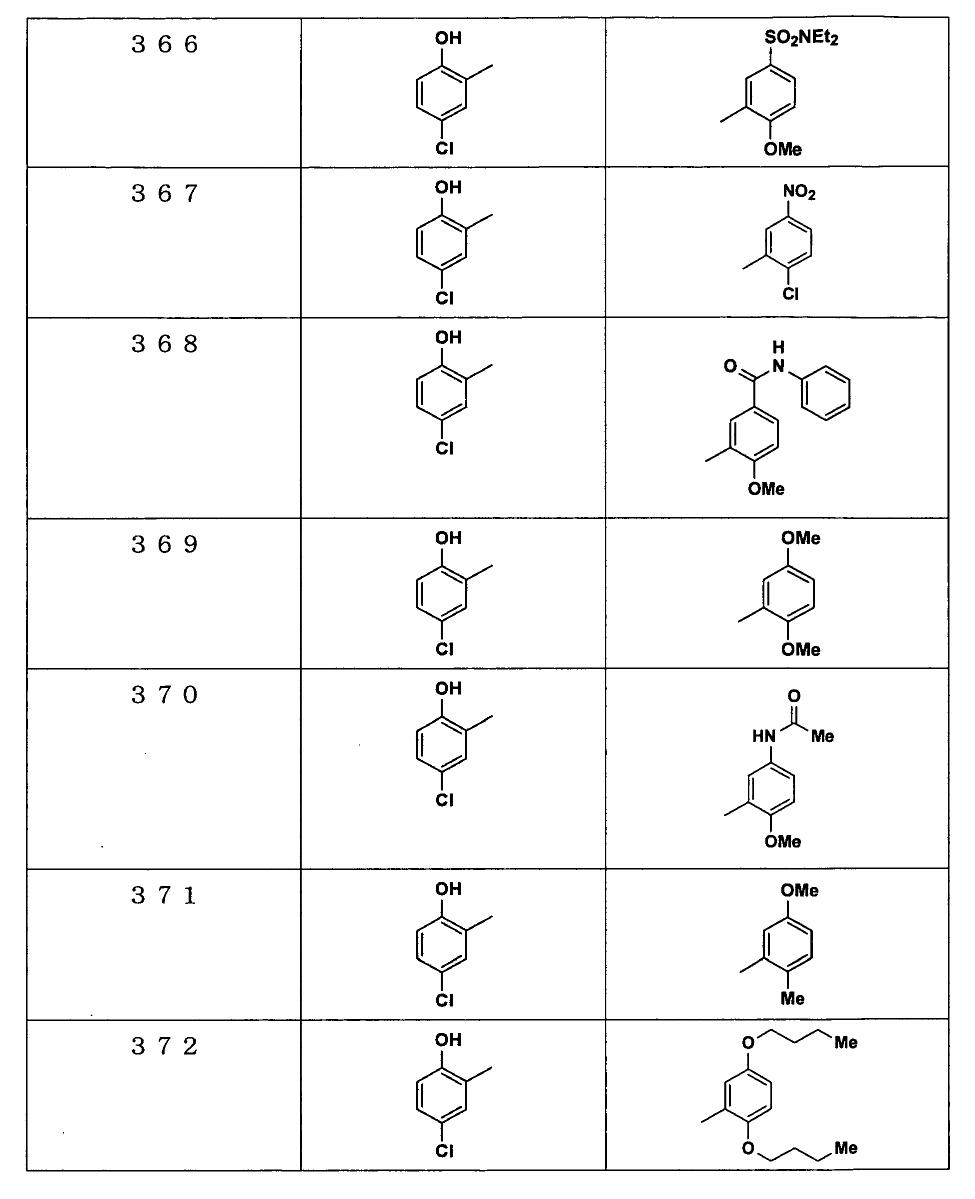

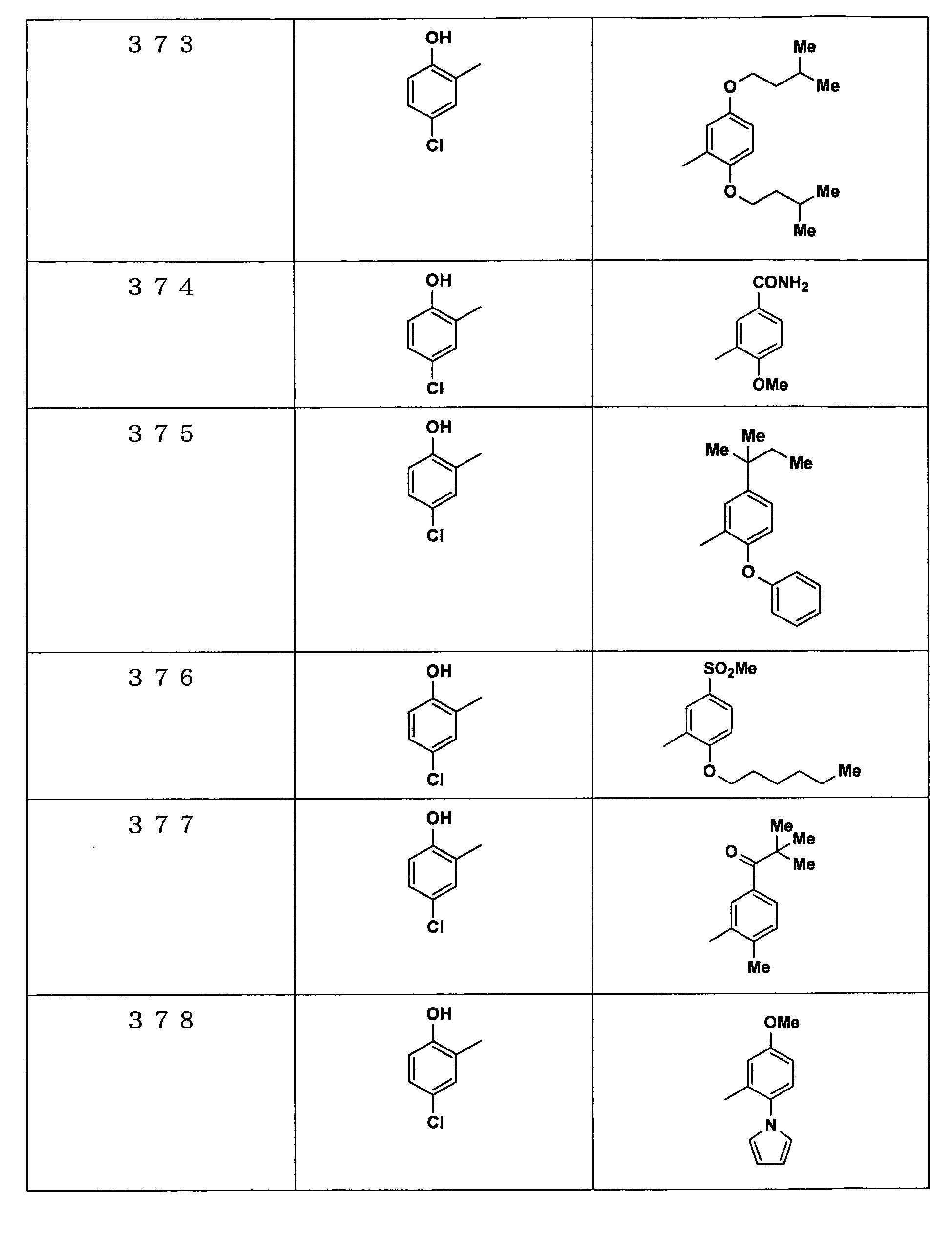

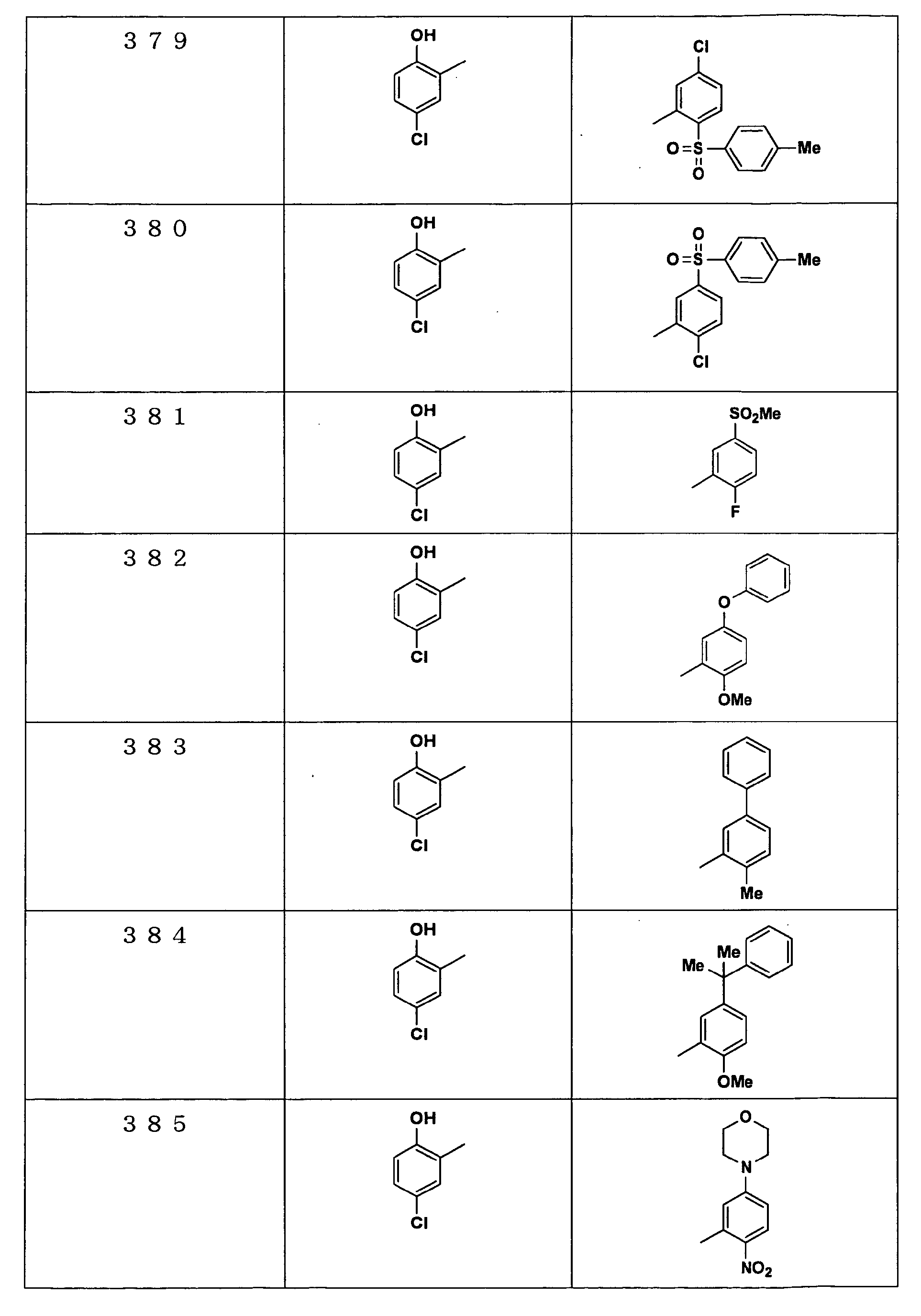

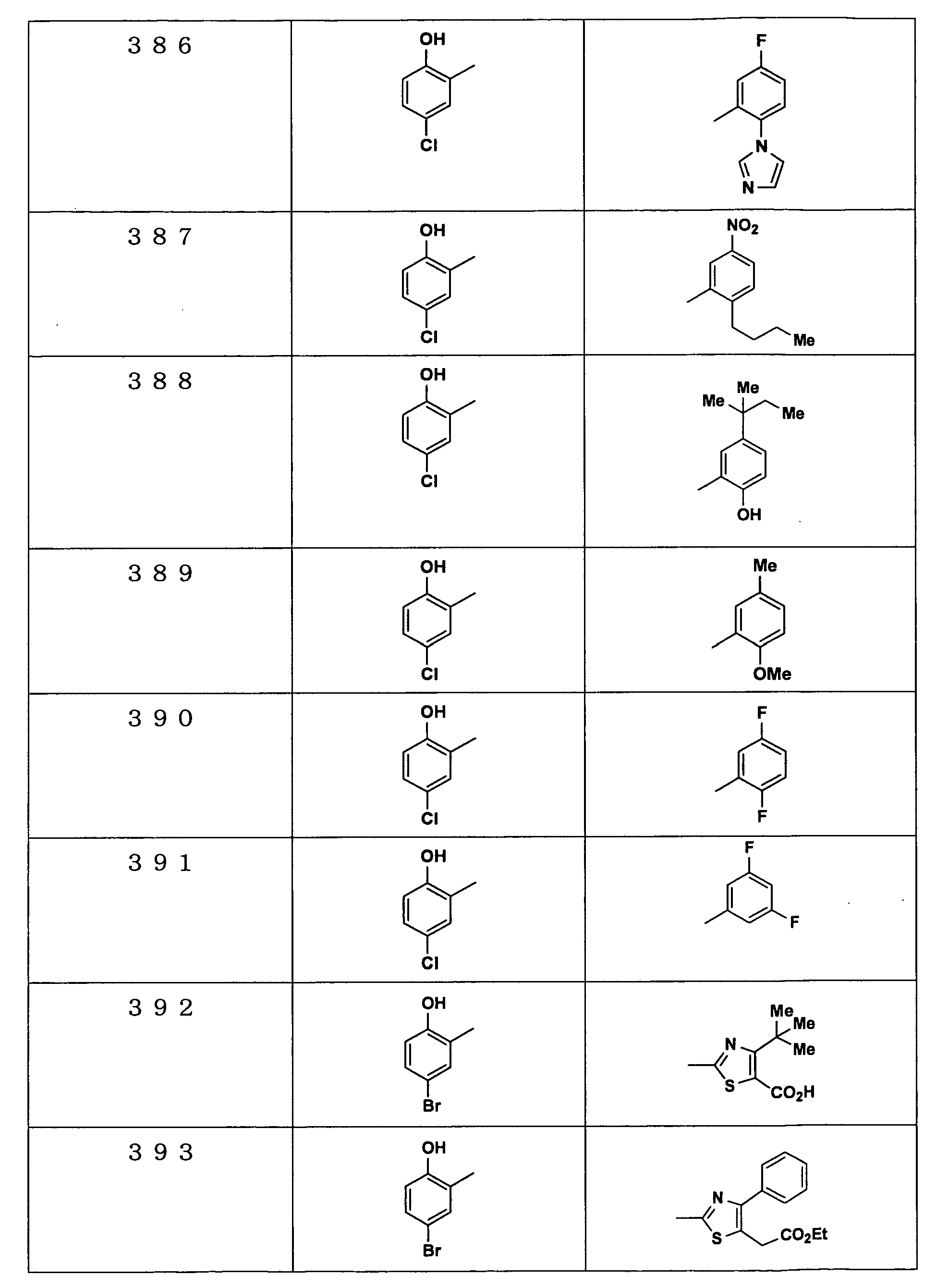

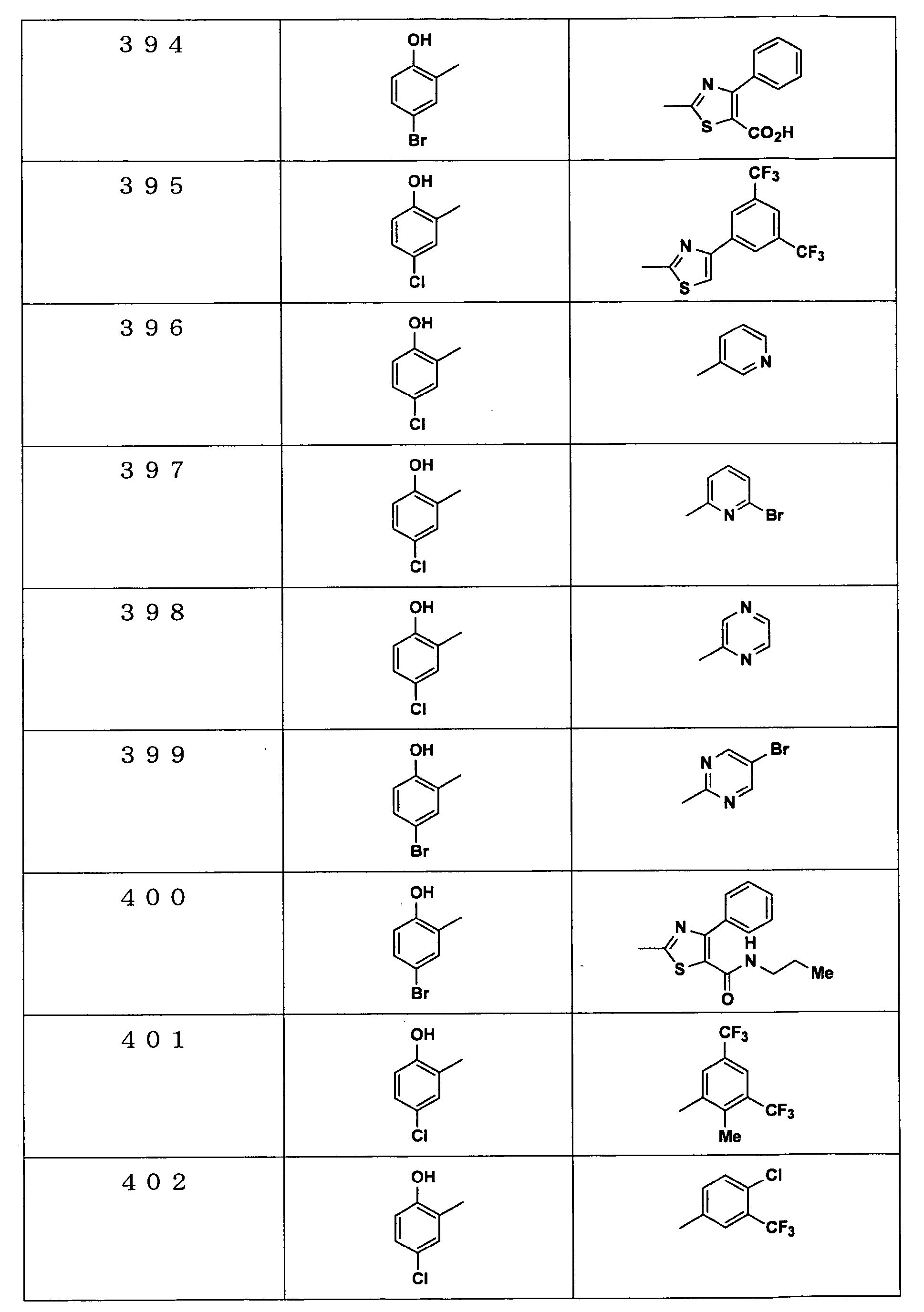

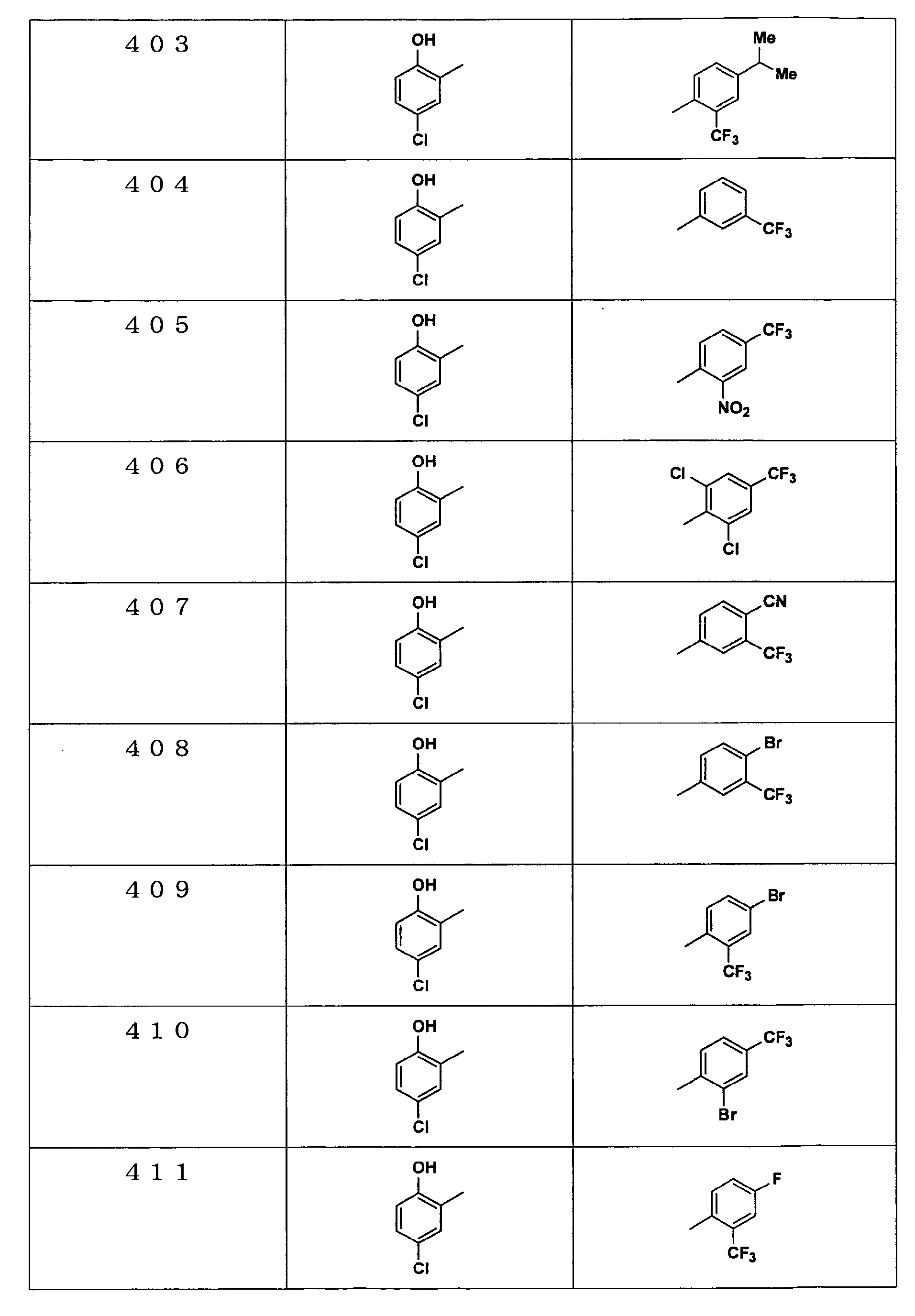

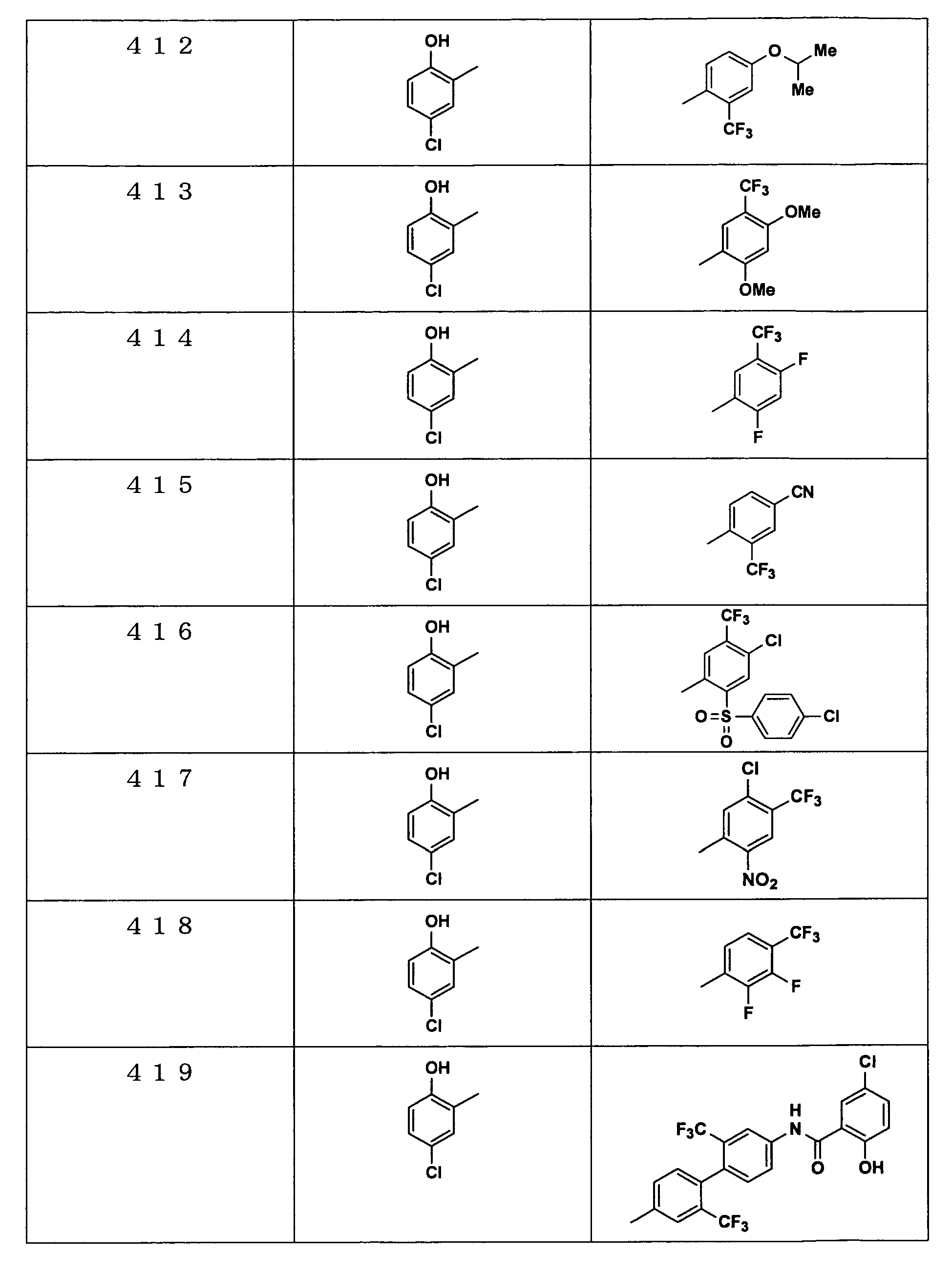

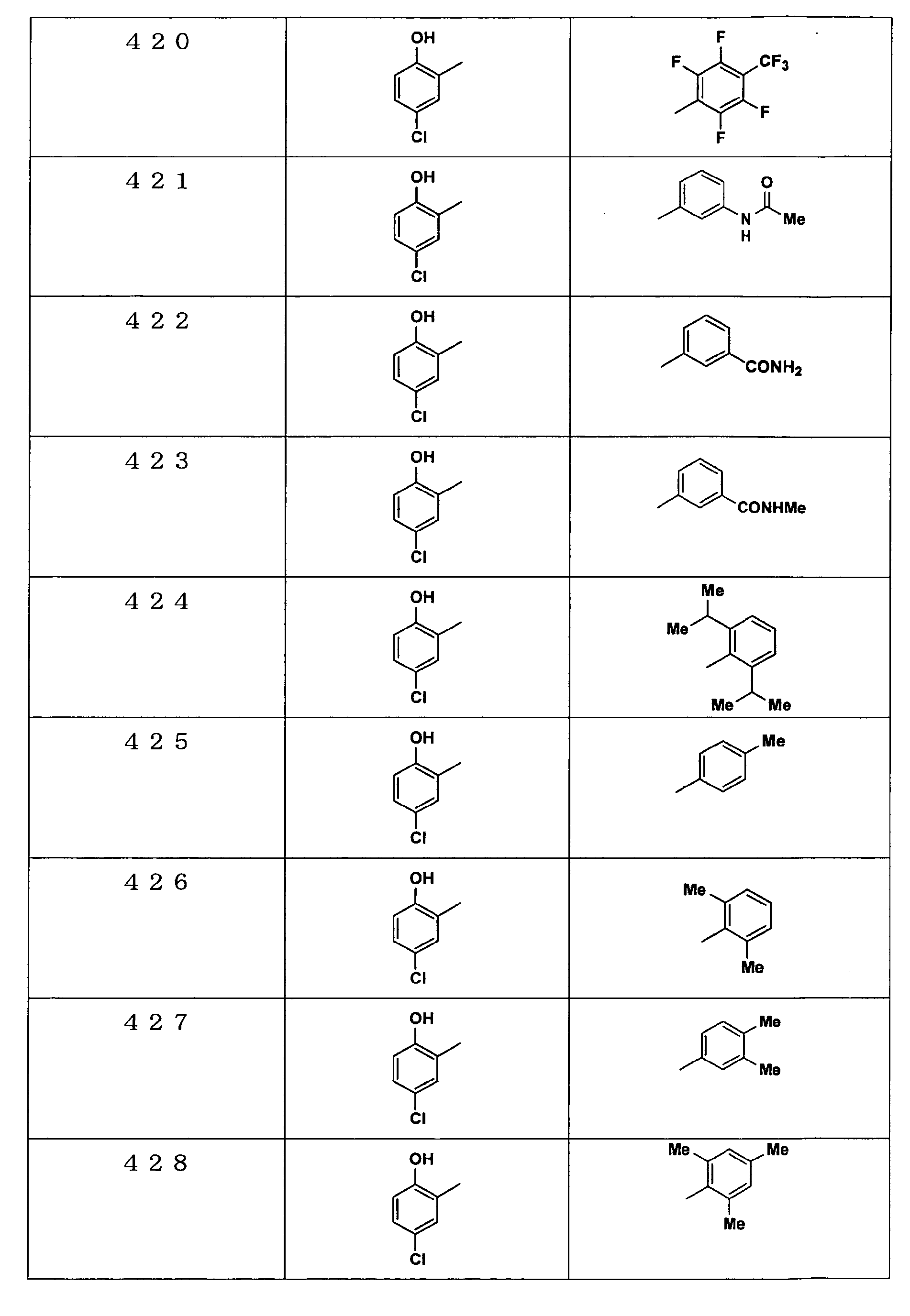

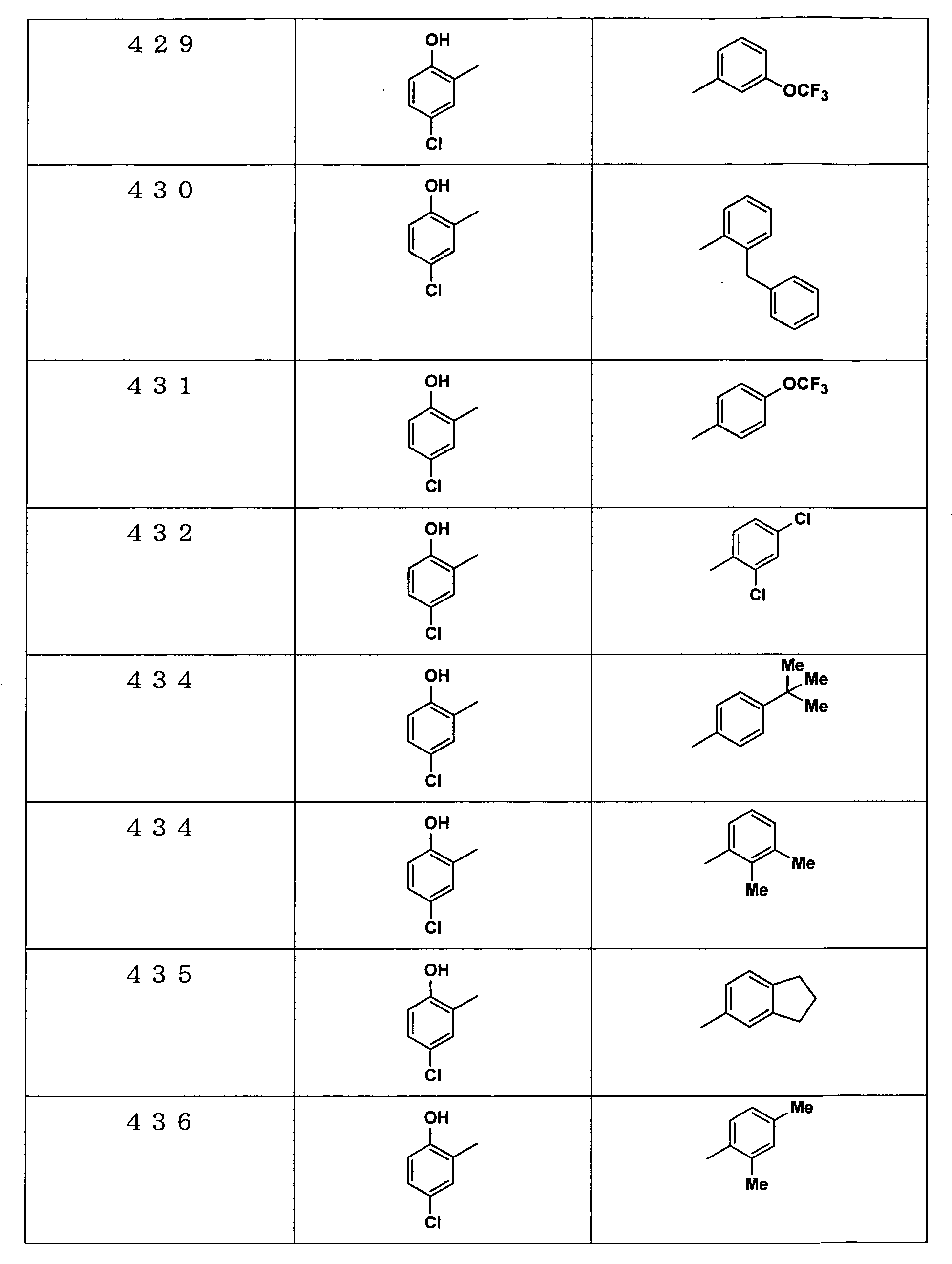

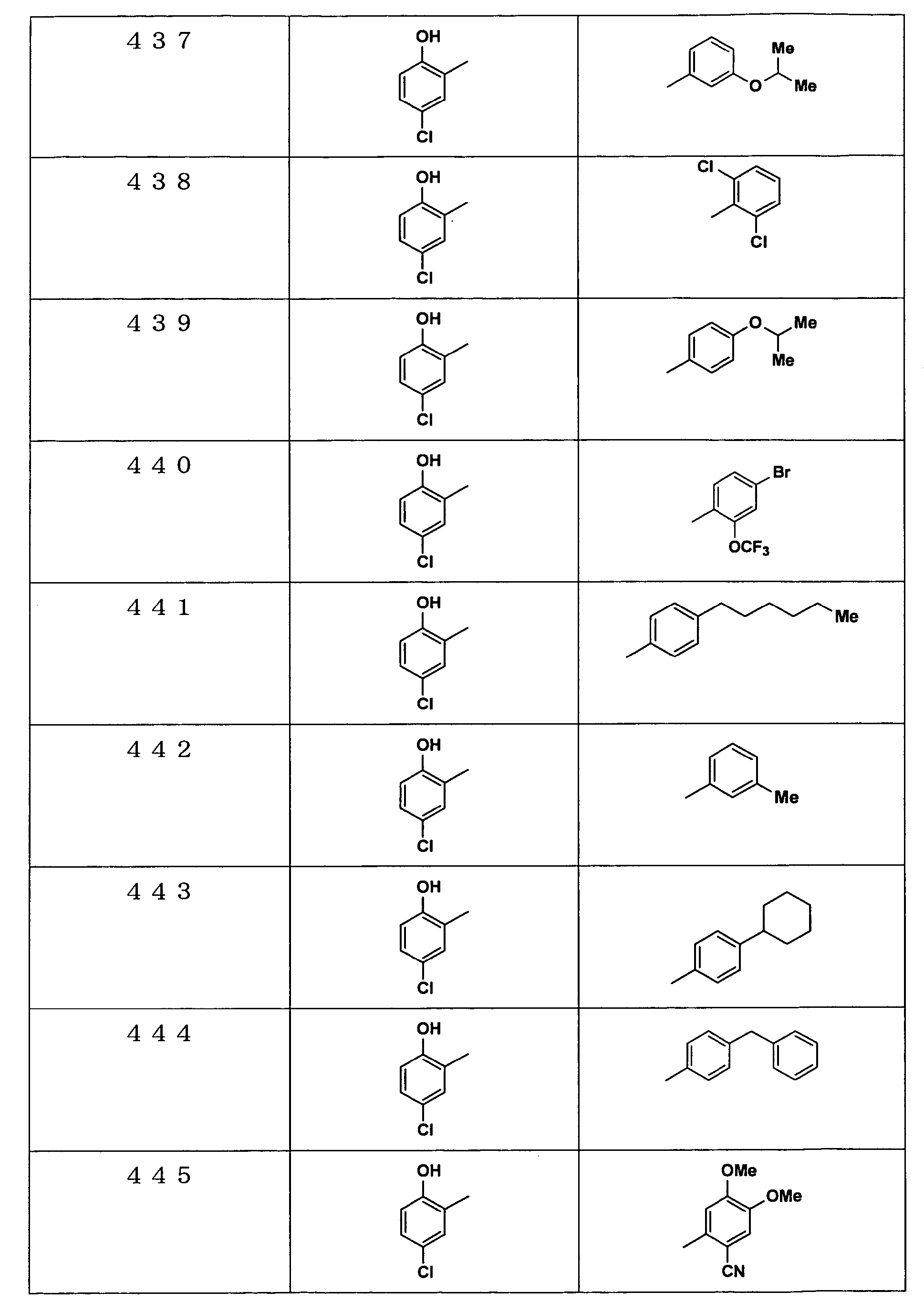

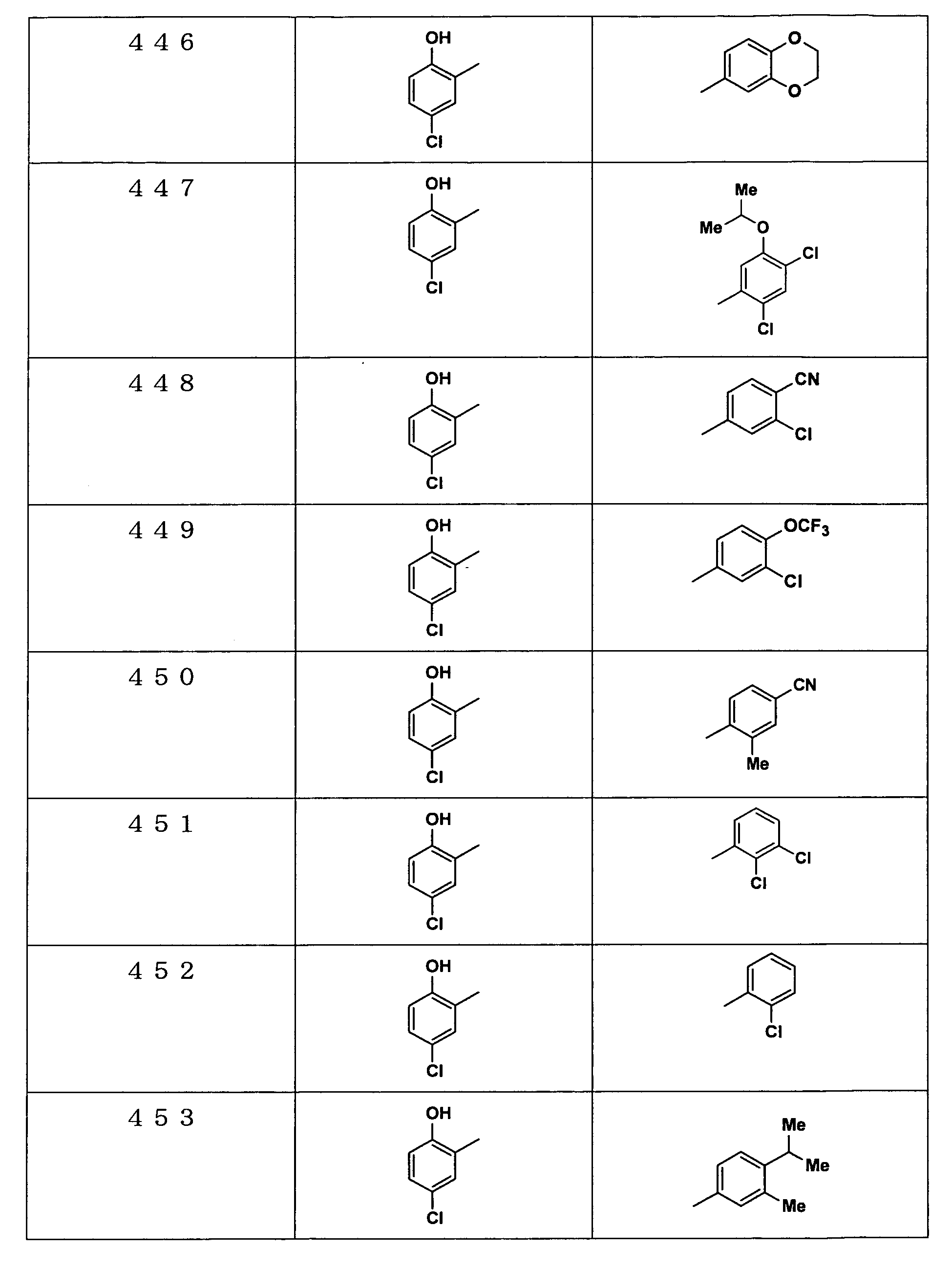

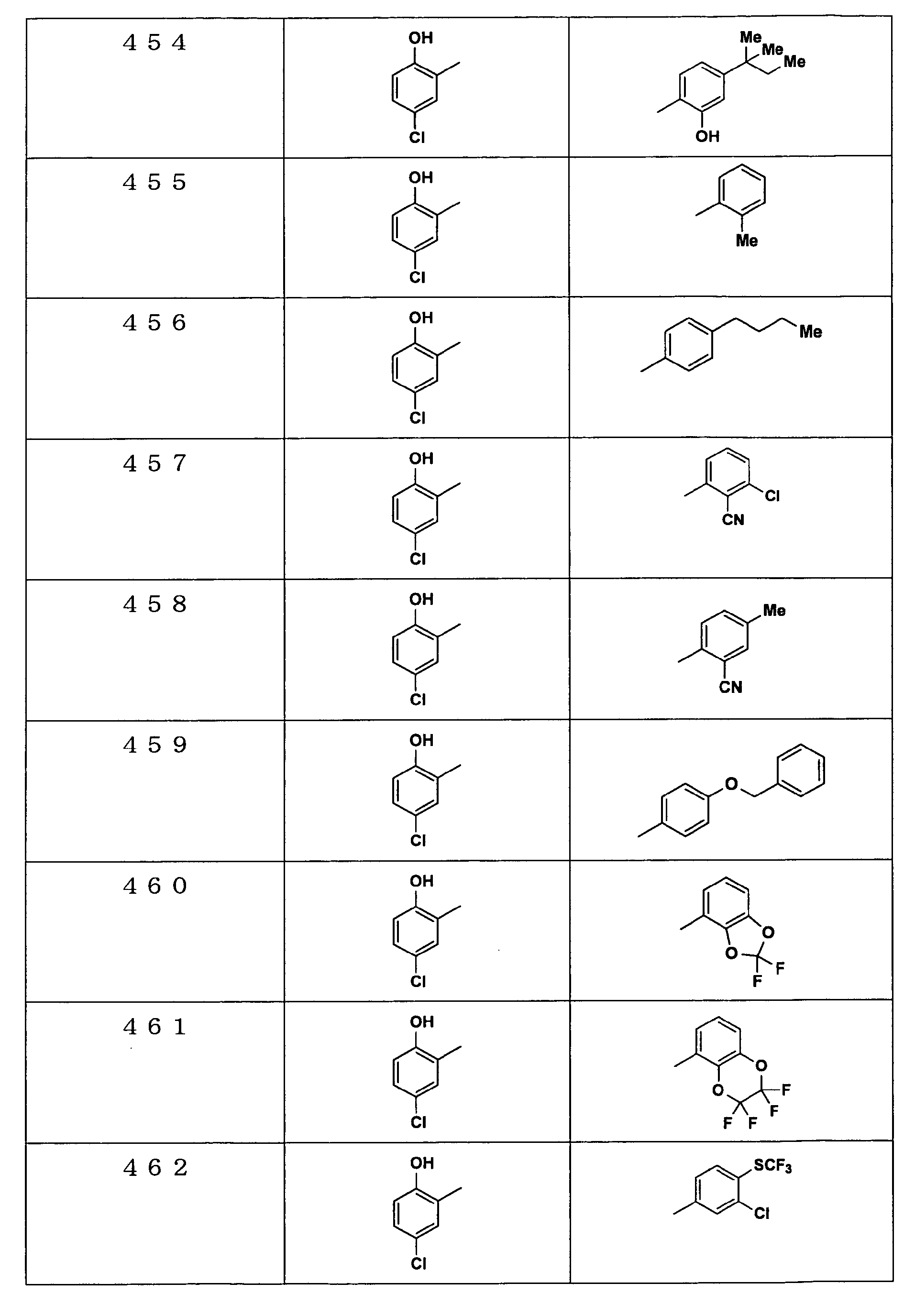

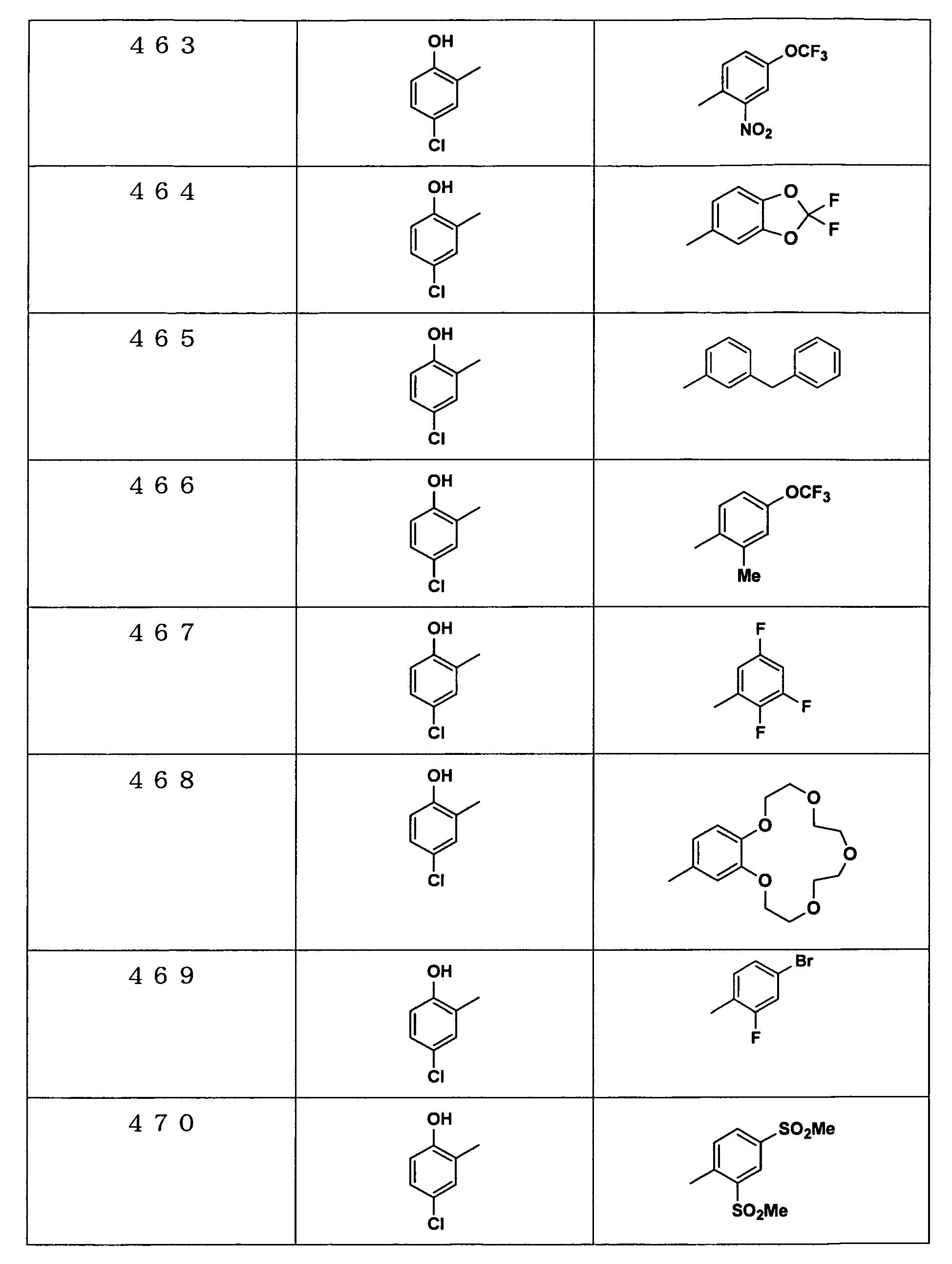

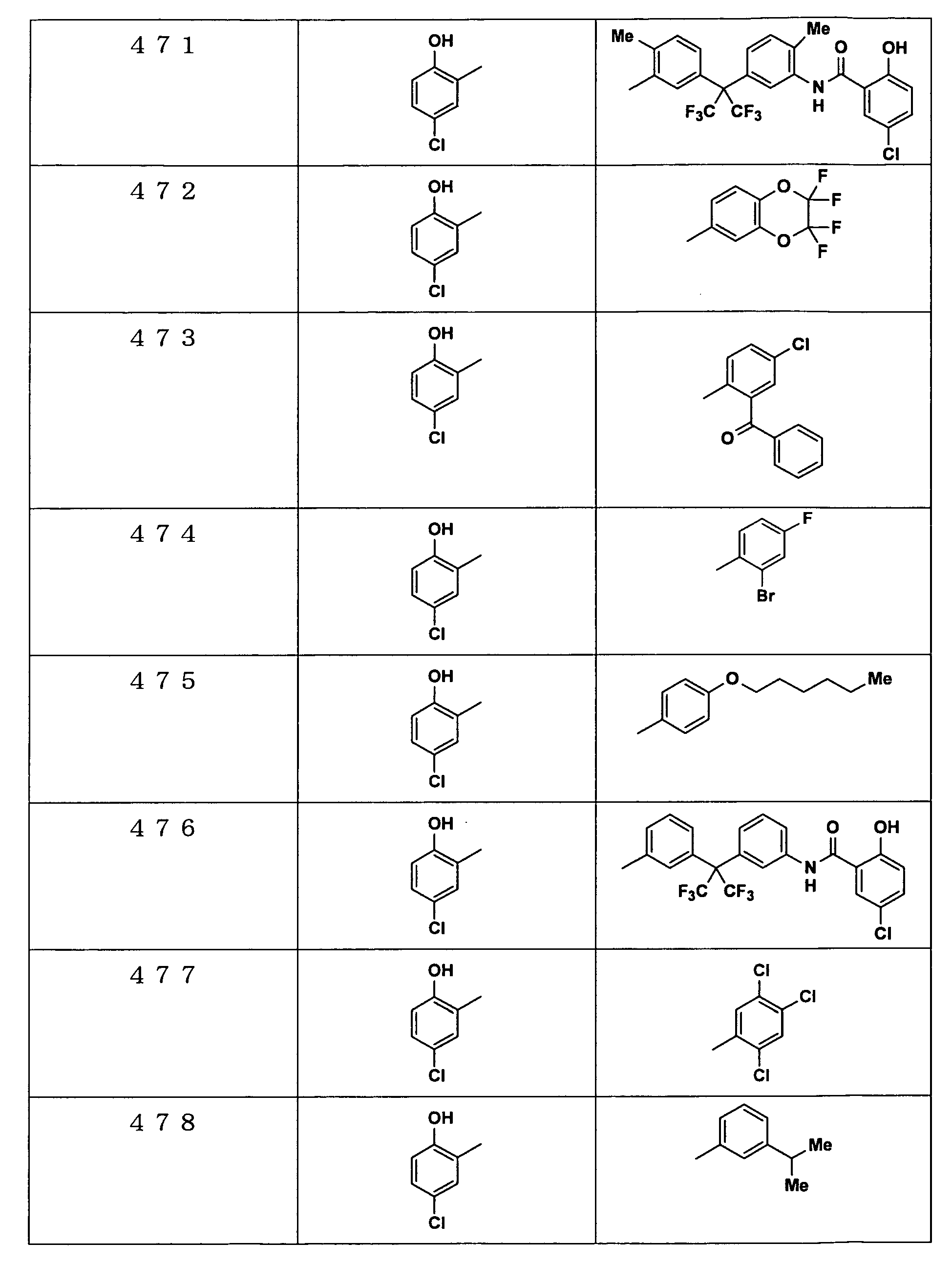

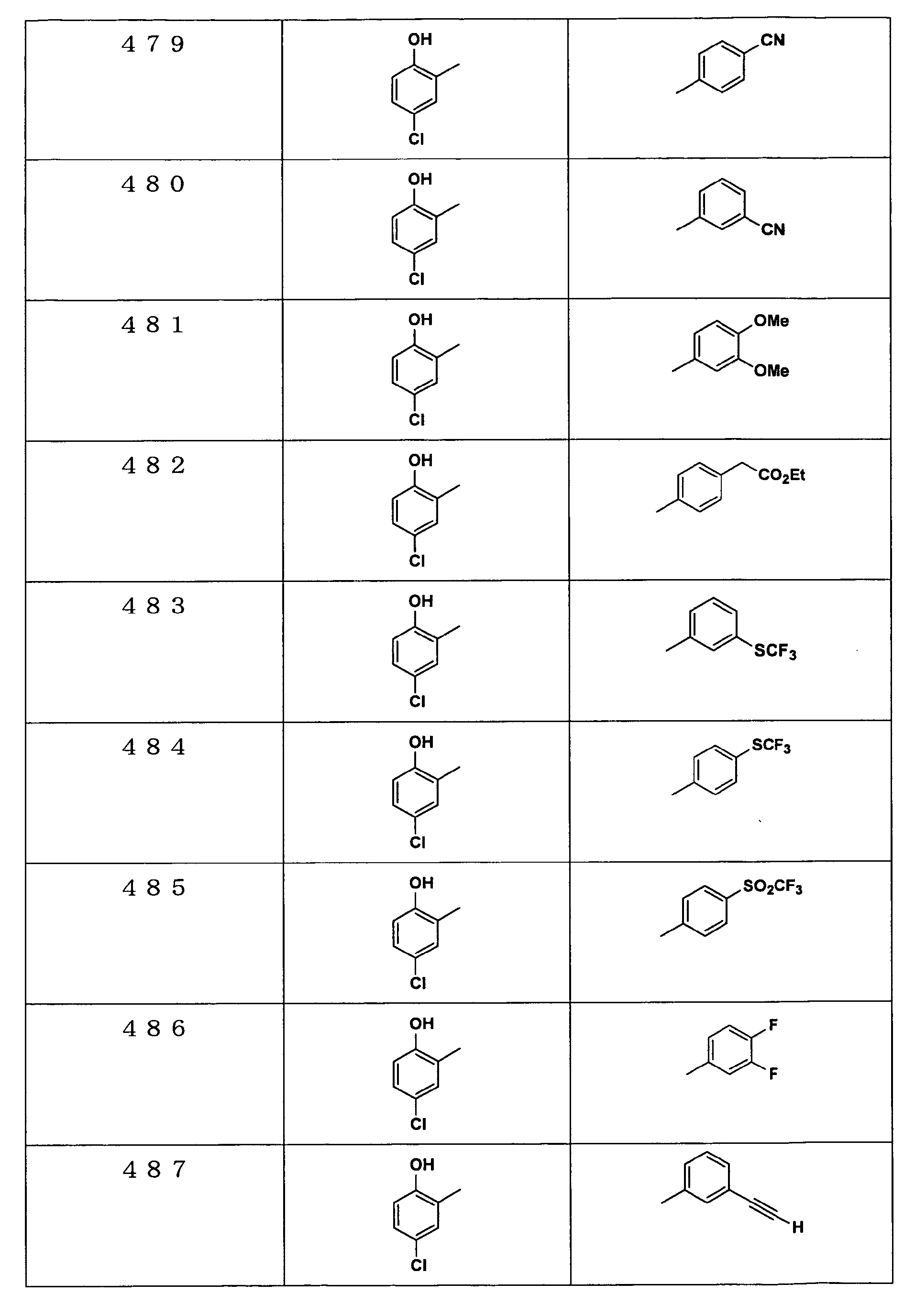

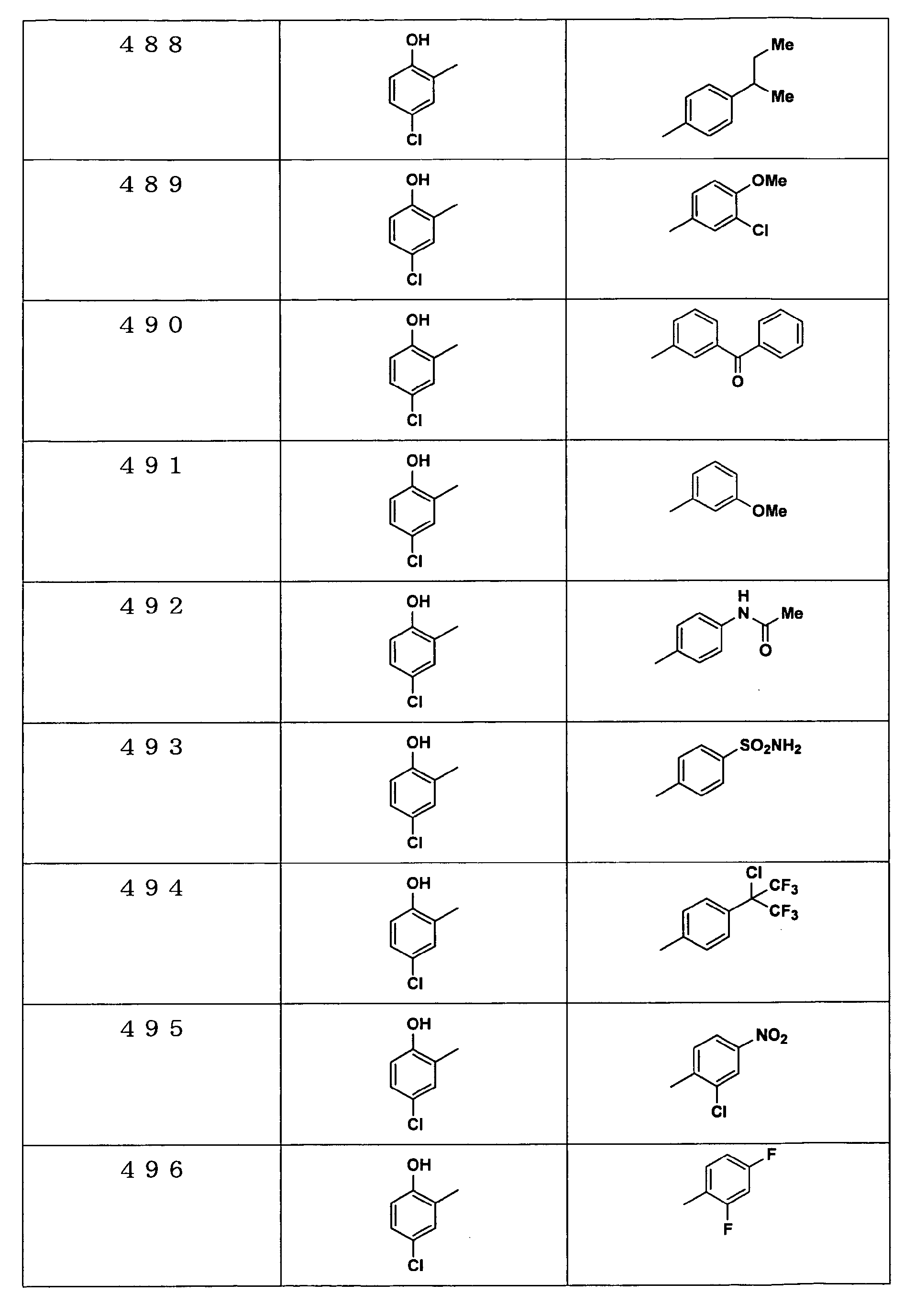

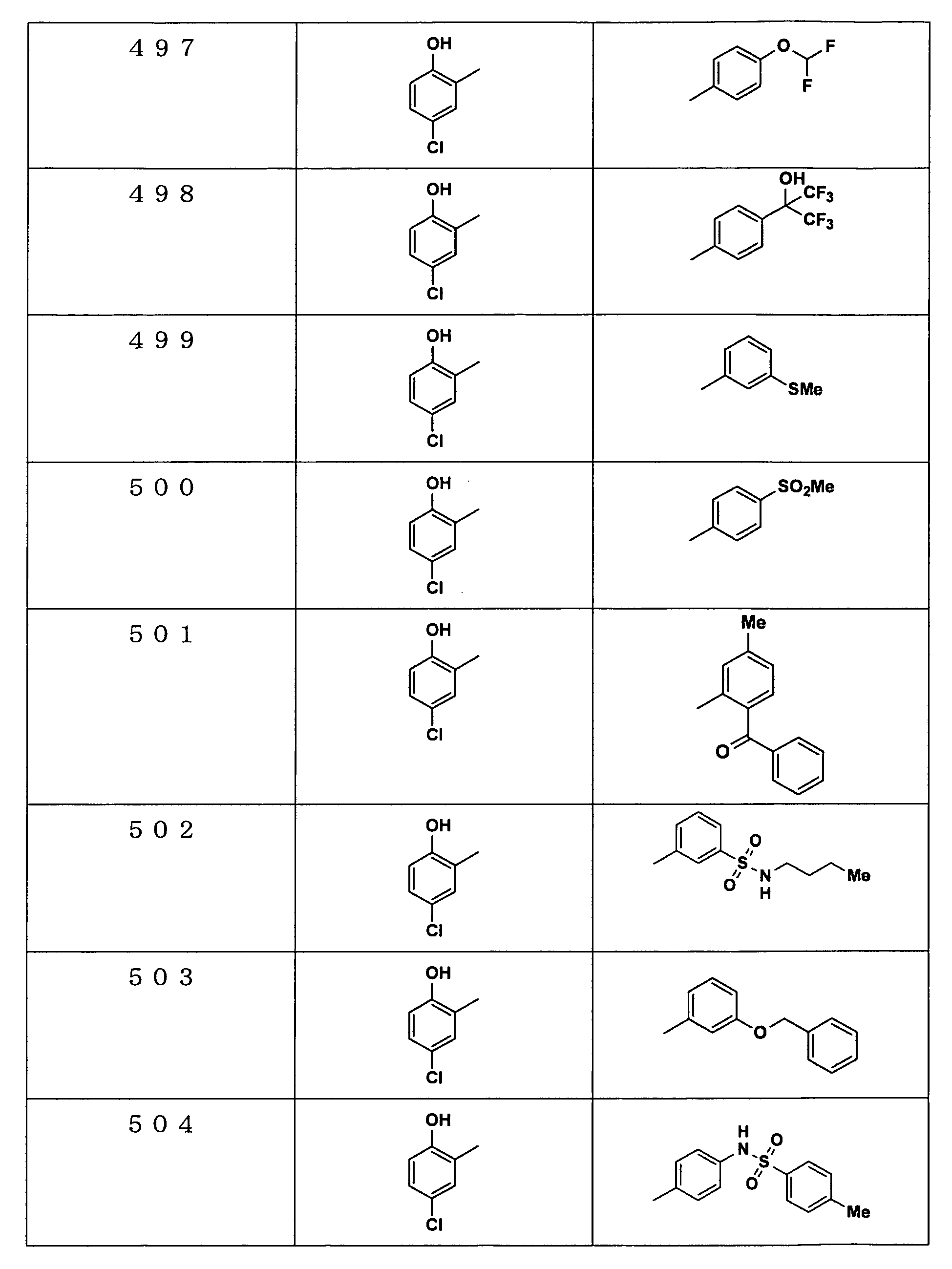

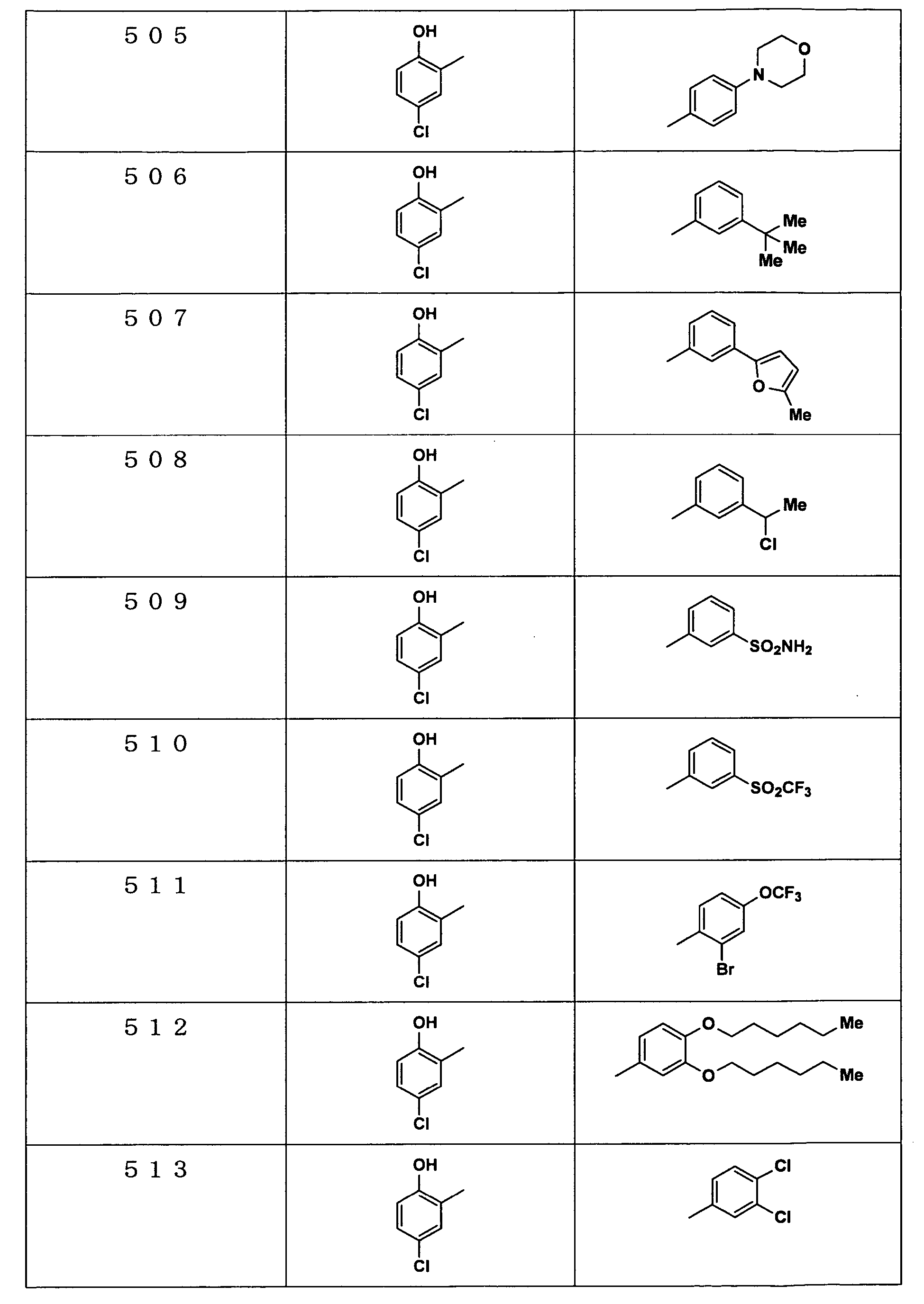

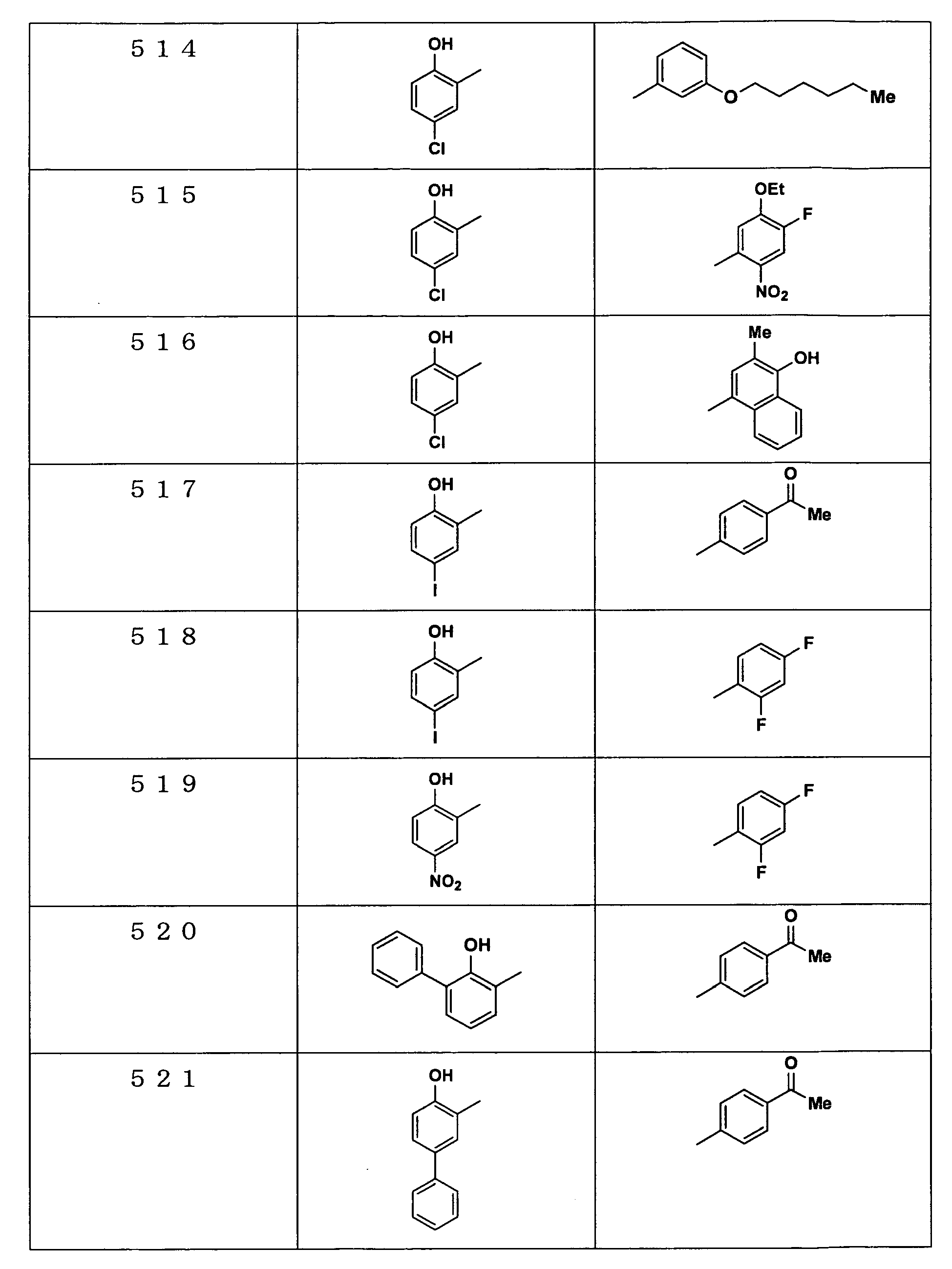

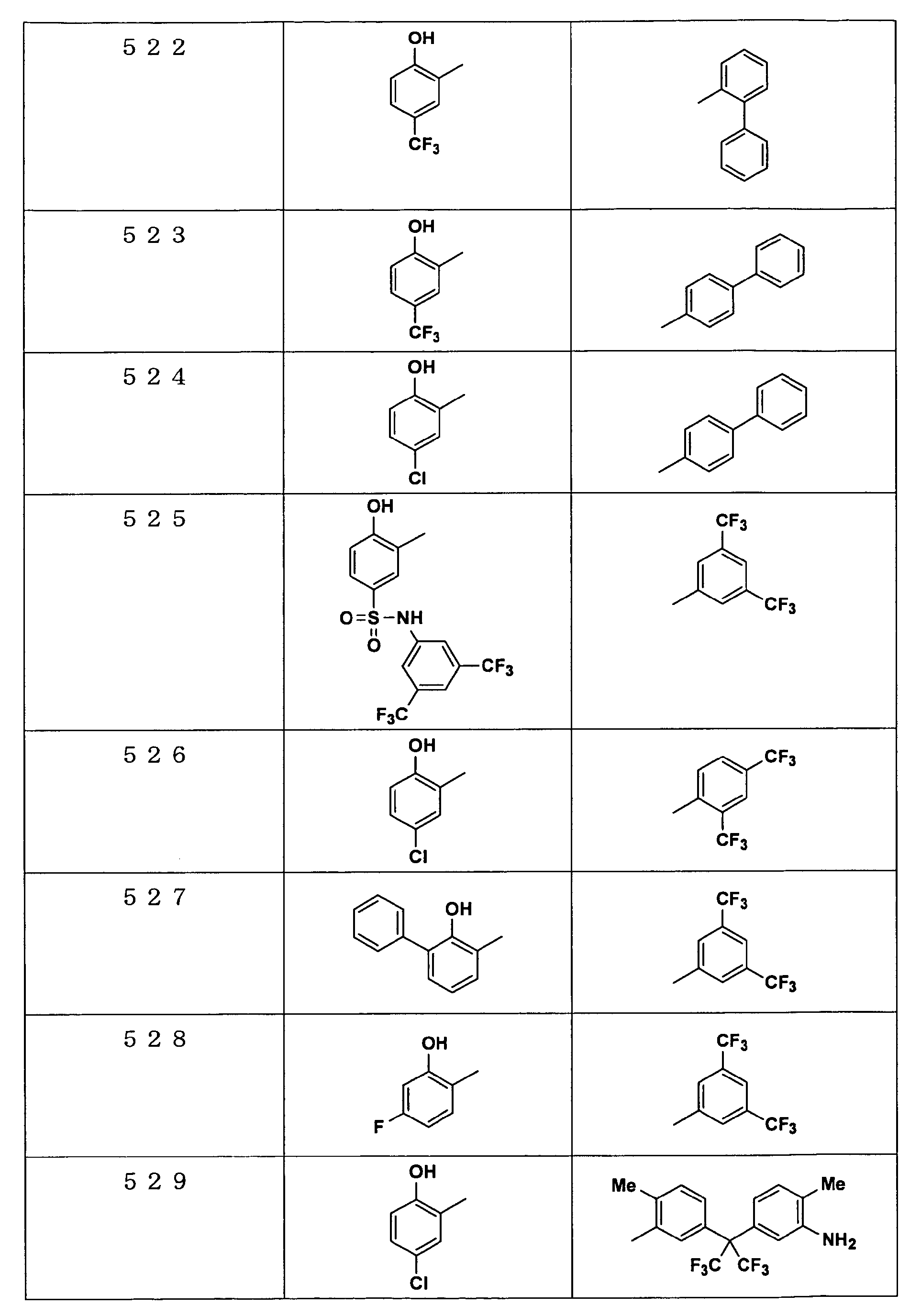

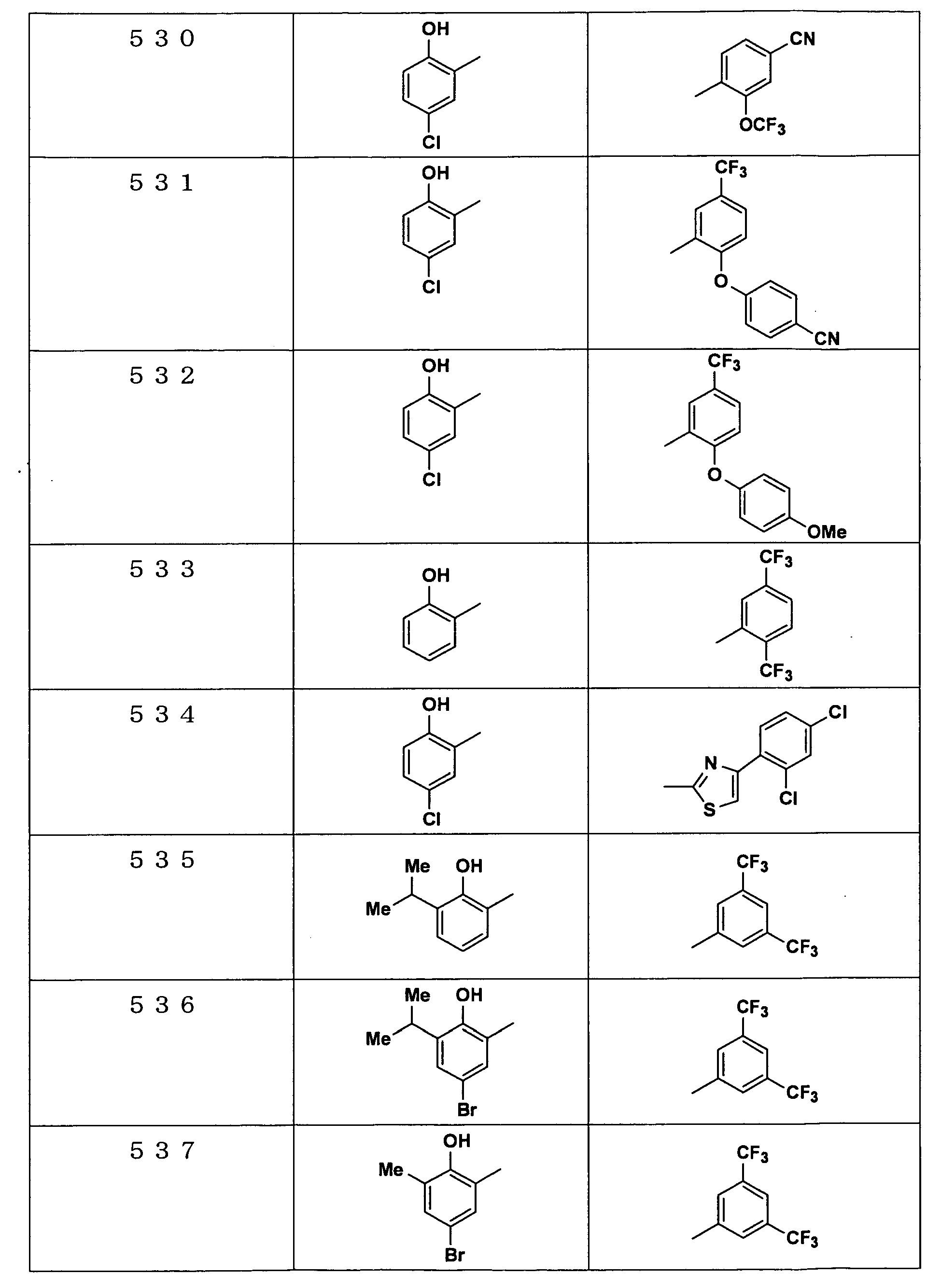

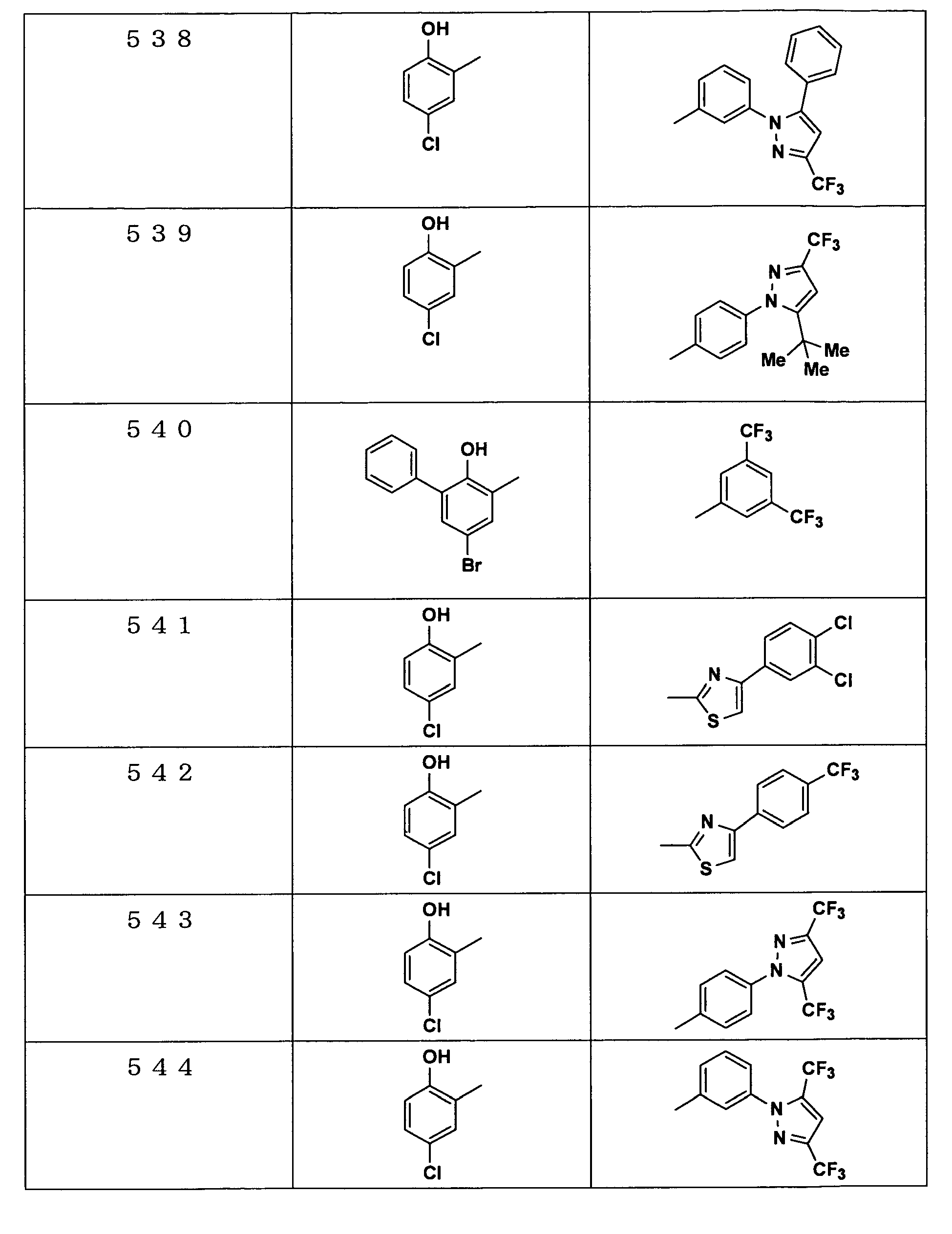

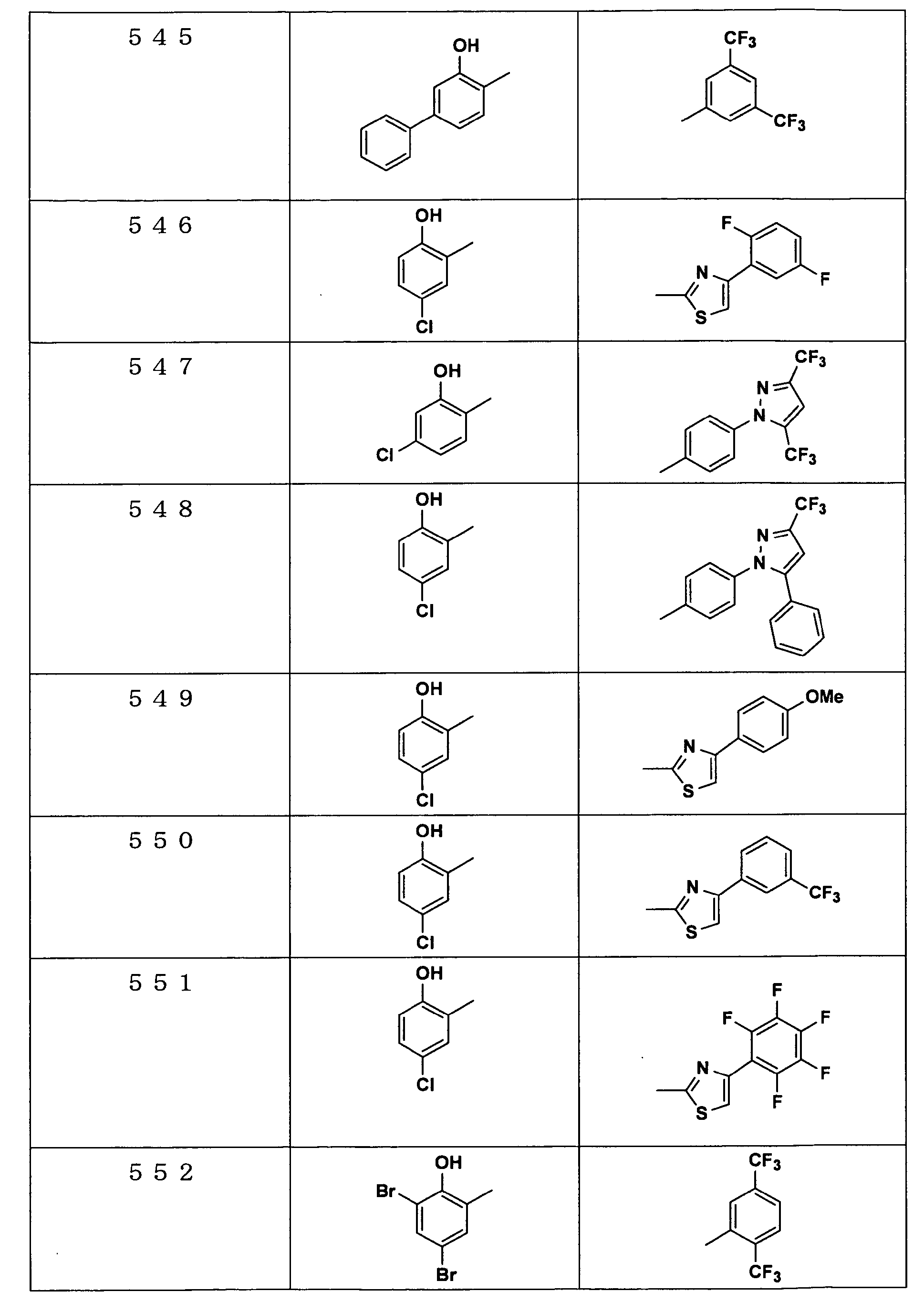

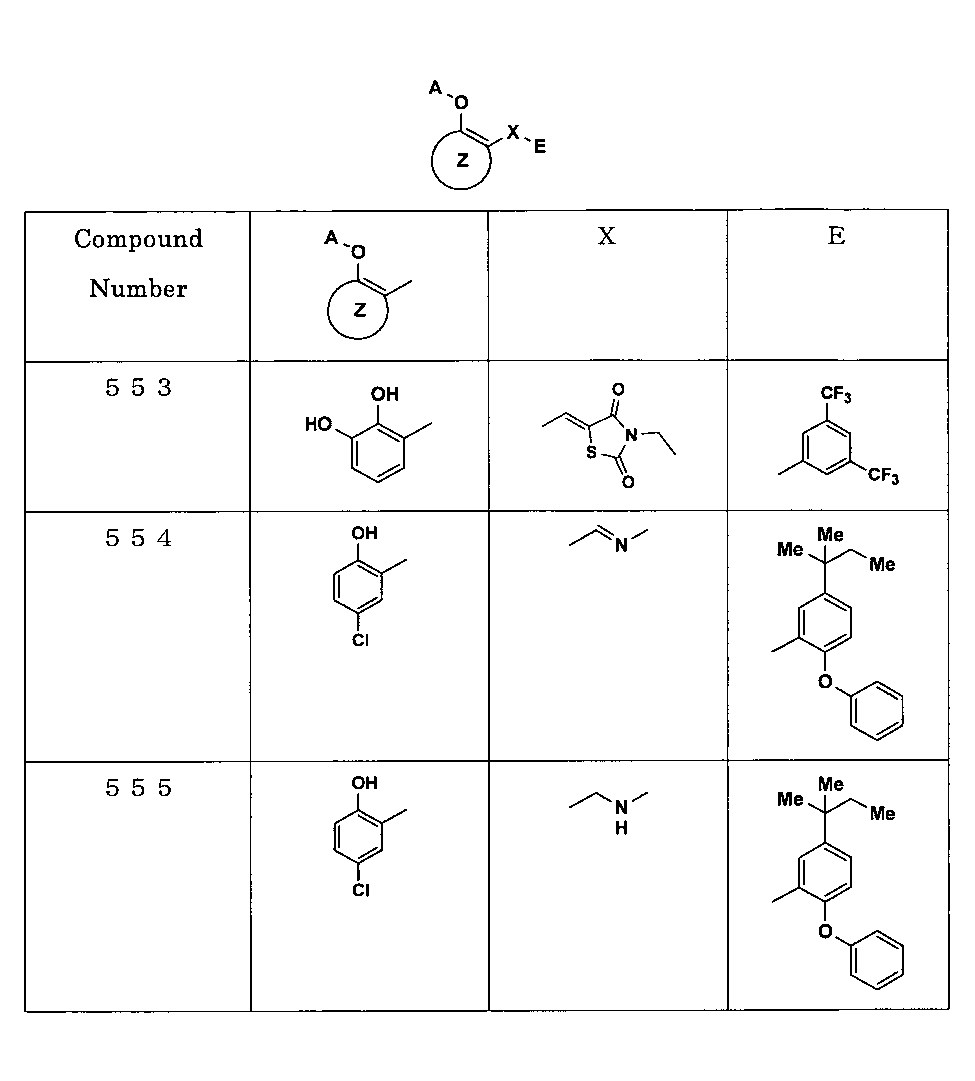

The compounds represented by the general formula (I) have inhibitory activity against transformation and proliferation of melanocytes caused by ultraviolet irradiation, and are effective as an active ingredient of a medicament for preventive and/or therapeutic treatment of dermal pigmentation caused by the transformation and proliferation of melanocytes, and/or of skin cancer development. More specifically, the medicament of the present invention is useful for preventive and/or therapeutic treatment of pigmentation due to sunburn, pigmentation caused by the transformation and proliferation of melanocytes involved in skin inflammation such as atopic dermatitis, pigmentation by the transformation and proliferation of melanocytes caused by proliferation-inducing stimulation, and pigmentation in melanocyte proliferating diseases. Furthermore, the medicament of the present invention is useful for prevention of skin cancer development caused by ultraviolet irradiation.

The compounds of the present invention can be blended in cosmetic compositions as an active ingredient having an effect for skin whitening.

The compounds of the present invention have inhibitory activity against secretion of cytokines inducing cell proliferation. Accordingly, the compounds are useful for preventive and/or therapeutic treatment of dermal diseases with cell proliferation such as keloid and psoriasis.

It is considered that secretion of a cytokine that induces cell proliferation can be suppressed by inhibiting NF- κ B activation. In the pamphlet of International Patent Publication WO03/49632 and the pamphlet of International Patent Publication WO03/103654, the compounds encompassed within the general formula (I) as being the active ingredients of the medicament of the present invention are disclosed to have an inhibitory activity against NF- κ B activation. Results of measurements of thir inhibitory activities against NF- κ B activation are disclosed on pages 289 to 298 of the pamphlet of International Patent Publication WO02/49632, and on pages 254 to 265 of the pamphlet of International Patent Publication WO03/103654. Accordingly, a class of the compounds encompassed within the general formula (I) as the active ingredients of the medicament of the present invention are expected to have the aforementioned various pharmacological actions.

As the active ingredient of the medicament on the present invention, one or more kinds of substances selected from the group consisting of the compound represented by the general formula (I) and a pharmacologically acceptable salt thereof, and a hydrate thereof and a solvate thereof may be used. The aforementioned substance, per se, may be administered as the medicament of the present invention, however, preferably, the medicament of the present invention is provided in the form of a pharmaceutical composition comprising the aforementioned substance which is an active ingredient together with one or more pharmacologically acceptable pharmaceutical additives. In the aforementioned pharmaceutical compositions, a ratio of the active ingredient to the pharmaceutical additives is 1 weight % to 90 weight %.

The medicament of the present invention may be administered as pharmaceutical compositions for oral administration, for example, granules, subtilized granules, powders, hard capsules, soft capsules, syrup, emulsion, suspension, or solution, or may be administered as pharmaceutical compositions for parenteral administration, for example, injections for intravenous administration, intramuscular administration, or subcutaneous administration, drip infusions, suppositories, percutaneous absorbent, transmucosal absorption preparations, nasal drops, ear drops, instillation, inhalants, creams, ointments, and cataplasms. Preparations made as pharmaceutical compositions in a form of powder may be dissolved upon use and administered as injections or drip infusions. Particularly, the medicament of the present invention may sometimes be preferably applied parenterally and topically as external preparation such as creams, ointments, and cataplasms.

For preparation of pharmaceutical compositions, solid or liquid pharmaceutical additives may be used. Pharmaceutical additives may either be organic or inorganic. When an oral solid preparation is prepared, an excipient is added to the active ingredient, and further binders, disintegrator, lubricant, colorant, corrigent are added, if necessary, to manufacture preparations in the forms of tablets, coating tablets, granules, powders, capsules and the like by ordinary procedures. Examples of the excipient include lactose, sucrose, saccharose, glucose, corn starch, starch, talc, sorbit, crystal cellulose, dextrin, kaolin, calcium carbonate, and silicon dioxide. Examples of the binder include, for example, polyvinyl alcohol, polyvinyl ether, ethyl cellulose, methyl cellulose, gum Arabic, tragacanth, gelatine, shellac, hydroxypropyl cellulose, hydroxypropyl methyl cellulose, calcium citrate, dextrin, and pectin. Examples of the lubricant include, for example, magnesium stearate, talc, polyethylene glycol, silica, and hydrogenated vegetable oil. As the coloring agent, any material can be used which are approved to be added to ordinary pharmaceuticals. As the corrigent, cocoa powder, menthol, aromatic acid, peppermint oil, d-borneol, cinnamon powder and the like can be used. These tables and granules may be applied with sugarcoating, gelatin coating, or an appropriate coating, if necessary. Preservatives, antioxidant and the like may be added, if required.

For liquid preparations for oral administration such as emulsions, syrups, suspensions, and solutions, ordinary used inactive diluents, for example, water or vegetable oil may be used. For these preparations, besides inactive diluents, adjuvants such as wetting agents, suspending aids, sweating agents, flavoring agents, coloring agents or preservatives may be blended. After a liquid preparation is manufactured, the preparation may be filled in capsules made of a absorbable substance such as gelatin. Examples of solvents or suspending agents used for the preparations of parenteral administration such as injections or suppositories include, for example, water, propylene glycol, polyethylene glycol, benzyl alcohol, ethyl oleate, and lecithin. Examples of base materials used for preparation of suppositories include, for example, cacao butter, emulsified cacao butter, lauric fat, and witepsol. Methods for preparation of the aforementioned preparations are not limited, and any method ordinarily used in the art may be used.

When the composition are prepared in the form of injections, carriers such as, for example, diluents including water, ethanol, macrogol, propylene glycol, citric acid, acetic acid, phosphoric acid, lactic acid, sodium lactate, sulfuric acid and sodium hydroxide, pH modifiers and buffer solutions including sodium citrate, sodium acetate and sodium phosphate, stabilizers such as sodium pyrosulfite, ethylenediaminetetraacetic acid, thioglycolic acid and thiolactate may be used. For the preparation, a sufficient amount of a salt, glucose, mannitol or glycerin may be blended in the preparation to manufacture an isotonic solution, and an ordinary solubilizer, a soothing agent, or a topical anesthetic may be used.

When the preparation in the form of an ointment such as a paste, a cream, and a gel is manufactured, an ordinarily used base material, a stabilizer, a wetting agent, and a preservative may be blended, if necessary, and may be prepared by mixing the components by a common method. As the base material, for example, white petrolatum, polyethylene, paraffin, glycerin, cellulose derivatives, polyethylene glycol, silicon, and bentonite may be used. As the preservative, paraoxy methyl benzoate, paraoxy ethyl benzoate, paraoxy propyl benzoate and the like may be used. When the preparation in the form of a patch is manufactured, the aforementioned ointment, cream gel, or paste and the like may be applied by a common method to an ordinary support. As the support, fabric made of cotton, span rayon, and synthetic fibersor or nonwoven fabric, and a film or a foam sheet such as made of soft vinyl chloride, polyethylene, and polyurethane and the like may be preferably used.

A dose of the medicament of the present invention is not particularly limited. For oral administration, a dose may generally be 0.01 to 5,000 mg per day for an adult as the weight of the compound of the present invention. It is preferred to increase or decrease the above dose appropriately depending on the age, pathological conditions, and symptoms of a patient. The above dose may be administered once a day or 2 to 3 times a day as divided portions with appropriate intervals, or intermittent administration for every several days may be applied. When the medicament is used as an injection, the dose may be 0.001 to 100 mg per day for an adult as the weight of the compound of the present invention.

A cosmetic composition having a skin whitening effect can be provided by blending in a cosmetics one or more kinds of substances selected from the group consisting of the compound represented by the general formula (I) and a pharmacologically acceptable salt thereof, and a hydrate thereof and a solvate thereof. The term "skin whitening" used in the present specification includes a concept of "beautifying skin," for example, and the term should be interpreted in the broadest sense including inhibition of pigmentation, and prevention and improvement of skin dullness, skin darkening by sunburn, spots, and freckles, and the term should not be construed in any limitative sense. A form of the cosmetic composition of the present invention is not particularly limited. The composition may be in any form such as an emulsion, a cream, a skin lotion, an essence, a pack, a facial wash, a make-up component, a dispersion liquid, and an ointment.

The cosmetic composition of the present invention may be added with, if necessary and within limit of not impairing the effect of the present invention, additives ordinarily used for cosmetics, quasi drugs, and external drugs, specifically one or more kinds of water (purified water, ordinary water, hot spring water, and deep-sea water), alcohols, oil solutions, detergents, fine particles, thickeners, ultraviolet ray absorbing agents, antibacterial agents, flavoring agents, pH adjusters, refrigerants, extracts from plants, animals, and microorganisms, blood circulation accelerators, astringent drugs, antiseborrheic drugs, whitening agents, anti-inflammatory agents, anti-wrinkle agents, active oxygen-eliminating agents, antioxidants, cytotonic agents, moisturizing agents, or chelating agents. An amount of the substances selected from the group consisting of the compound represented by the general formula (I) and a pharmacologically acceptable salt thereof, and a hydrate thereof and a solvate thereof to be added in a cosmetic composition may be appropriately selected by those skilled in the art depending on a form of a cosmetic composition and a kind of the above substance which is an active ingredient for skin whitening. The amount may be, for example, within a range of 0.0001 weight % to 10 weight %.

### Examples

The present invention will be explained more specifically with reference to the following examples. However the scope of the present invention is not limited to the following examples. The compound numbers in the following examples correspond to those described in the pamphlet of International Patent Publication WO03/103647.

### Test Example 1: Proliferation Inhibitory Test against Melanocyte Caused by Ultraviolet Irradiation

Melanocytes were cultured according to the authentic method, and the proliferation of melanocytes in the presence or absence of test drugs under the ultraviolet irradiation was measured. As a result, the compound (N-[3,5-bis(trifluoromethyl)phenyl]-5-chloro-2-hydroxybenzamide) with the compound number 50 described in the pamphlet of International Patent Publication WO03/103647 almost completely inhibited the transformation and proliferation of melanocyte at a concentration of 1 *µ* g/ml.

### Industrial Applicability

The medicaments of the present invention are useful for preventive and/or therapeutic treatment of dermal pigmentation and/or development of skin cancer.

## Claims

1. A medicament for preventive and/or therapeutic treatment of dermal pigmentation and/or development of skin cancer, which comprises as an active ingredient a substance selected from the group consisting of a compound represented by the following general formula (I) and a pharmacologically acceptable salt thereof, and a hydrate thereof and a solvate thereof: 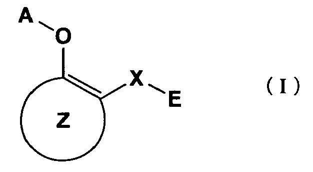 wherein X represents a connecting group whose number of atoms in a main chain is 2 to 5 (said connecting group may be substituted),
A represents hydrogen atom or acetyl group,
E represents an aryl group which may be substituted or a heteroaryl group which may be substituted,
ring Z represents an arene which may have one or more substituents in addition to the group represented by formula -0-A wherein A has the same meaning as that defined above and the group represented by formula -X-E wherein each of X and E has the same meaning as that defined above, or a heteroarene which may have one or more substituents in addition to the group represented by formula -O-A wherein A has the same meaning as that defined above and the group represented by formula - X-E wherein each of X and E has the same meaning as that defined above.

2. The medicament according to claim 1, wherein X is a group selected from the following connecting group α (said group may be substituted),
A is hydrogen atom or acetyl group,
E is a C₆ to C₁₀ aryl group which may be substituted or a 5- to 13-membered heteroaryl group which may be substituted,
ring Z is a C₆ to C₁₀ arene which may have one or more substituents in addition to the group represented by formula -O-A wherein A has the same meaning as that defined above and the group represented by formula -X-E wherein each of X and E has the same meaning as that defined above, or a 5- to 13-membered heteroarene which may have one or more substituents in addition to the group represented by formula ―O―A wherein A has the same meaning as that defined above and the group represented by formula -X-E wherein each of X and E has the same meaning as that defined above.
[Connecting Group α] The following formulas: 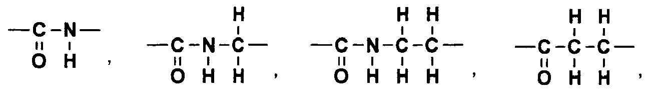 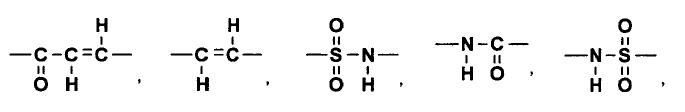 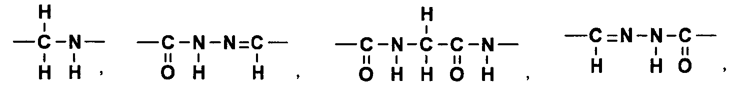 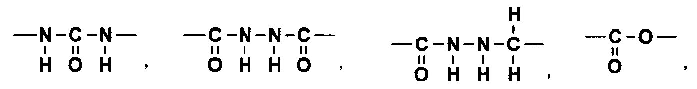 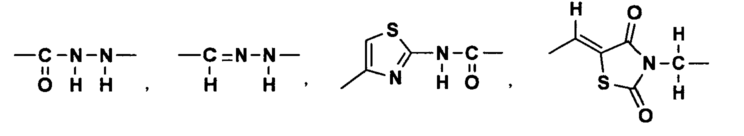 wherein a bond at the left end binds to ring Z and a bond at the right end binds to E.

3. The medicament according to claim 1, wherein X is a group represented by the following formula (said group may be substituted): 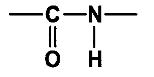 wherein a bond at the left end binds to ring Z and a bond at the right end binds to E, A is hydrogen atom or acetyl group,
E is a C₆ to C₁₀ aryl group which may be substituted or a 5- to 13-membered heteroaryl group which may be substituted,
ring Z is a C₆ to C₁₀ arene which may have one or more substituents in addition to the group represented by formula ―O―A wherein A has the same meaning as that defined above and the group represented by formula -X-E wherein each of X and E has the same meaning as that defined above, or a 5- to 13-membered heteroarene which may have one or more substituents in addition to the group represented by formula ―O―A wherein A has the same meaning as that defined above and the group represented by formula ―X―E wherein each of X and E has the same meaning as that defined above.

4. The medicament according to claim 3, wherein A is hydrogen atom or acetyl group,
E is a phenyl group which may be substituted or a thiazol-2-yl group which may be substituted,
ring Z is a benzene ring which may have one or more substituents in addition to the group represented by formula -O-A wherein A has the same meaning as that defined above and the group represented by formula -X-E wherein each of X and E has the same meaning as that defined above, or a naphthalene ring which may have one or more substituents in addition to the group represented by formula -O-A wherein A has the same meaning as that defined above and the group represented by formula -X-E wherein each of X and E has the same meaning as that defined above.

5. The medicament according to claim 3, wherein A is hydrogen atom, E is a 2,5-di-substituted phenyl group, a 3,5-di-substituted phenyl group, or a 4,5-di-substituted thiazol-2-yl group,
ring Z is a benzene ring which has one to three substituents in addition to the group represented by formula ―O―A wherein A has the same meaning as that defined above and the group represented by formula -X-E wherein each of X and E has the same meaning as that defined above.

6. The medicament according to claim 3, wherein A is hydrogen atom, E is a 2,5-di-substituted phenyl group wherein at least one of said substituents is trifluoromethyl group, a 3,5-di-substituted phenyl group wherein at least one of said substituents is trifluoromethyl group, or a 4,5-di-substituted thiazol-2-yl group, ring Z is a benzene ring which has one to three groups selected from the following substituent group γ -1z, in addition to the group represented by formula ―O―A wherein A has the same meaning as that defined above and the group represented by formula -X-E wherein each of X and E has the same meaning as that defined above. [Substituent Group γ -1z] halogen atoms, nitro group, cyano group, hydroxy group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group [thiophen-2-yl group], 3-thienyl group [thiophen-3-yl group], 1-pyrrolyl group [pyrrol-1-yl group], 2-methylthiazol-4-yl group, imidazo[1,2-a]pyridin-2-yl group, 2-pyridyl group [pyridin-2-yl group], acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group

7. The medicament according to claim 3, wherein A is hydrogen atom, E is a group selected from the group consisting of the following substituent group δ -3e, substituent group δ -5e, and substituent group δ -8e,
the following partial formula (Iz-1) in the general formula (I) containing ring Z 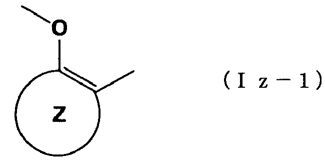 is the following formula (Iz-2): 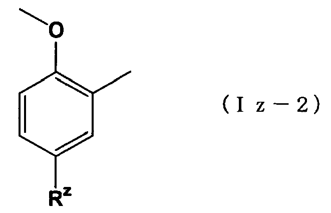 wherein R^{z} represents a group selected from the following substituent group γ -2z. [Substituent Group δ -3e] 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 2-(4-cyanophenoxy)-5-(trifluoromethyl)phenyl group, 2-(4-methoxyphenoxy)-5-(trifluoromethyl)phenyl group
[Substituent Group δ -5e] 3,5-bis(trifluoromethyl)phenyl group, 3-fluoro-5-(trifluoromethyl)phenyl group, 3-bromo-5-(trifluoromethyl)phenyl group, 3-methoxy-5-(trifluoromethyl)phenyl group, 3-methoxycarbonyl-5-(trifluoromethyl)phenyl group, 3-carboxy-5-(trifluoromethyl)phenyl group
[Substituent Group δ -8e] 5-bromo-4-[(1,1-dimethyl)ethyl]thiazol-2-yl group, 5-bromo-4-(trifluoromethyl)thiazol-2-yl group, 5-cyano-4-[(1,1-dimethyl)ethyl]thiazol-2-yl group, 5-methylthiazol-2-yl group, 4,5-dimethylthiazol-2-yl group, 5-methyl-4-phenylthiazol-2-yl group, 5-(4-fluorophenyl)-4-methylthiazol-2-yl group, 4-methyl-5-[3-(trifluoromethyl)phenyl]thiazol-2-yl group, 4-[(1,1-dimethyl)ethyl]-5-ethylthiazol-2-yl group, 4-ethyl-5-phenylthiazol-2-yl group, 4-isopropyl-5-phenylthiazol-2-yl group, 4-butyl-5-phenylthiazol-2-yl group, 4-[(1,1-dimethyl)ethyl]-5-[(2,2-dimethyl)propionyl]thiazol-2-yl group, 4-[(1,1-dimethyl)ethyl]-5-(ethoxycarbonyl)thiazol-2-yl group, 4-[(1,1-dimethyl)ethyl]-5-piperidinothiazol-2-yl group, 4-[(1,1-dimethyl)ethyl]-5-morpholinothiazol-2-yl group, 4-[(1,1-dimethyl)ethyl]-5-(4-methylpiperazin-1-yl)thiazol-2-yl group, 4-[(1,1-dimethyl)ethyl]-5-(4-phenylpiperazin-1-yl)thiazol-2-yl group, 5-carboxymethyl-4-phenylthiazol-2-yl group, 4,5-diphenylthiazol-2-yl group, 4-benzyl-5-phenylthiazol-2-yl group, 5-phenyl-4-(trifluoromethyl)thiazol-2-yl group, 5-acetyl-4-phenylthiazol-2-yl group, 5-benzoyl-4-phenylthiazol-2-yl group, 5-ethoxycarbonyl-4-phenylthiazol-2-yl group, 5-ethoxycarbonyl-4-(pentafluorophenyl)thiazol-2-yl group, 5-methylcarbamoyl-4-phenylthiazol-2-yl group, 5-ethylcarbamoyl-4-phenylthiazol-2-yl group, 5-isopropylcarbamoyl-4-phenylthiazol-2-yl group, 5-(2-phenylethyl)carbamoyl-4-phenylthiazol-2-yl group, 5-ethoxycarbonyl-4-(trifluoromethyl)thiazol-2-yl group, 5-carboxy-4-[(1,1-dimethyl)ethyl]thiazol-2-yl group, 5-(ethoxycarbonyl)methyl-4-phenylthiazol-2-yl group, 5-carboxy-4-phenylthiazol-2-yl group, 5-propylcarbamoyl-4-phenylthiazol-2-yl group
[Substituent Group γ -2z] halogen atoms, nitro group, cyano group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group, 3-thienyl group, 1-pyrrolyl group, 2-methylthiazol-4-yl group, imidazo[1,2-a]pyridin-2-yl group, 2-pyridyl group, acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, {[4-(pyridin-2-yl)sulfamoyl]phenyl}diazenyl group

8. The medicament according to claim 3, wherein A is hydrogen atom, E is a group selected from the group consisting of the aforementioned substituent group δ -3e, substituent group δ -5e, and substituent group δ -8e, the following partial formula (Iz-1) in the general formula (I) containing ring Z 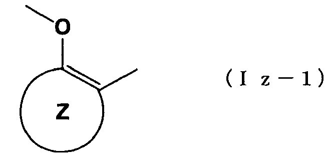 is the following formula (Iz-2): 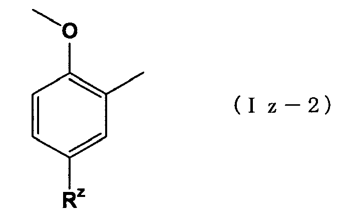 wherein R^{z} represents a halogen atom.

9. The medicament according to claim 3, wherein A is hydrogen atom, E is 2,5-bis(trifluoromethyl)phenyl group, 3,5-bis(trifluoromethyl)phenyl group, or 4-[(1,1-dimethyl)ethyl]-5-[(2,2-dimethyl)propionyl]thiazol-2-yl group,
the following partial formula (Iz-1) in the general formula (I) containing ring Z 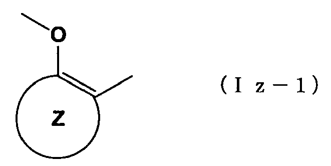 is the following formula (Iz-2): 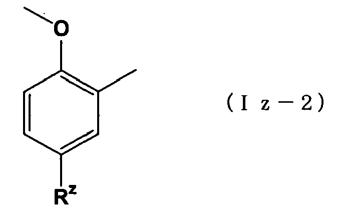 wherein R^{z} represents a halogen atom.

10. The medicament according to claim 3, wherein A is hydrogen atom, E is 3,5-bis(trifluoromethyl)phenyl group,
the following partial formula (Iz-1) in the general formula (I) containing ring Z 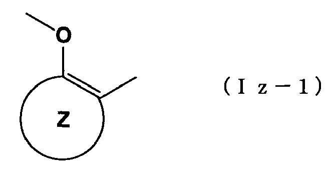 is the following formula (Iz-2): 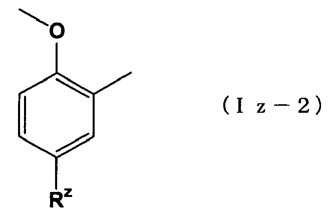 wherein R^{z} represents a halogen atom.

11. The medicament according to claim 3, wherein A is hydrogen atom, E is a C₆ to C₁₀ aryl group which may be substituted or a 5- to 13-membered heteroaryl group which may be substituted,
the following partial formula (Iz-1) in the general formula (I) containing ring Z 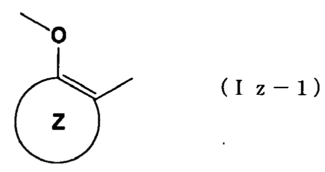 is the following formula (Iz-2): 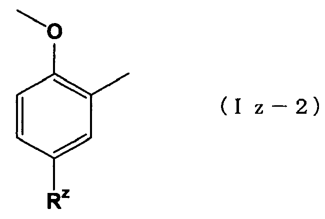 wherein R^{z} represents a halogen atom.

12. The medicament according to claim 3, wherein A is hydrogen atom, E is a 2,5-di-substituted phenyl group, a 3,5-di-substituted phenyl group, or a 4,5-di-substituted thiazol-2-yl group,
the following partial formula (Iz-1) in the general formula (I) containing ring Z 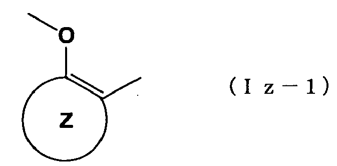 is the following formula (Iz-2): 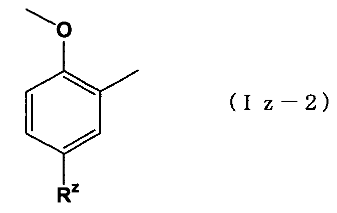 wherein R^{z} represents a halogen atom.

13. The medicament according to claim 3, wherein A is hydrogen atom, E is 3,5-bis(trifluoromethyl)phenyl group,
ring Z is a C₆ to C₁₀ arene which may have one or more substituents in addition to the group represented by formula -O-A wherein A has the same meaning as that defined above and the group represented by formula -X-E wherein each of X and E has the same meaning as that defined above, or a 5- to 13-membered heteroarene which may have one or more substituents in addition to the group represented by formula ― O―A wherein A has the same meaning as that defined above and the group represented by formula ―X―E wherein each of X and E has the same meaning as that defined above.

14. The medicament according to claim 2, wherein the compound represented by the general formula (I) is a compound selected from the group consisting of the compounds described in the pamphlet of International Patent Publication WO03/103647 as Compound No. 1 to 555.

15. The medicament according to claim 3, wherein the compound represented by the general formula (I) is a compound selected from the group consisting of the compounds described in the pamphlet of International Patent Publication WO03/103647 as Compound No. 18 to 223 and Compound No. 322 to 555.

16. The medicament according to any one of claims 1 to 15, having inhibitory activity against transformation and/or proliferation of melanocytes caused by ultraviolet irradiation.
